# EUROPEAN PATENT APPLICATION

(11) **EP 4 636 055 A1**
(43) Date of publication of application: **22.10.2025**
(21) Application number: 23902732.9
(22) Date of filing: 13.12.2023
(51) Int. Cl.: C09K 19/56, C09K 19/44, G02F 1/1337

(54) **SELF ALIGNMENT AGENT AND LIQUID CRYSTAL COMPOSITION THEREOF**

(30) Priority: 15.12.2022 CN 202211618868; 15.12.2022 CN 202211620433; 15.12.2022 CN 202211620434
(71) Applicant: Jiangsu Hecheng Display Technology Co., Ltd., Nanjing, Jiangsu 210000 (CN)
(72) Inventor: YANG, Yafei, Nanjing, Jiangsu 210000 (CN); DING, Jiajia, Nanjing, Jiangsu 210000 (CN); LI, Rongrong, Nanjing, Jiangsu 210000 (CN); DING, Wenquan, Nanjing, Jiangsu 210000 (CN); WANG, Panpan, Nanjing, Jiangsu 210000 (CN); HE, Di, Nanjing, Jiangsu 210000 (CN); DAI, Huijuan, Nanjing, Jiangsu 210000 (CN); ZHOU, Zhenting, Nanjing, Jiangsu 210000 (CN); WANG, Lina, Nanjing, Jiangsu 210000 (CN); SUN, Chenglong, Nanjing, Jiangsu 210000 (CN); ZHAO, Tengyun, Nanjing, Jiangsu 210000 (CN)
(74) Representative: Serjeants LLP
(86) International application number: PCT/CN2023/138322
(87) International publication number: WO 2024/125536

(57) **Abstract**

The present invention provides a self-aligning agent and a liquid crystal composition thereof. The liquid crystal composition comprising the self-aligning agent of general formula O in the present invention has a lower residue concentration, a smaller roughness, a better alignment effect as well as a better low-temperature storage stability and a better pre-tilt angle stability while maintaining an appropriate clearing point, an appropriate optical anisotropy, an appropriate absolute value of dielectric anisotropy, a larger K value (K₁₁ and K₃₃) and a smaller rotational viscosity.

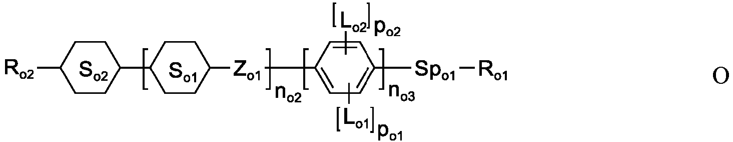

## Description

### Technical Field

The present invention relates to the field of liquid crystal, specifically to a self-aligning agent, a liquid crystal composition thereof and a liquid crystal display device comprising the liquid crystal composition.

### Background Arts

Liquid crystal displays (LCDs) have gained rapid development due to their small size, light weight, low power consumption and excellent display quality, and in particular, have been widely used in portable electronic information products. Based on the displaying mode, liquid crystal displays can be classified into the types of PC (phase change), TN (twisted nematic), STN (super twisted nematic), ECB (electrically controlled birefringence), OCB (optically compensated bend), IPS (in-plane switching), FFS (fringe field switching), VA (vertical alignment), PSA (polymer stable alignment), and the like. According to the driving modes of elements, liquid crystal display elements can be classified into PM (passive matrix) type and AM (active matrix) type. PM is classified into the static type, multiplex type and so forth. AM is classified into TFT (thin film transistor) type, MIM (metal insulator metal) type and so forth. The types of TFT comprise amorphous silicon and polycrystal silicon. The latter is classified into a high-temperature type and a low-temperature type according to the manufacturing process.

A liquid crystal display device includes a nematic liquid crystal composition, and the liquid crystal composition has appropriate characteristics. An AM device having good characteristics can be obtained via improving the characteristics of the liquid crystal composition. The correlations between the characteristics of the liquid crystal composition and AM device are summarized in Table A below. The characteristics of liquid crystal composition are further described based on commercially available AM elements. The temperature range for application of the nematic phase is related to the temperature range for application of the element. The viscosity of the liquid crystal composition is related to the response time of the element. In order for the element to display a dynamic image, it is preferable that the response time of the element is short.

**Table A Characteristics of liquid crystal composition and AM element**

| No. | Characteristics of liquid crystal composition | Characteristics of AM element |
|---|---|---|
| 1 | wide temperature range of a nematic phase | wide temperature range for application |
| 2 | small viscosity | short response time |
| 3 | large optical anisotropy | large contrast |
| 4 | large absolute value of dielectric anisotropy | low threshold voltage, small electric power consumption, large contrast |
| 5 | large specific resistance | large voltage holding ratio, large contrast |
| 6 | ultraviolet light and heat stabilities | long service life |
| 7 | large elastic constant | large contrast, short response time, fast response speed |

In the application of liquid crystal display devices, the impact of contrast on the visual effect is very critical. Generally speaking, the larger the contrast is, the clearer and more eye-catching the image will be, and the more vibrant and gorgeous the colors will be. In turn, if the contrast is small, the whole picture becomes gray and dull. High contrast is of great help for image clarity, detail performance, gray level performance. High contrast products have advantages in black and white contrast, clarity, integrity and so forth. Contrast also has a great impact on the display effect of dynamic video. The light-dark conversion in the dynamic image is fast, therefore, the higher the contrast is, the easier it is for the human eyes to distinguish such a conversion process.

In order to improve the response speed of liquid crystal display device, it is required to minimize the rotational viscosity of liquid crystal materials as much as possible. However, generally, a liquid crystal material with low viscosity has a low clearing point, a low optical anisotropy and the like. Therefore, when formulating the liquid crystal compositions, other performance requirements need to be considered while reducing viscosity.

A PSA-type liquid crystal display device is manufactured by adding a small amount (for example, 0.3 wt.%, typically, < 1 wt.%) of one or more polymerizable compounds into a liquid crystal composition, ensuring that after the liquid crystal composition is filled into a liquid crystal cell, and polymerizing, usually via a UV photopolymerization, or crosslinking the polymerizable compounds in situ under a condition where liquid crystal molecules are initially aligned with or without a voltage applied between electrodes, thereby fixing the alignment of the liquid crystal molecules. With the continuous development of PSA-type liquid crystal display device, it is used in diverse classical liquid crystal displays, such as the known PSA-VA, PSA-OCB, PSA-IPS, PSA-FFS, and PSA-TN displays. In a PSA liquid crystal display, the liquid crystal composition containing the polymerizable compound(s) is positioned between two substrates. Each substrate is equipped with an electrode structure, or alternatively two electrode structures are provided on only one of the substrates, and an orthogonal polarizer is attached to the outside of the substrate. In addition, one or both substrates may contain an alignment film which is provided on the substrate or the electrode structure (if present). Like the conventional liquid crystal displays, PSA liquid crystal displays can be operated as active-matrix displays or passive-matrix displays. In the case of active-matrix displays, individual pixels are addressed by integrated, non-linear active elements, such as transistors, while in the case of passive-matrix displays, individual pixels are usually addressed by the multiplex method, as known from the prior art.

After filling the liquid crystal composition into the display device, the polymerizable compounds contained in the liquid crystal composition are polymerized or crosslinked in situ, usually by UV photopolymerization, which is achieved by exposing the liquid crystal composition to UV radiation, preferably while a voltage is applied to the electrode structures. As a result of UV exposure, the polymerized or crosslinked polymerizable compounds phase-separate from other compounds in the liquid crystal composition and form a polymer layer on the substrate surfaces, where they induce a pre-tilt angle of the liquid crystal molecules relative to the substrates. Polymerization of the polymerizable compounds preferably takes place with an applied voltage in case of PSA-VA, PSA-OCB, PSA-FFS and PSA-TN liquid crystal displays, and with or without, preferably without, an applied voltage in case of PSA-IPS displays.

Usually, UV photopolymerization in the PSA liquid crystal display production process is carried out through the following two steps.

In the first step (hereinafter also referred to as "UV1 step"), the liquid crystal composition is exposed to UV radiation emitted by a radiation source (hereinafter also referred to as "UV1 radiation"), while applying a voltage to the electrode structures, thereby generating a pre-tilt angle. A more preferred polymerizable compound should generate a smaller pre-tilt angle within the same time or a same pre-tilt angle within a shorter UV1 radiation time (*i.e*., a faster speed for forming pre-tilt angle), so as to improve production efficiency, shorten the tact time during batch production, and reduce costs. At the same time, the faster the speed for forming pre-tilt angle of the polymerizable compounds, the more favorable it is for the polymerizable compounds to achieve complete polymerization and thereby reduce polymer residue. In order to improve the speed for forming pre-tilt angle, shorter wavelength UV1 radiation is preferred, while in order to improve the voltage holding ratio (VHR), longer wavelength UV1 radiation is preferred. Therefore, it is usually difficult to balance a faster speed for forming pre-tilt angle with a higher voltage holding ratio.

In the second step (hereinafter also referred to as "UV2 step"), the liquid crystal composition is exposed to UV radiation (hereinafter also referred to as "UV2 radiation") without applying a voltage to the electrode structures, so as to ensure complete polymerization of any residual polymerizable compounds that did not polymerize in UV1 step. It is expected that after UV2 step, the change in pre-tilt angle should be minimized as much as possible to reduce the possibility of display unevenness in PSA type liquid crystal displays due to uneven conditions of the UV process (uneven external conditions such as light, heat, stress and the like). At the same time, the UV radiation intensity in UV2 step should be reduced to avoid or reduce negative effects such as reduced reliability or image stickiness.

In the current production of PSA type liquid crystal displays, a layer of polyimide (PI) alignment layer (referred to as PI alignment layer) needs to be coated on the glass substrate to achieve vertical alignment of liquid crystal molecules. However, this method has significant drawbacks (such as complex, complicated, and time-consuming PI coating process), as well as many other adverse effects, thereby greatly limiting the quality of liquid crystal displays. PI alignment process significantly reduces production efficiency and increases production costs. At the same time, due to the limited precision control ability of PI printing area, the deviation of PI printing area can affect the sealing performance of frame glue and the display effect of edges of narrow-bezel products, thereby greatly restricting the development of mainstream narrow-bezel products and substantially reducing the production yield. In existing technology, self-alignment agents are mainly added to liquid crystal compositions to replace the use of PI alignment layers, but not all liquid crystal compositions can achieve perfect compatibility with polymerizable compounds and self-alignment agents. For example, the pre-tilt angle formation rate during UV processes is too slow, requiring extended UV irradiation time to achieve the desired pre-tilt angle, thereby reducing production efficiency; polymers and self-aligning agents exhibit excessively fast polymerization rates and poor diffusion during UV polymerization, which can easily lead to explosive polymerization, thereby resulting in a large surface roughness of polymer layer and the formation of fragmented bright spots that impair display quality of the panel; after the UV1 and UV2 steps, there may be a high residue concentration of polymerizable compounds and self-alignment agents, resulting in issues such as deterioration of panel IS (image sticking). At the same time, poor mutual solubility between liquid crystal compositions, polymerizable compounds, and self-aligning agents can lead to precipitation of polymerizable compounds and self-aligning agents during storage, resulting in the failure of liquid crystal performance. Meanwhile, weak rigidity of polymer networks formed after polymerization may cause structural changes in the structure of the polymer networks when PSA type liquid crystal display elements continuously display the same pattern for a long time, which in turn alter the pre-tilt angle of liquid crystal molecules and cause display defects. Large liquid crystal contact angle of the self-aligning agents slow down liquid crystal diffusion in the ODF (One Drop Filling) processes, which result in uneven concentration distribution of the self-aligning agents within the panels, thereby leading to non-uniform alignment effect or poor alignment effect in the panel corner area, ultimately causing display defects.

Furthermore, with the development of display technology, the liquid crystal display industry has more stringent requirements for the display quality of liquid crystal display, especially in the TV industry, with the general increase of the size of TVs, the LCD generation line has been increased. The difficulty of manufacturing the large-size LCD panels has also been significantly increased. Therefore, how to ensure the display quality is an urgent problem to be solved. Meanwhile, in addition to continuously optimizing the panel manufacturing process, the development of liquid crystal materials is also one of the means. Especially for PSA liquid crystal displays, the selection of liquid crystal compositions used in combination with polymerizable compounds has become a hot spot of the research.

Therefore, the research focus in the art is to develop a self-aligning agent with a fast polymerization speed, a controllable polymerization process, and a good comprehensive performance to meet the needs of PSA type liquid crystal display elements, as well as to provide a display technology that can achieve vertical alignment of liquid crystal molecules without the need for a PI alignment layer.

### Summary of the Invention

**Objectives of Invention:** The objective of the present invention is to provide a self-aligning agent of general formula O, when used in the liquid crystal composition, the liquid crystal composition comprising the self-aligning agent of general formula O may have a lower residue concentration, a smaller roughness, a better alignment effect as well as a better low-temperature storage stability and a better pre-tilt angle stability while maintaining an appropriate clearing point, an appropriate optical anisotropy, an appropriate absolute value of dielectric anisotropy, a larger K value (K₁₁ and K₃₃) and a smaller rotational viscosity.

The objective of the present invention is also to provide a liquid crystal composition comprising the above self-aligning agent.

Further, the objective of the present invention is also to provide a liquid crystal display device comprising the above liquid crystal composition.

**Technical solutions:** To realize the above invention objects, the present invention provides a self-aligning agent of general formula O 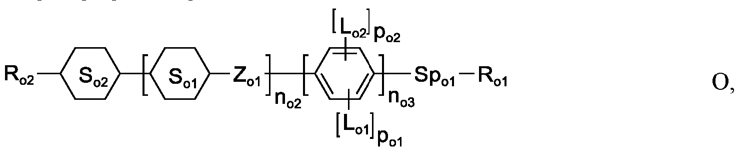 wherein,
Rₒ₂ represents -Spₒ₂-Pₒ₁, -H, C₁₋₁₂ (for example, may be C₂, C₃, C₄, C₅, C₆, C₇, C₈, C₉, C₁₀ or C₁₁ linear alkyl, C₃₋₁₂ (for example, may be C₃, C₄, C₅, C₆, C₇, C₈, C₉, C₁₀ or C₁₁) branched alkyl, 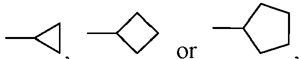 wherein one or more than two nonadiacent -CH₂- in the C₁₋₁₂ linear alkyl, 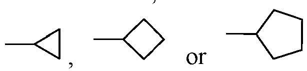 can each be independently replaced by -CH=CH-, -C≡C-, -O-, -CO-, -CO-O- or -O-CO-, and one or more -H in the C₁₋₁₂ linear alkyl can each be independently substituted by -F or -Cl;
ring 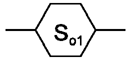 represents 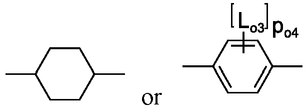 , wherein one or more -CH₂-in 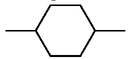 can be replaced by -O-, and one or at most two single bonds in the rings can be replaced by double bond;
ring 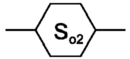 represents 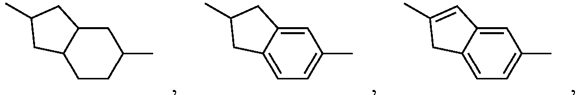 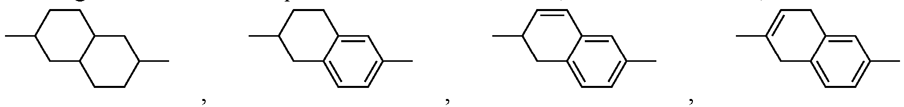 or 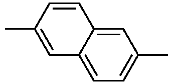 , wherein one or more than two nonadjacent -CH₂- in the above groups can each be independently replaced by -O- or -S-, and one or more -H on the above groups can each be independently substituted by -F or C₁₋₅ halogenated or unhalogenated linear alkyl; Lₒ₁ and Lₒ₃ each independently represents -F, -Cl, -CN, -NO₂, -NCO, -NCS, -OCN, -SCN, -C(O)N(R^{o0})₂, -C(O)R^{o0}, C₁₋₁₂ for example, may be C₂, C₃, C₄, C₅, C₆, C₇, C₈, C₉, C₁₀ or C₁₁) linear alkyl, C₃₋₁₂ for example, may be C₃, C₄ C_{5,} C₆ C₇, C₈ C₉, C₁₀ or C₁₁) branched alkyl, 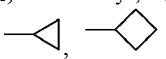 or 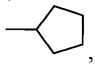 wherein one or more than two nonadjacent -CH₂- in the C₁₋₁₂ linear alkyl, 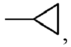 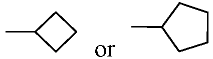 can each be independently replaced by -CH=CH-, -C≡C-, -O-, -CO-, -CO-O- or -O-CO-, and one or more -H in the C₁₋₁₂ linear alkyl can each be independently substituted by -F, wherein R^{o0} represents C₁₋₁₂ (for example, C₁, C₂, C₃, C₄, C₅, C₆, C₇, C₈, C₉, C₁₀, C₁₁ or C₁₂) linear alkyl or C₃₋₁₂ (for example, C₃, C₄, C₅, C₆, C₇, C₈, C₉, C₁₀, C₁₁ or C₁₂) branched alkyl;
Lₒ₂ represents -Spₒ₃-Pₒ₂ or 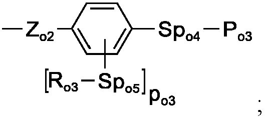
Rₒ₁ and Rₒ₃ each independently represents an anchoring group, the anchoring group is 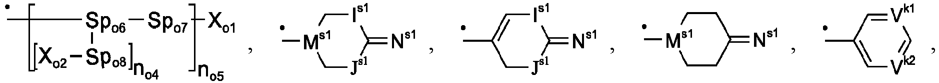 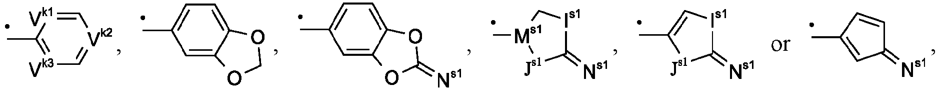 wherein 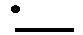 represents the binding site in the bound structure;
nₒ₄ represents 1 or 2, wherein when nₒ₄ represents 2, -Spₒ₈-Xₒ₂ can be the same or different;
nₒ₅ represents 0 or 1;
M^{S1} represents 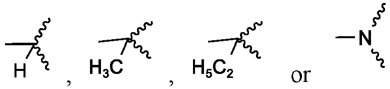 , wherein, 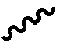 represents the binding site between M^{S1} and -CH₂- in the six-membered ring;
I^{S1} and J^{S1} each independently represents -CH₂-, -O- or -S-;
N^{S1} represents =O or =S;
V^{K1}, V^{K2} and V^{K3} each independently represents -CH= or -N=;
Xₒ₁ and Xₒ₂ each independently represents -H, -OH, -SH, -NH₂, -NHR¹¹, -N(R¹¹)₂, -NHC(O)R¹¹, -OR¹¹, -C(O)OH, C₁₋₁₂ (for example C₁, C₂, C₃, C₄, C₅, C₆, C₇, C₈, C₉, C₁₀, C₁₁ or C₁₂) linear halogenated alkyl or C₃₋₁₂ (for example, C₃, C₄, C₅, C₆, C₇, C₈, C₉, C₁₀, C₁₁ or C₁₂) branched halogenated or unhalogenated alkyl, wherein at leasr one of Xₒ₁ and Xₒ₂ is selected from a group consisting of -OH, -SH, -NH₂, -NHR¹¹, -C(O)OH and -CHO, wherein R¹¹ represents C₁₋₁₂ (for example, C₁, C₂, C₃, C₄, C₅, C₆, C₇, C₈, C₉, C₁₀, C₁₁ or C₁₂) linear alkyl or C₃₋₁₂ (for example, C₃, C₄, C₅, C₆, C₇, C₈, C₉, C₁₀, C₁₁ or C₁₂) branched alkyl;
Pₒ₁, Pₒ₂ and Pₒ₃ each independently represents polymerizable group;
Spₒ₁, Spₒ₂, Spₒ₃, Spₒ₄, Spₒ₅, Spₒ₇ and Spₒ₈ each independently represents spacer group or single bond;
Spₒ₆ represents 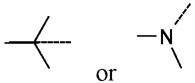 , wherein, 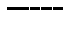 represents the binding site to Spₒ₇ or Spₒ₈;
Zₒ₁ and Zₒ₂ each independently represents -O-, -S-, -CO-, -CO-O-, -O-CO-, -O-CO-O-, -CH₂O-, -OCH₂-, -CH₂S-, -SCH₂-, -CF₂O-, -OCF₂-, -CF₂S-, -SCF₂-, -(CH₂)_{d}-, -CF₂CH₂-, -CH₂CF₂-, -(CF₂)_{d}-, -CH=CH-, -CF=CF-, -CH=CF-, -CF=CH-, -C≡C-, -CH=CH-CO-O-, -O-CO-CH=CH-, -CH₂CH₂-CO-O-, -O-CO-CH₂CH₂-, -CHR¹-, -CR¹R²- or single bond, wherein R¹ and R² each independently represents C₁₋₁₂ linear alkyl or C₃₋₁₂ branched alkyl, and d represents an integer of 1-4;
pₒ₁, pₒ₂, pₒ₃ and pₒ₄ each independently represents 0, 1 or 2, wherein when pₒ₁ represents 2, Lₒ₁ can be the same or different, wherein when pₒ₂ represents 2, Lₒ₂ can be the same or different; wherein when pₒ₃ represents 2, -SPₒ₅-Rₒ₃ can be the same or different; wherein when pₒ₄ represents 2, Lₒ₃ can be the same or different; and
nₒ₂ represents 0, 1, 2 or 3, nₒ₃ represents 1, 2 or 3, wherein when nₒ₂ represents 2 or 3, 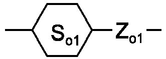 can be the same or different, wherein when nₒ₃ represents 2 or 3, 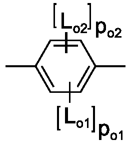 can be the same or different.

In some embodiments of the present invention, the self-aligning agent of general formula O is selected from a group consisting of the following compounds: 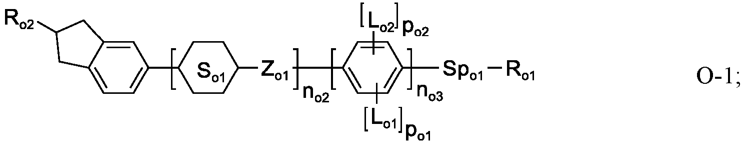 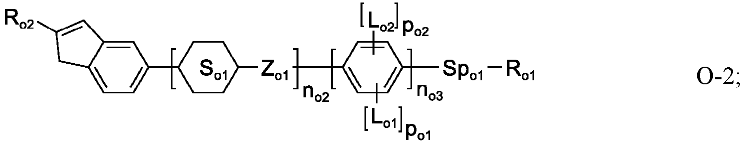 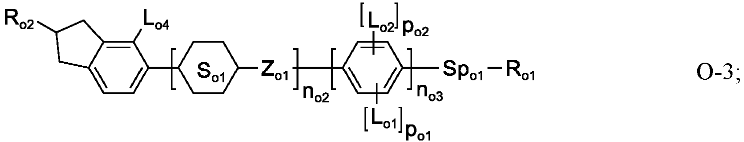 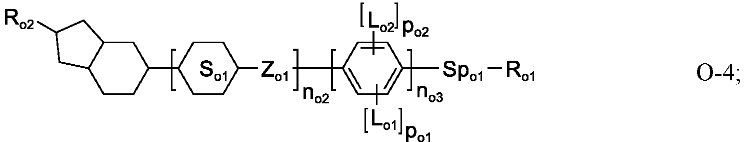 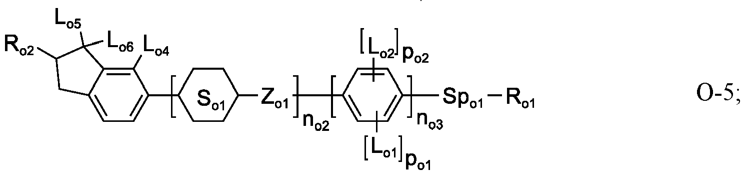 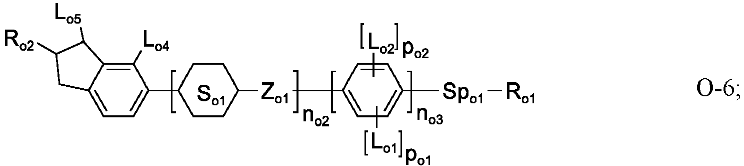 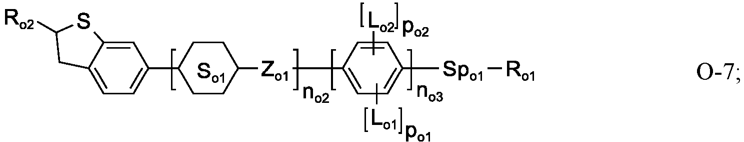 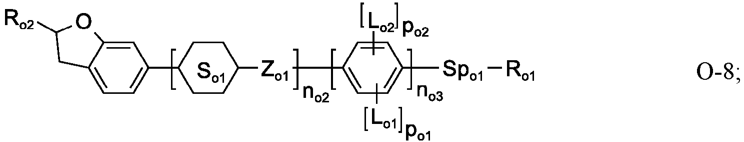 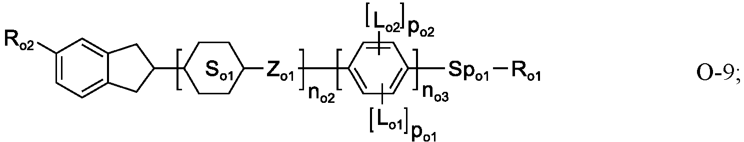 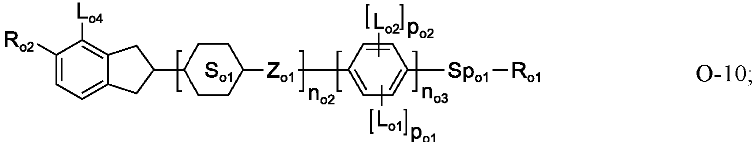 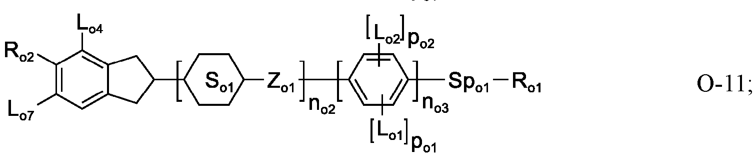 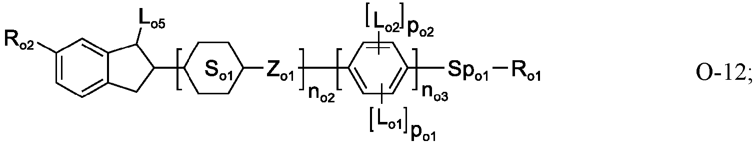 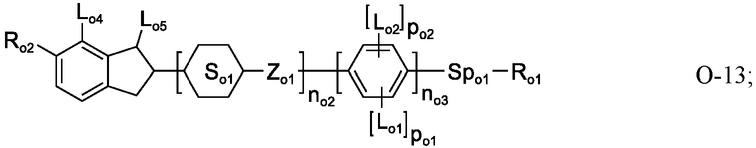 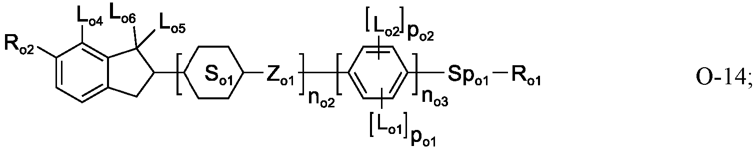 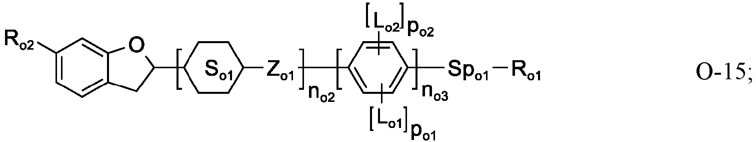 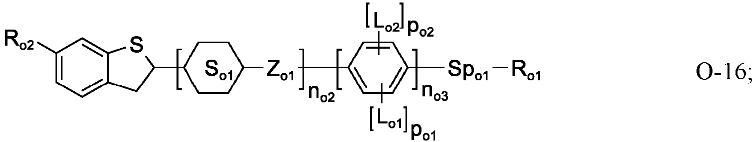 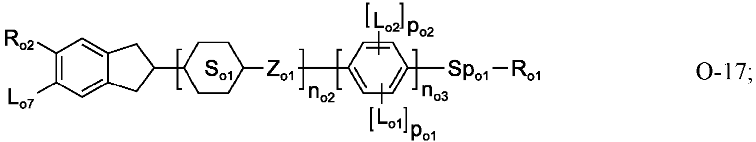 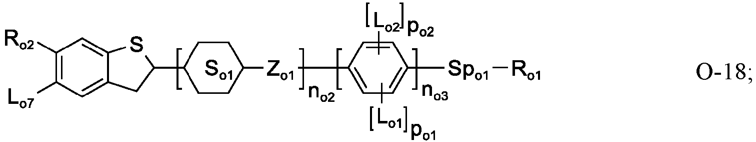 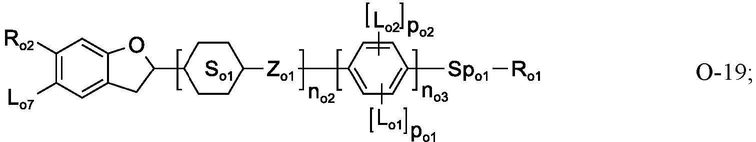 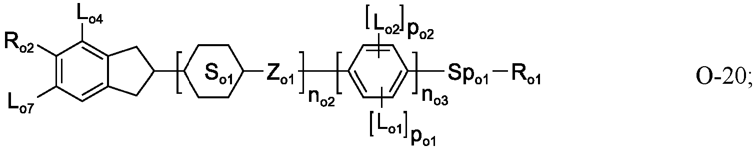 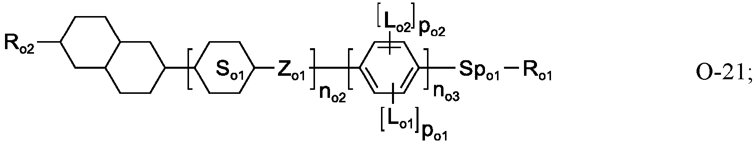 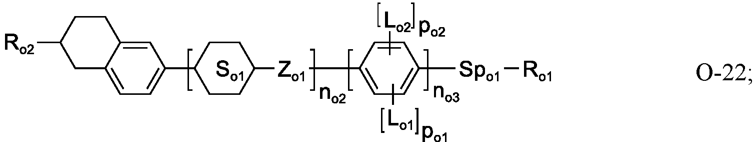 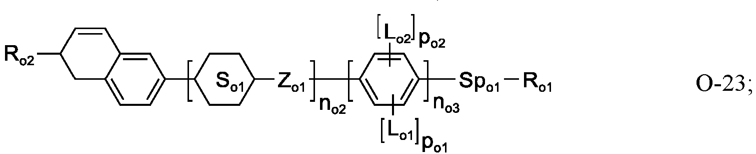 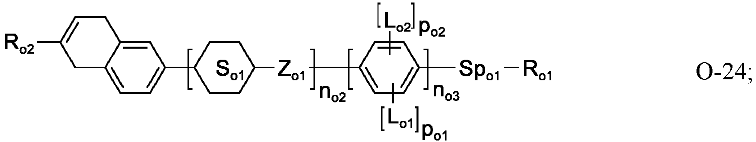 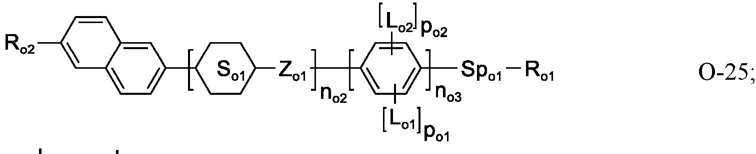 and 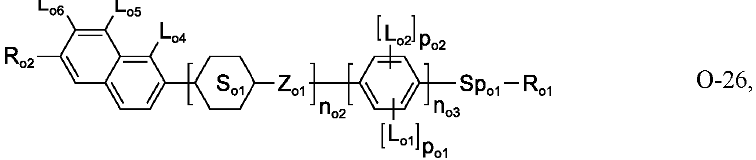 wherein,
Lₒ₄~Lₒ₇ each independently represents -F or C₁₋₅ (for example, may be C₂, C₃, C₄) halogenated or unhalogenated linear alkyl.

In the present invention, through a large number of experiments, it is found that, when the leftmost ring in the main structure of the self-aligning agent is 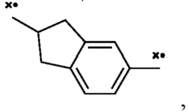 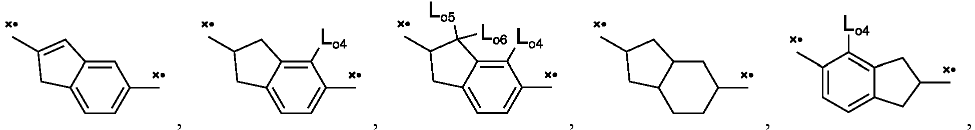 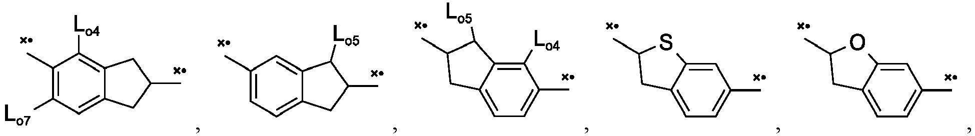 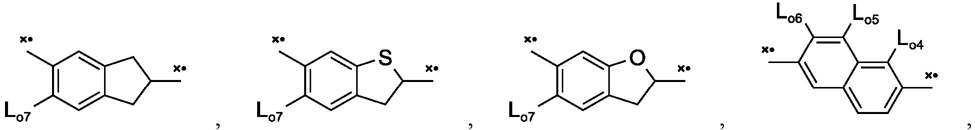 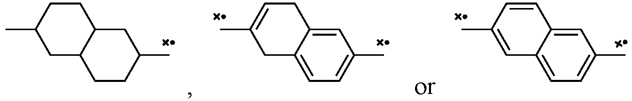 (wherein × ˙ represents the binding site in the bound structure), the use of such self-aligning agent in the liquid crystal composition will make the liquid crystal composition have a lower residue concentration, a smaller roughness and a better alignment effect relative to the self-aligning agent wherein the leftmost ring in the main structure is 3-6 cycloalkyls, particularly when the leftmost ring in the main structure of the self-aligning agent is 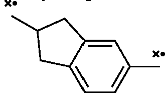 , the liquid crystal composition comprising such self-aligning agent has a lower residue concentration, a smaller roughness and a better alignment effect.

In some embodiments of the present invention, the self-aligning agent of general formula O-1 is selected from a group consisting of the following compounds: 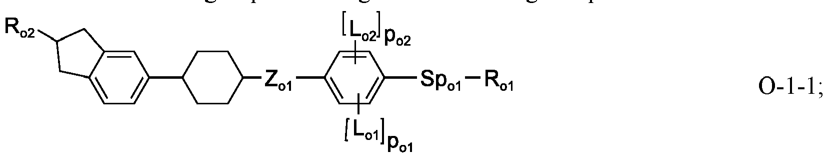 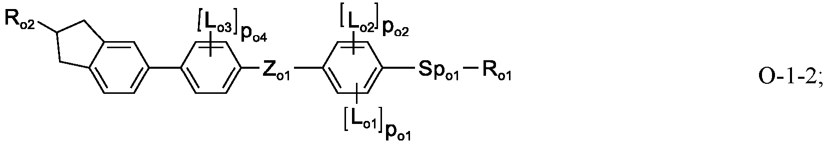 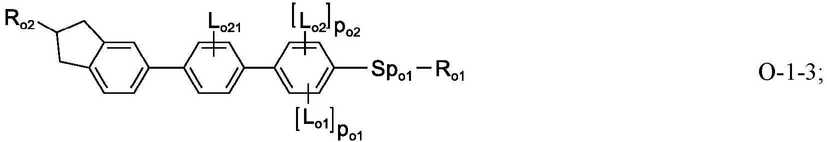 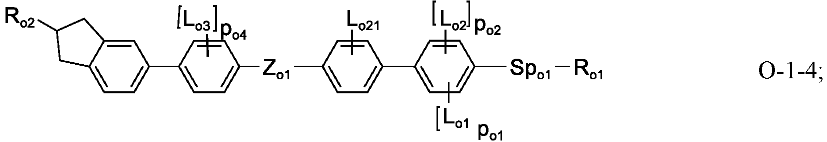 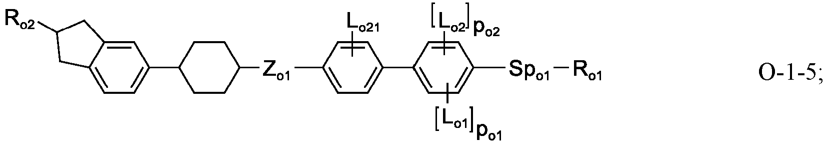 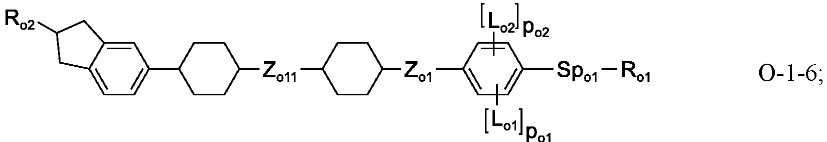 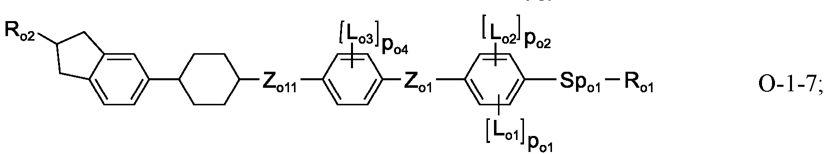 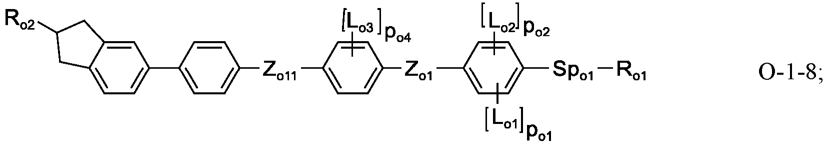 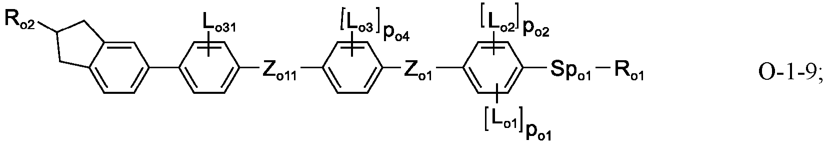 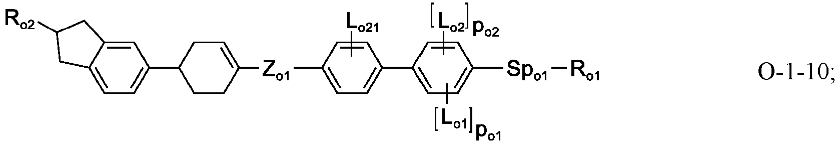 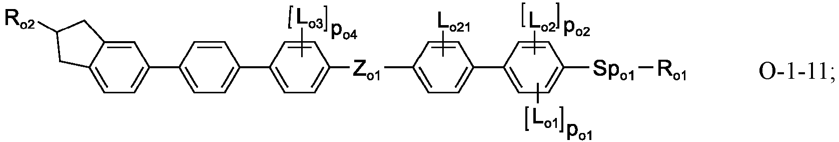 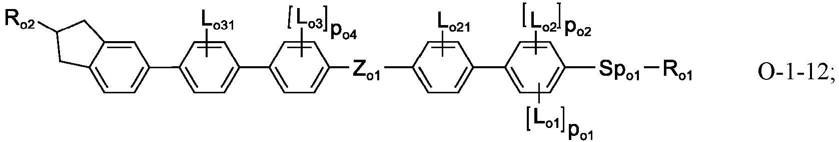 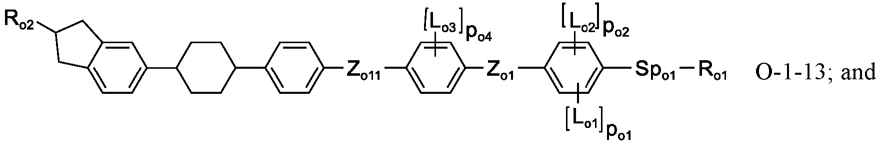 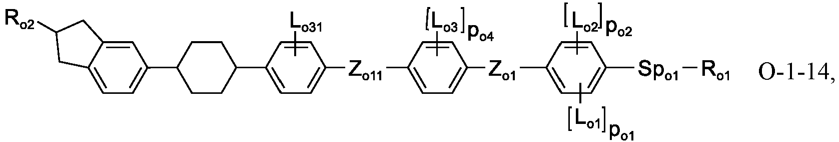 wherein,
Zₒ₁₁ represents -O-, -S-, -CO-, -CO-O-, -O-CO-, -O-CO-O-, -CH₂O-, -OCH₂-, -CH₂S-, -SCH₂-, -CF₂O-, -OCF₂-, -CF₂S-, -SCF₂-, -(CH₂)_{d}-, -CF₂CH₂-, -CH₂CF₂-, -(CF₂)_{d}-, -CH=CH-, -CF=CF-, -CH=CF-, -CF=CH-, -C≡C-, -CH=CH-CO-O-, -O-CO-CH=CH-, -CH₂CH₂-CO-O-, -O-CO-CH₂CH₂-, -CHR¹-, -CR¹R²- or single bond, wherein R¹ and R² each independently represents C₁₋₁₂ linear alkyl or C₃₋₁₂ branched alkyl, and d represents an integer of 1-4;
Lₒ₃₁ represents -F, -Cl, -CN, -NO₂, -NCO, -NCS, -OCN, -SCN, -C(O)N(R^{o0})₂, -C(O)R^{o0}, C₁₋₁₂ (for example, may be C₂, C₃, C₄, C₅, C₆, C₇, C₈, C₉, C₁₀ or C₁₁) linear alkyl, C₃₋₁₂ (for example, may be C₃, C₄, C₅, C₆, C₇, C₈, C₉, C₁₀ or C₁₁) branched alkyl, 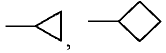 or 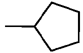 , wherein one or more than two nonadjacent -CH₂- in the C₁₋₁₂ linear alkyl, 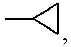 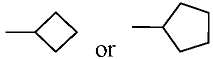 can each be independently replaced by -CH=CH-, -C≡C-, -O-, -CO-, -CO-O- or -O-CO-, and one or more -H in the C₁₋₁₂ linear alkyl can each be independently substituted by -F, wherein R^{o0} represents C₁₋₁₂ (for example, C₁, C₂, C₃, C₄, C₅, C₆, C₇, C₈, C₉, C₁₀, C₁₁ or C₁₂) linear alkyl or C₃₋₁₂ (for example, C₃, C₄, C₅, C₆, C₇, C₈, C₉, C₁₀, C₁₁ or C₁₂) branched alkyl; and
Lₒ₂₁ represents -Spₒ₃-Pₒ₂ or

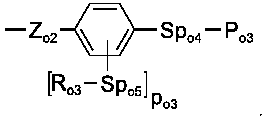

In some embodiments of the present invention, the self-aligning agent of general formula O-1 is selected from a group consisting of the following compounds: 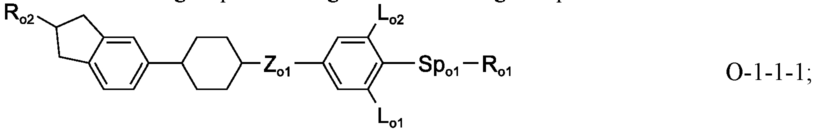 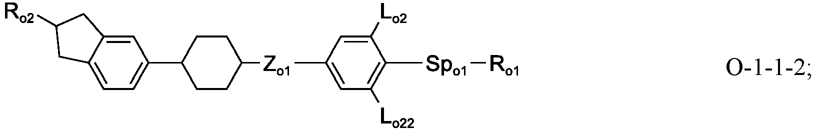 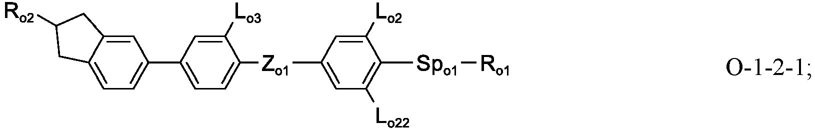 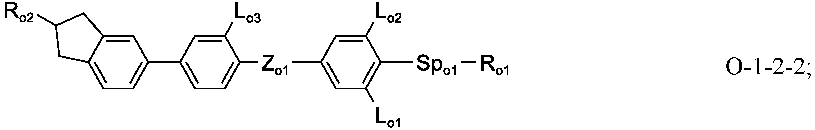 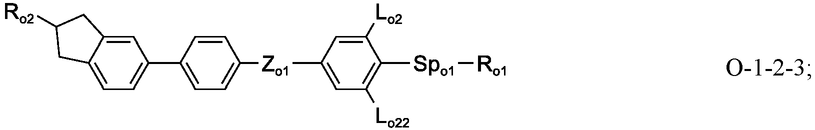 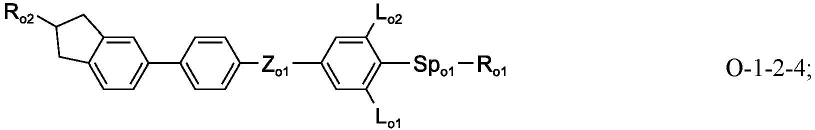 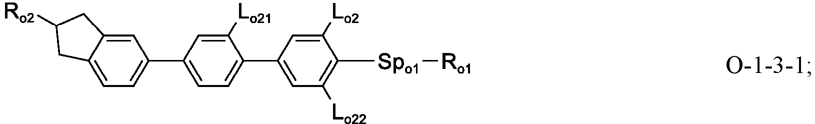 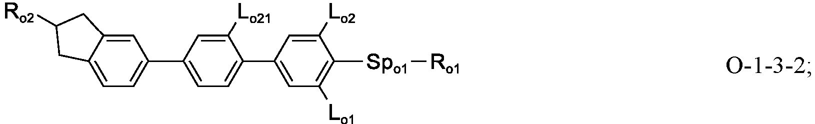 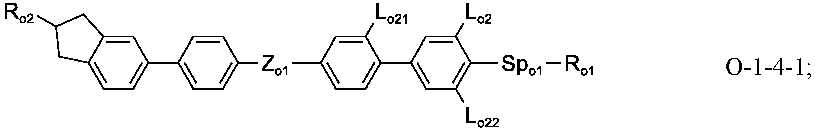 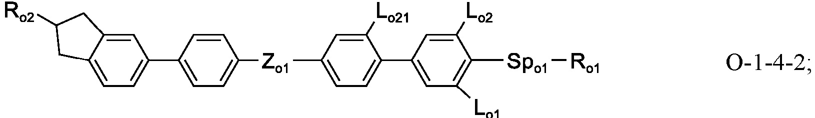 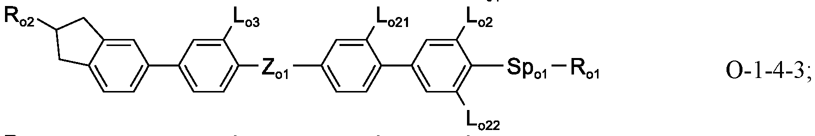 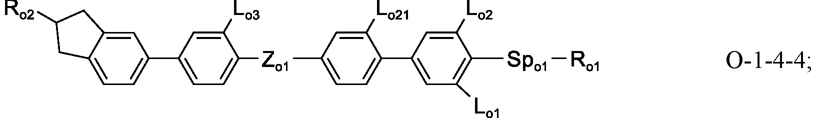 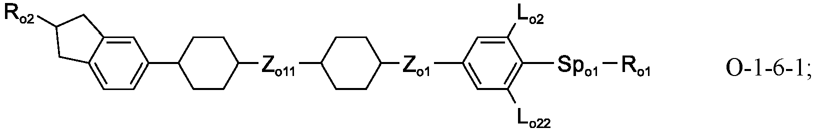 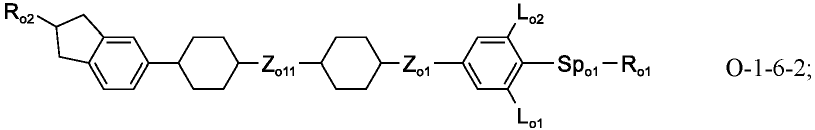 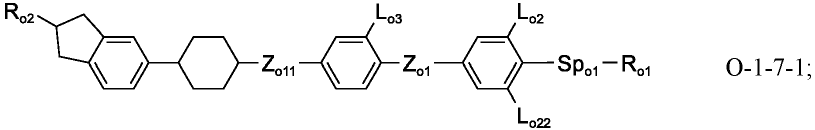 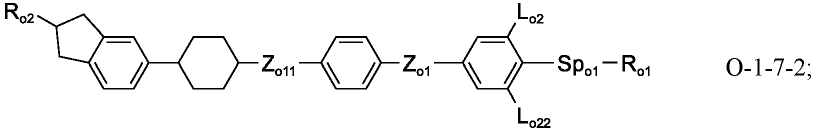 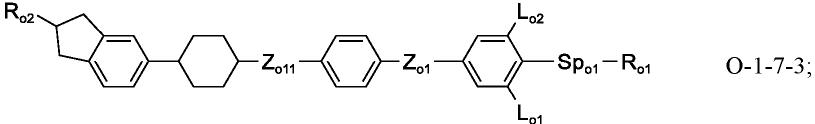 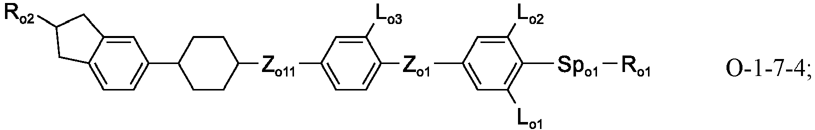 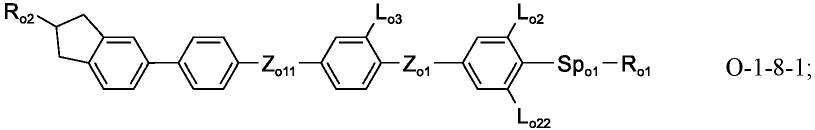 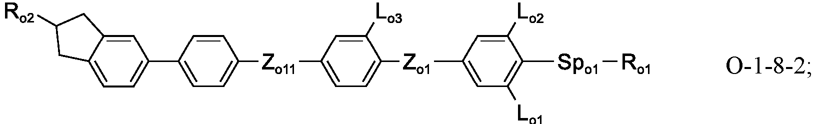 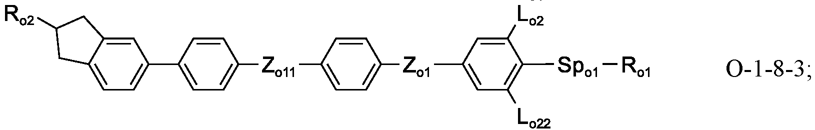 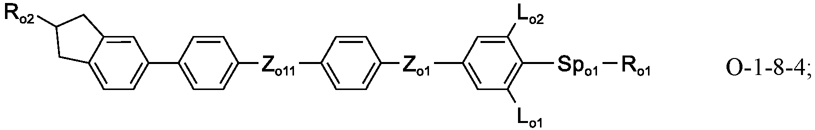 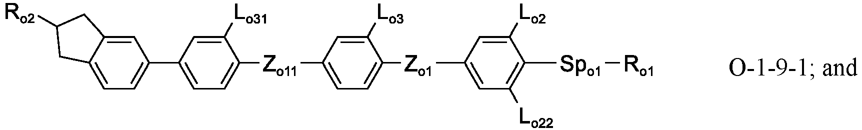 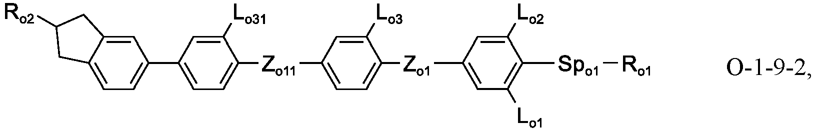 wherein,
Lₒ₂₂ represents -Spₒ₃-Pₒ₂ or

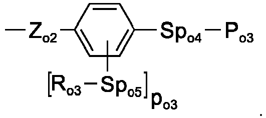

In some embodiments of the present invention, Rₒ₂ represents -H, C₁₋₁₂ linear alkyl, C₁₋₁₁ linear alkoxy, C₂₋₁₂ alkenyl,

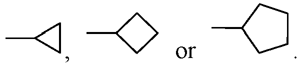

In some embodiments of the present invention, Lₒ₁, Lₒ₃ and Lₒ₃₁ each independently represents -F, -Cl, C₁₋₁₂ linear alkyl, C₁₋₁₁ linear alkoxy, C₂₋₁₂ linear alkenyl, 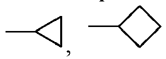 or

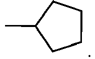

In some embodiments of the present invention, the polymerizable groups Pₒ₁, Pₒ₂ and Pₒ₃ each independently represents 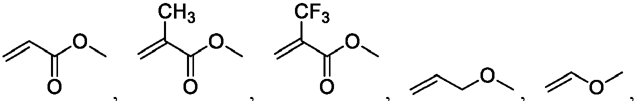 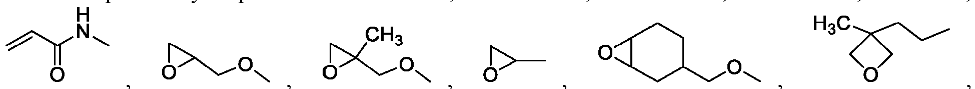 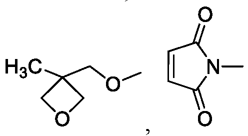 or -SH; preferably, the polymerizable groups Pₒ₁, Pₒ₂ and Pₒ₃ each independently represents 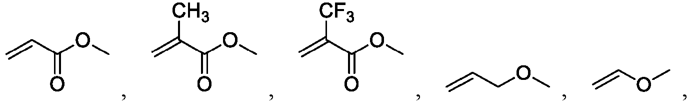 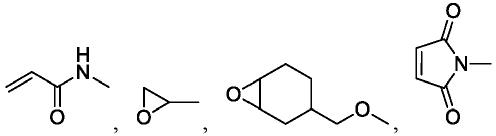 or -SH; further preferably, the polymerizable groups Pₒ₁, Pₒ₂ and Pₒ₃ each independently represents

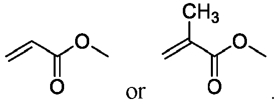

In some embodiments of the present invention, Lₒ₂ and Lₒ₂₂ each independently represents 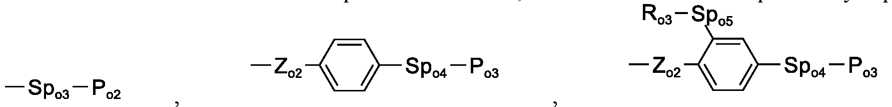 or

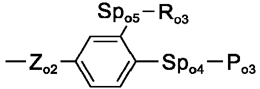

In some embodiments of the present invention, Zₒ₂ represents single bond.

In some embodiments of the present invention, Spₒ₁, Spₒ₂, Spₒ₃, Spₒ₄, Spₒ₅, Spₒ₇ and Spₒ₈ each independently represents -(CH₂)ₚ₁-, -(CH₂)ₚ₁-O-, -O-(CH₂)ₚ₁-, -(CH₂)ₚ₁-O-CO-, -(CH₂)ₚ₁-CO-O-, -(CH₂)ₚ₁-O-CO-O-, -CR⁰R⁰⁰-(CH₂)ₚ₁- or single bond, wherein, p₁ represents an integer of 1-10 (for example, 2, 3, 4, 5, 6, 7, 8 or 9), R⁰ and R⁰⁰ each independently represents -H, C₁₋₁₀ linear alkyl, C₃₋₁₀ branched alkyl or C₃₋₁₀ cycloalkyl.

In some embodiments of the present invention, Spₒ₁, Spₒ₃, Spₒ₄ and Spₒ₅ each independently represents -(CH₂)ₚ₁- or -(CH₂)ₚ₁-O-.

In some embodiments of the present invention, Rₒ₁ and Rₒ₃ each is independently selected from a group consisting of the following groups: 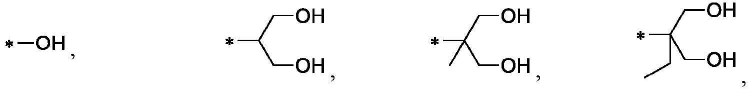 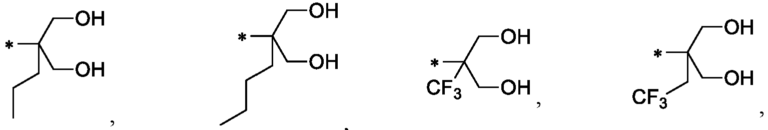 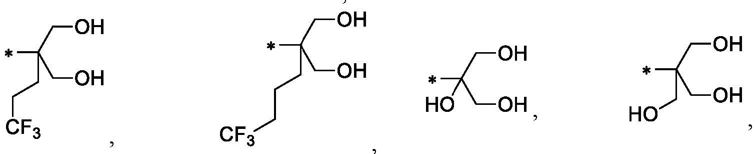 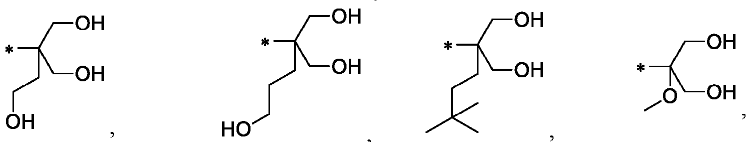 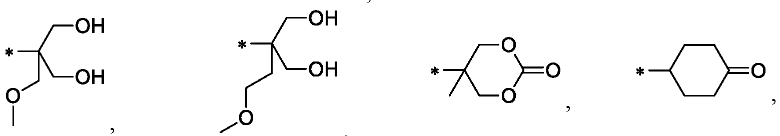 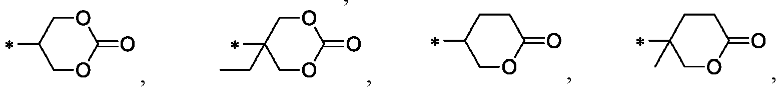 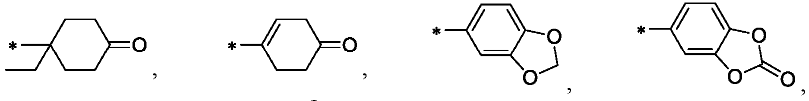 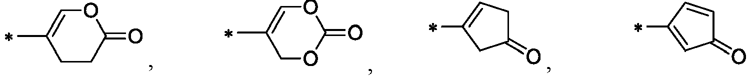 and 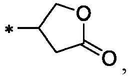 wherein, * represents the binding site in the bound structure.

In some embodiments of the present invention, in order to achieve an appropriate clearing point, an appropriate optical anisotropy, an appropriate absolute value of dielectric anisotropy, a larger K value, a smaller rotational viscosity, a lower residue concentration, a smaller roughness and a better alignment effect, Rₒ₁ and Rₒ₃ each is independently selected from a group consisting of the following groups: 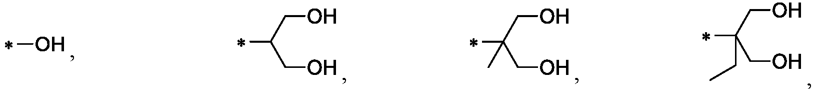 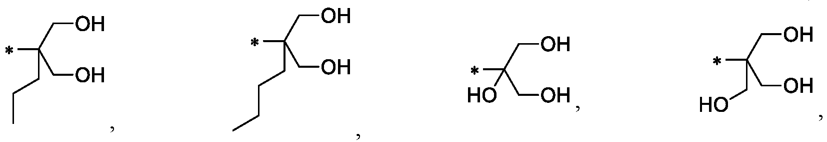 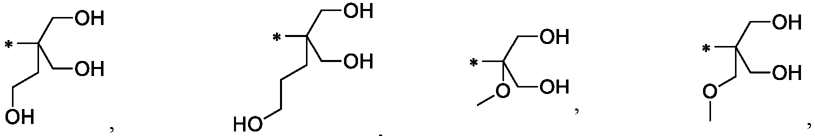 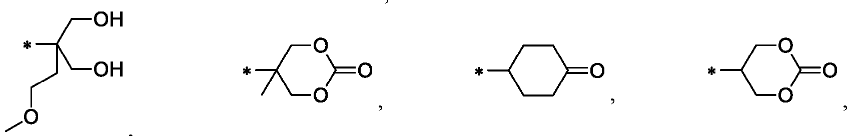 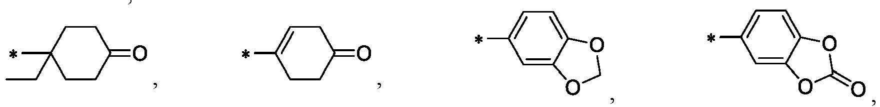 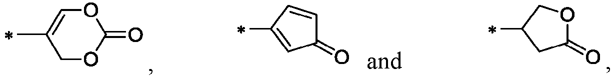 wherein, * represents the binding site in the bound structure.

In some embodiments of the present invention, preferably, Rₒ₁ and Rₒ₃ each is independently selected from: 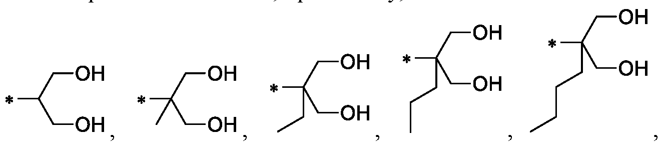 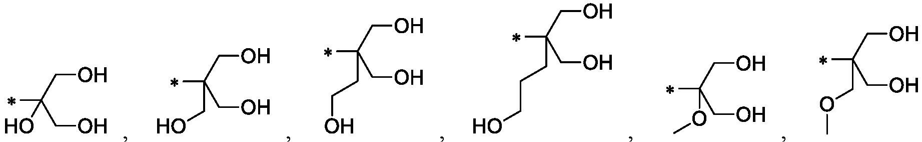 or 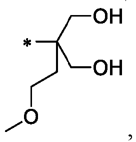 wherein, * represents the binding site in the bound structure.

In some embodiments of the present invention, the compound of general formula O is selected from a group consisting of the compound of general formula O-1-2-1, the compound of general formula 0-1-2-3, and the compound of general formula 0-1-6-2.

In some embodiments of the present invention, the self-aligning agent of general formula 0-1-1-2 is selected from a group consisting of the following compounds:

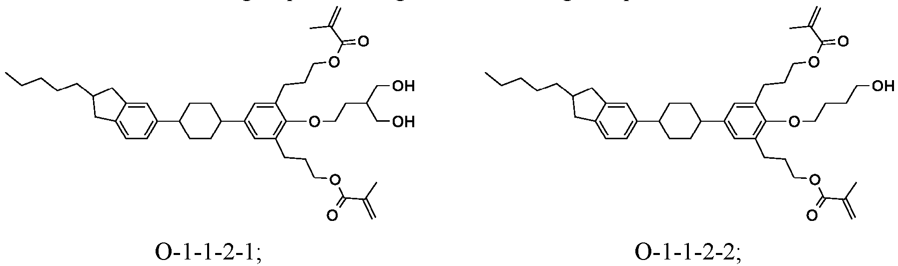

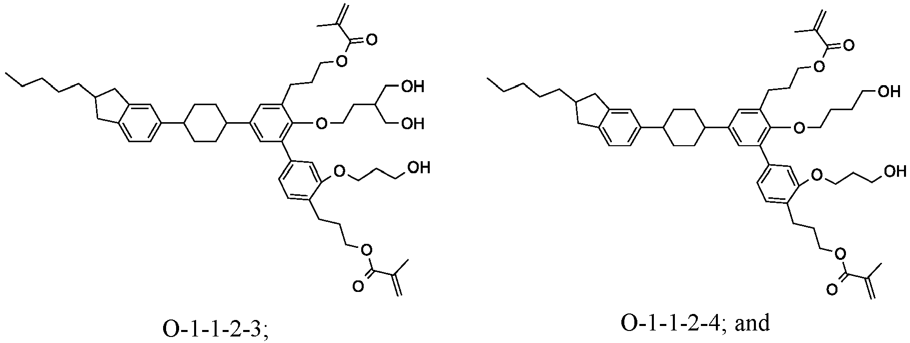

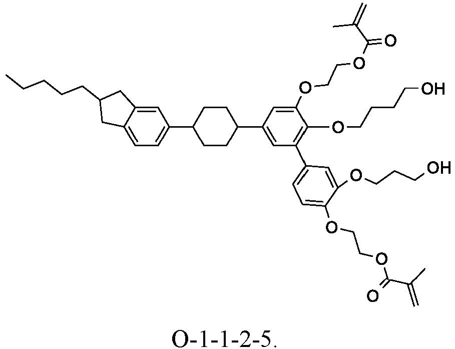

In some embodiments of the present invention, the self-aligning agent of general formula 0-1-2-1 is selected from a group consisting of the following compounds:

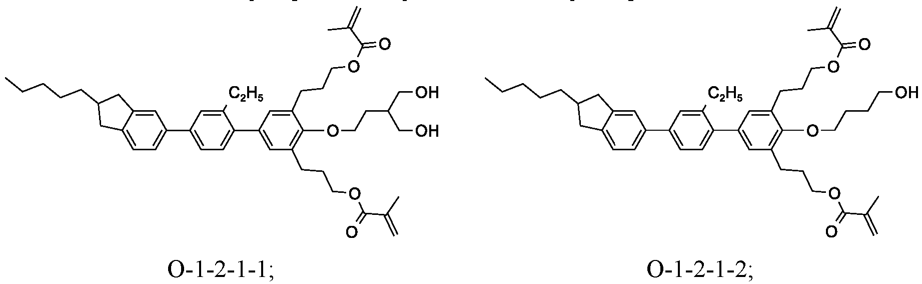

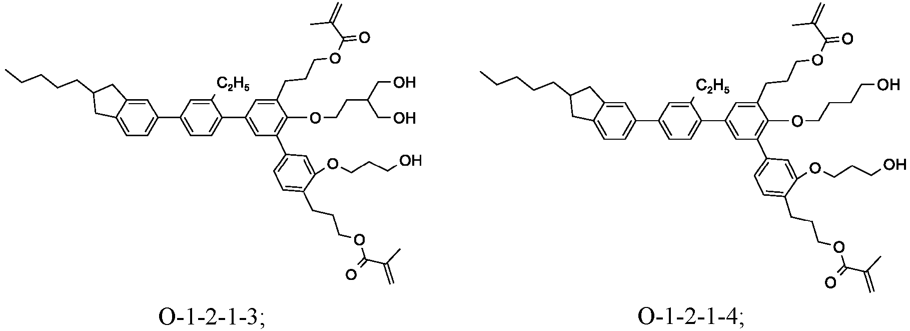

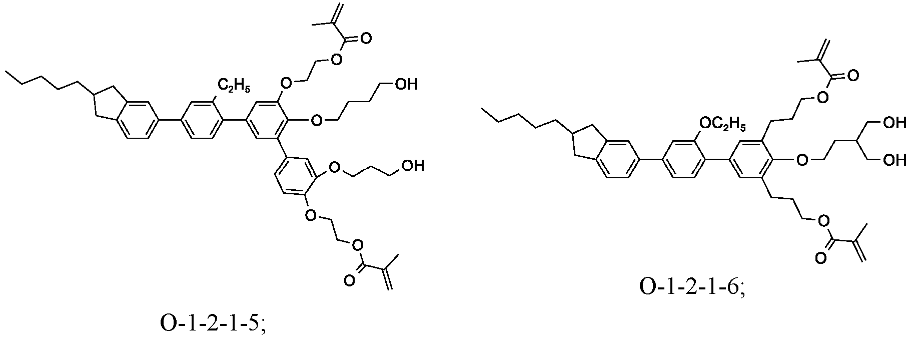

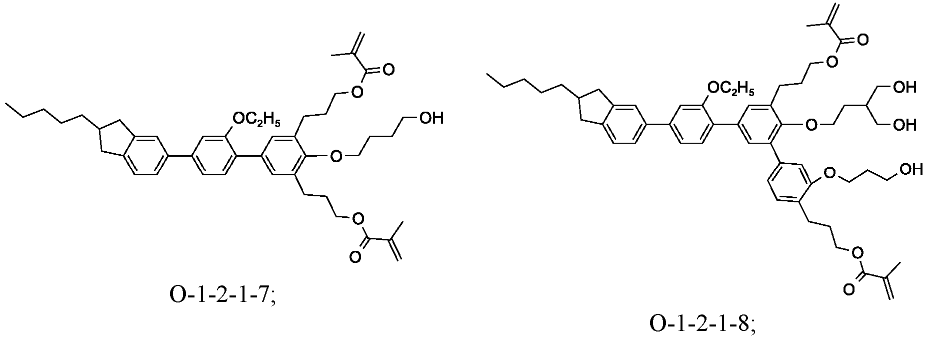

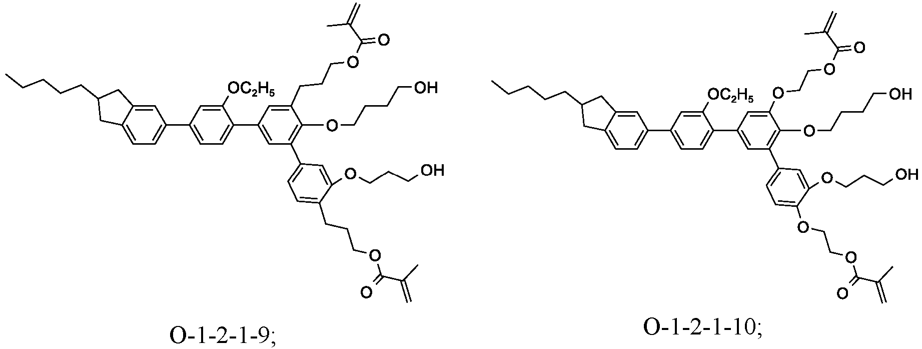

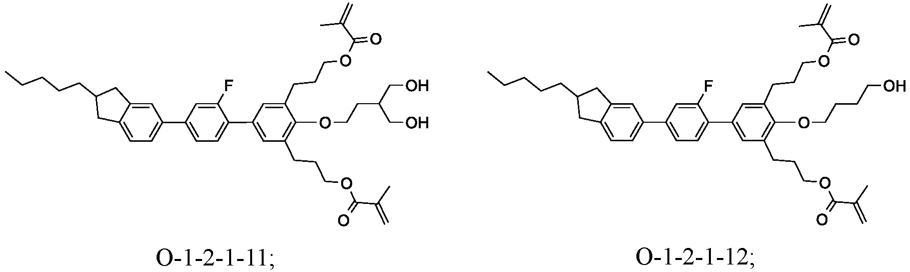

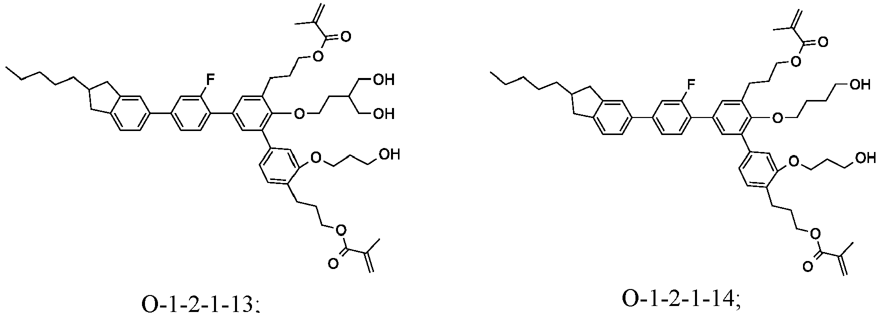

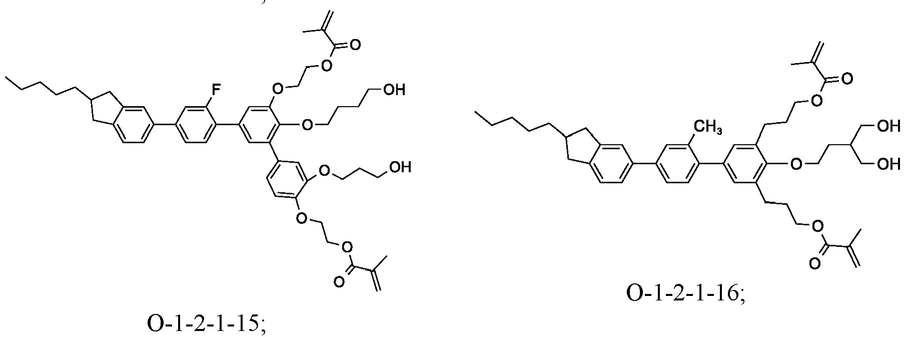

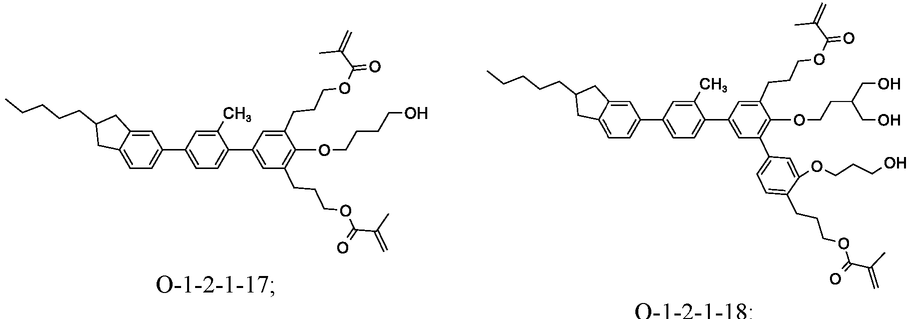

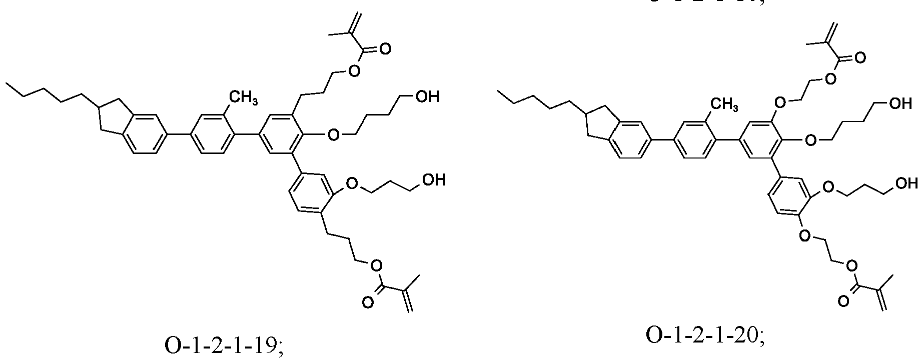

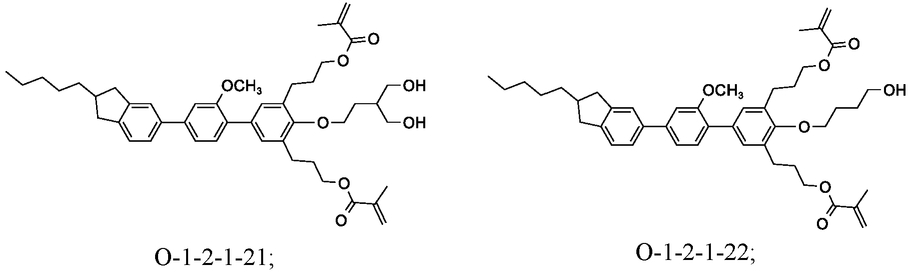

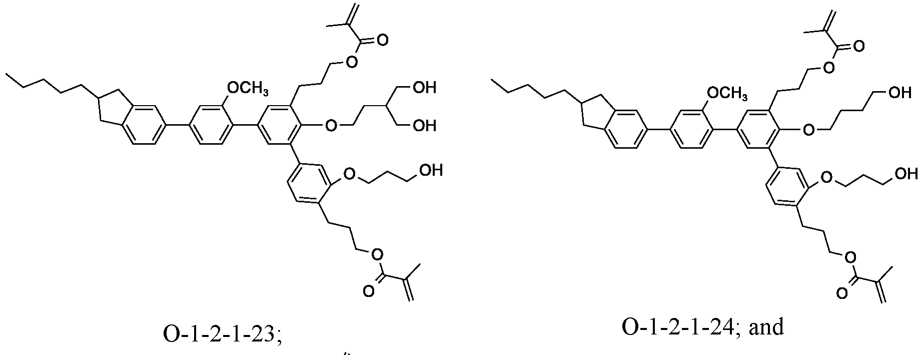

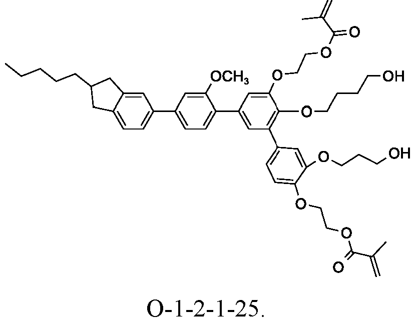

In some embodiments of the present invention, the compound of general formula O-1-2-3 is selected from a group consisting of the following compounds:

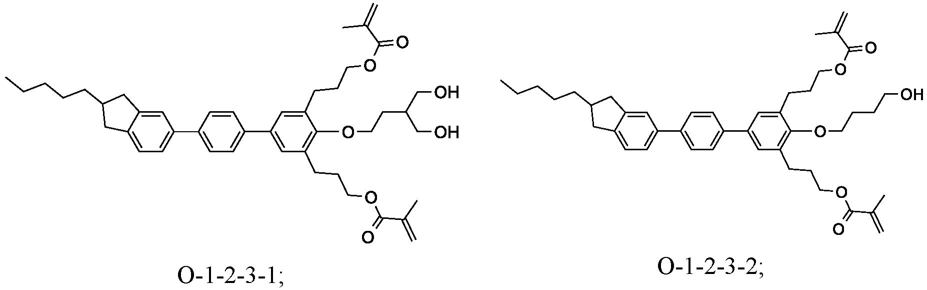

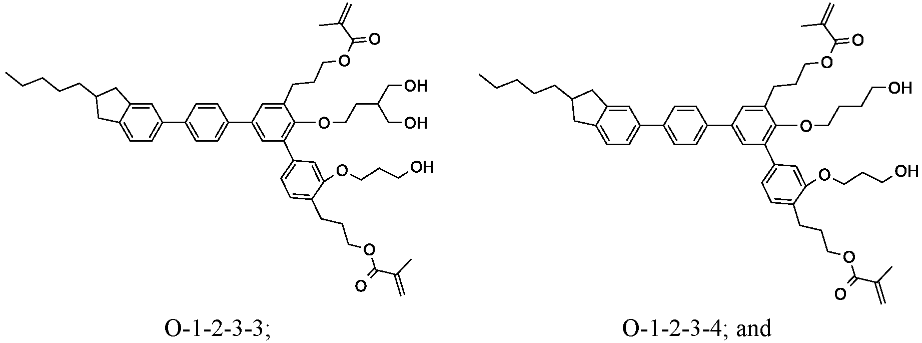

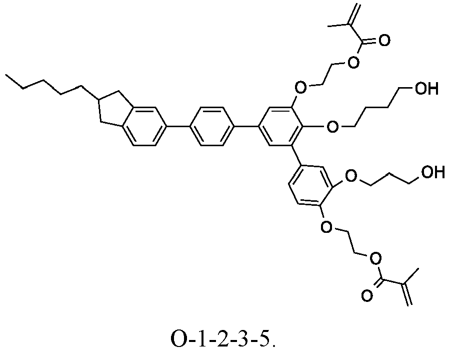

In some embodiments of the present invention, the compound of general formula O is selected from a group consisting of the compound of general formula O-1-2-1-1, the compound of general formula O-1-2-1-11, the compound of general formula O-1-2-3-1, the compound of general formula 0-1-2-3-4 and the compound of general formula 0-1-6-2.

Another aspect of the present invention provides a liquid crystal composition comprising the self-aligning agent of general formula O.

In some embodiments of the present invention, it is preferred to adjust the content of the compound of general formula O, such that the liquid crystal composition of the present invention has a smaller polymer residue, a smaller roughness and a better alignment effect.

In some embodiments of the present invention, the compound of general formula O provides (in percentage by weight) 0.001%~5% (including any of the numerical values or sub-ranges therebetween) of the liquid crystal composition, for example, 0.001%, 0.005%, 0.05%, 0.1%, 0.2%, 0.25%, 0.3%, 0.35%, 0.4%, 0.45%, 0.5%, 0.55%, 0.6%, 0.65%, 0.7%, 0.75%, 0.8%, 0.85%, 0.9%, 0.95%, 1.0%, 2%, 3%, 4%, 5%, or a range between any two numerical values therein; preferably, the compound of general formula O provides (in percentage by weight) 0.1%~2% of the liquid crystal composition.

In the present invention, when the self-aligning agent of general formula O is added into the liquid crystal composition, it allows liquid crystal molecules in the liquid crystal composition of the present invention to orient without PI alignment layer, such that the liquid crystal composition comprising the same has a lower residue concentration, a smaller roughness and a better alignment effect.

In some embodiments of the present invention, the liquid crystal composition comprises at least one compound of general formula M: 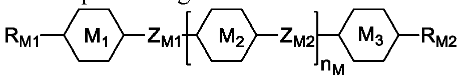 M, wherein,
R_{M1} and R_{M2} each independently represents C₁₋₁₂ (for example, C₂, C₃, C₄, C₅, C₆, C₇, C₈, C₉, C₁₀ or C₁₁) linear alkyl, C₃₋₁₂ (for example, C₄, C₅, C₆, C₇, C₈, C₉, C₁₀ or C₁₁) branched alkyl, 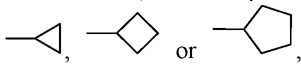 wherein one or more than two nonadjacent -CH₂- in the C₁₋₁₂ linear alkyl or C₃₋₁₂ branched alkyl can each be independently replaced by -CH=CH-, -C≡C-, -O-, -CO-, -CO-O- or -O-CO-; 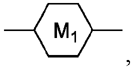 ring 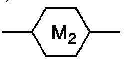 and ring 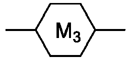 each independently represents 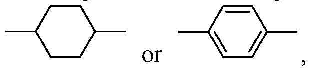 wherein one or more -CH₂- on 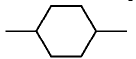 can be replaced by -O-, one or at most two sing bonds in the ring can be replaced by double bond, at most one -H on 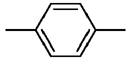 can be substituted by halo;
Z_{M1} and Z_{M2} each independently represents single bond, -CO-O-, -O-CO-, -CH₂O-, -OCH₂-, -C≡C-. -CH=CH-, -CH₂CH₂- or -(CH₂)₄-; and n_{M} represents 0, 1 or 2, wherein when n_{M} = 2, ring 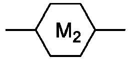 can be the same or different, Z_{M2} can be the same or different.

In some embodiments of the present invention, preferably, R_{M1} and R_{M2} each independently represents C₁₋₁₀ linear alkyl, C₃₋₁₀ branched alkyl, C₁₋₉ linear alkoxy, C₃₋₉ branched alkoxy, C₂₋₁₀ linear alkenyl or C₄₋₁₀ branched alkenyl; further preferably, R_{M1} and R_{M2} each independently represents C₁₋₈ linear alkyl, C₁₋₇ linear alkoxy or C₂₋₈ linear alkenyl.

In some embodiments of the present invention, preferably, R_{M1} and R_{M2} each independently represents C₂₋₈ linear alkenyl; further preferably, R_{M1} and R_{M2} each independently represents C₂₋₅ linear alkenyl.

In some embodiments of the present invention, preferably, one of R_{M1} and R_{M2} is C₂₋₅ linear alkenyl, and the other is C₁₋₅ linear alkyl.

In some embodiments of the present invention, preferably, R_{M1} and R_{M2} each independently represents C₁₋₈ linear alkoxy; further preferably, R_{M1} and R_{M2} each independently represents C₁₋₅ linear alkoxy.

In some embodiments of the present invention, preferably, one of R_{M1} and R_{M2} is C₁₋₅ linear alkoxy, and the other is C₁₋₅ linear alkyl.

In some embodiments of the present invention, the compound of general formula M is selected from a group consisting of the following compounds:

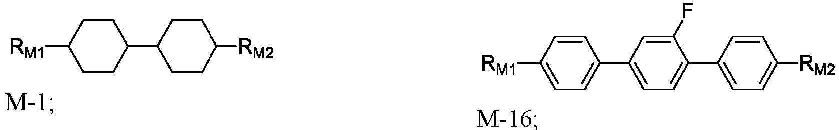

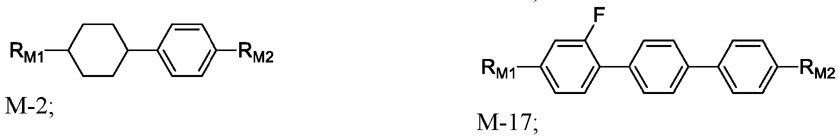

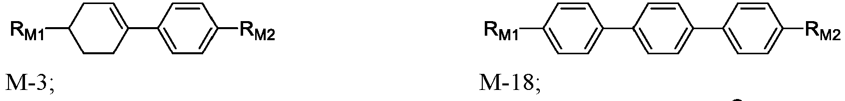

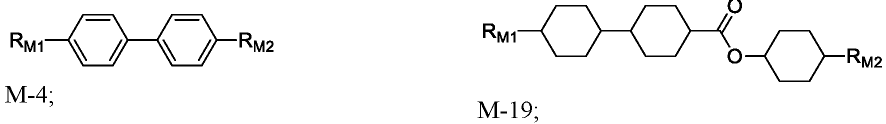

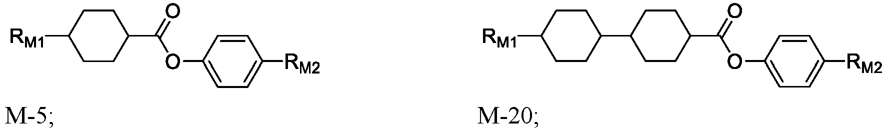

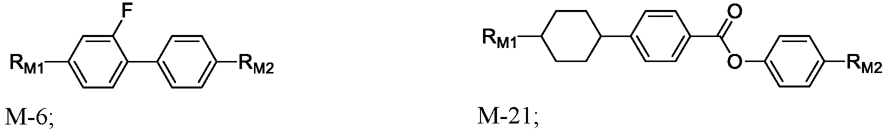

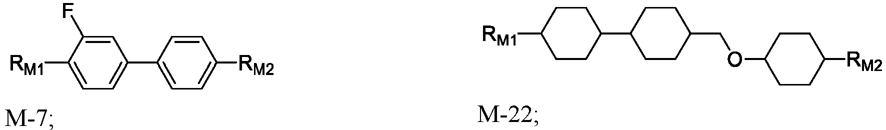

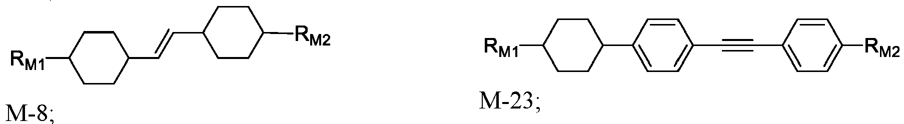

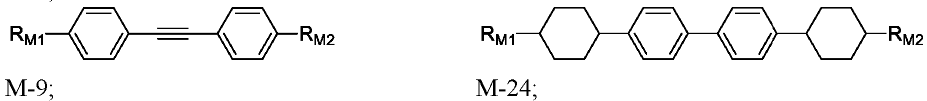

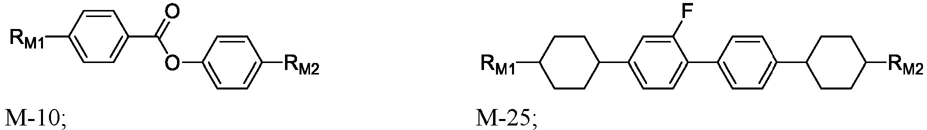

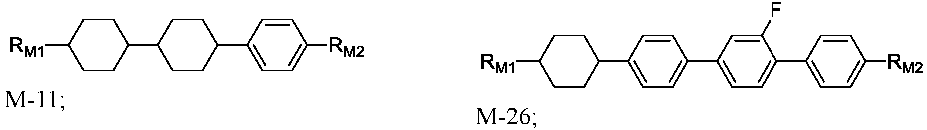

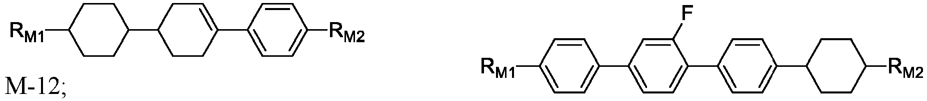

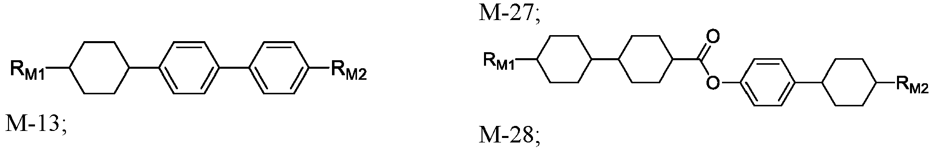

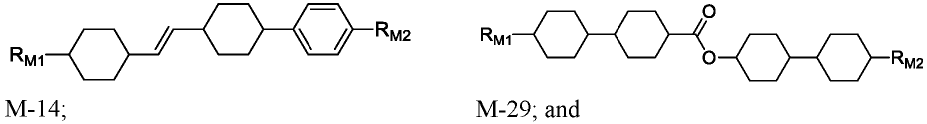

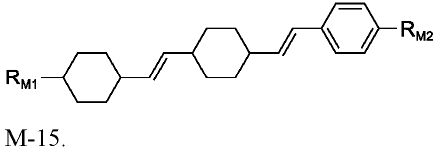

In some embodiments of the present invention, in order to achieve an appropriate clearing point, an appropriate optical anisotropy, an appropriate absolute value of dielectric anisotropy, a larger K value, a smaller rotational viscosity, a lower residue concentration, a smaller roughness and a better alignment effect, the compound of general formula M is selected from a group consisting of the compound of general formula M-1, the compound of general formula M-2, the compound of general formula M-4, the compound of general formula M-11 and the compound of general formula M-13.

In some embodiments of the present invention, the compound of general formula M-1 is selected from a group consisting of the following compounds:

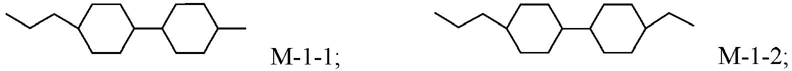

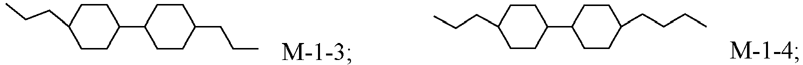

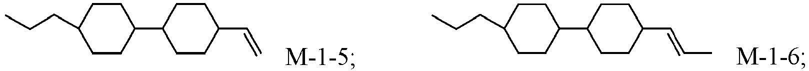

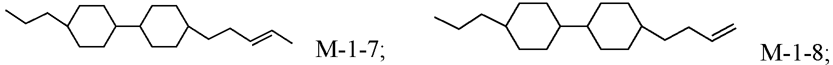

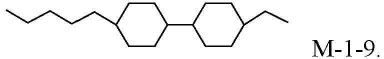

In some embodiments of the present invention, the compound of general formula M-2 is selected from a group consisting of the following compounds:

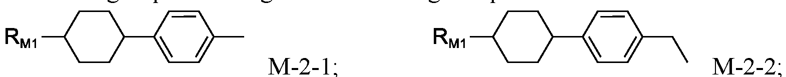

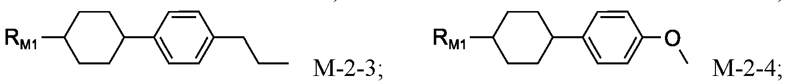

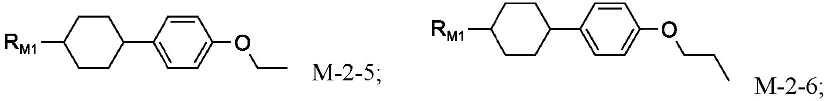

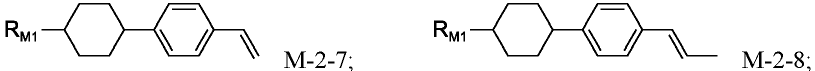

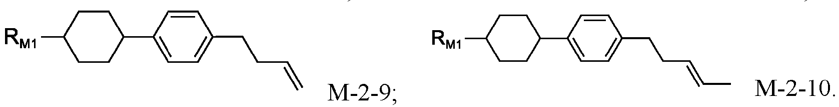

In some embodiments of the present invention, the compound of general formula M-11 is selected from a group consisting of the following compounds:

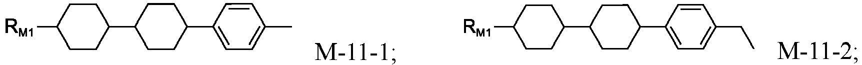

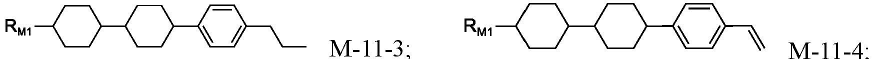

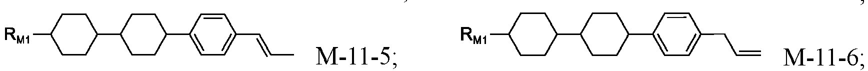

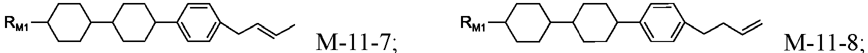

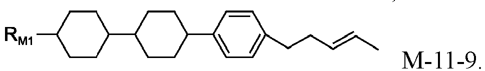

In some embodiments of the present invention, the compound of general formula M-13 is selected from a group consisting of the following compounds:

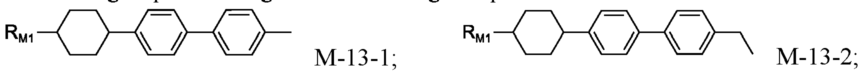

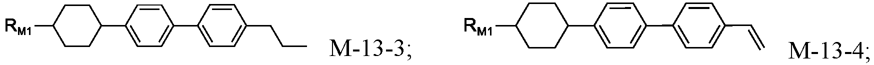

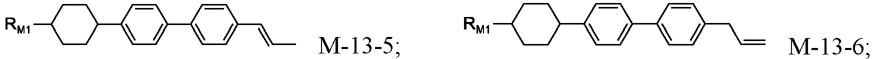

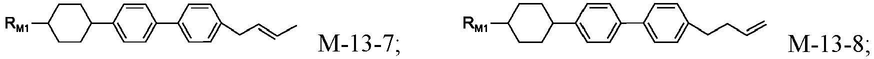

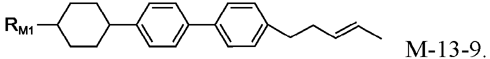

In some embodiments of the present invention, the compound of general formula M includes at least two compounds selected from a group consisting of the compound of general formula M-11-1 and the compound of general formula M-13-2.

In some embodiments of the present invention, the compound of general formula M includes at least one compound selected from a group consisting of the compound of general formula M-1-2, the compound of general formula M-1-5, the compound of general formula M-1-6, the compound of general formula M-11-1, the compound of general formula M-11-3, the compound of general formula M-4 and the compound of general formula M-13-2.

In some embodiments of the present invention, it is preferred to adjust the content of the compound of general formula M, such that the liquid crystal composition of the present invention has a lower residue concentration, a smaller roughness and a better alignment effect while maintaining an appropriate clearing point, an appropriate optical anisotropy, a larger absolute value of dielectric anisotropy, a larger K value and an appropriate rotational viscosity.

In some embodiments of the present invention, the compound of general formula M provides (in percentage by weight) 0.1%-70% (including any of the numerical values or sub-ranges therebetween) of the liquid crystal composition, for example, 0.1%, 1%, 4%, 6%, 8%, 10%, 12%, 14%, 16%, 18%, 20%, 22%, 24%, 26%, 28%, 30%, 32%, 34%, 36%, 38%, 40%, 42%, 44%, 46%, 48%, 50%, 52%, 54%, 56%, 58%, 60%, 62%, 64%, 66%, 68%, 70%, or a range between any two numerical values.

In some embodiments of the present invention, the liquid crystal composition further comprises at least one compound of general formula N: 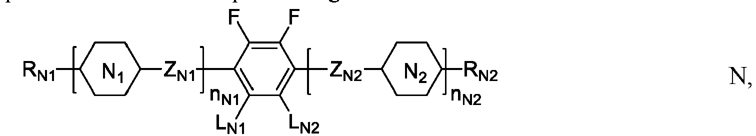 wherein,
R_{N1} and R_{N2} each independently represents -H, C₁₋₁₂ (for example, C₂, C₃, C₄, C₅, C₆, C₇, C₈, C₉, C₁₀ or C₁₁) linear alkyl, C₃₋₁₂ (for example, C₄, C₅, C₆, C₇, C₈, C₉, C₁₀ or C₁₁) branched alkyl, 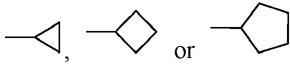 , wherein one or more than two nonadjacent -CH₂- in the C₁₋₁₂ linear alkyl or C₃₋₁₂ branched alkyl can each be independently replaced by -CH=CH-, -C≡C-. -O-, -CO-, -CO-O- or -O-CO-;
ring 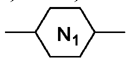 and ring 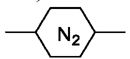 each independently represents 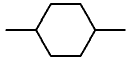 or 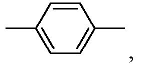 wherein one or more -CH₂- in 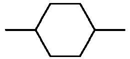 can be replaced by -O-, and one or at most two single bonds in the ring can be replaced by double bond, wherein one or more -H on 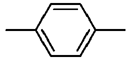 can each be independently substituted by -F, -Cl or -CN, and one or more -CH= in the rings can be replaced by -N=;
Z_{N1} and Z_{N2} each independently represents single bond, -CO-O-, -O-CO-, -CH₂O-, -OCH₂-, -CH=CH-, -C≡C-, -CH₂CH₂-, -CF₂CF₂-, -(CH₂)₄-, -CH=CH(CH₂)n_{N3}, -CF₂O- or -OCF₂-;
L_{N1} and L_{N2} each independently represents -H, halo or C₁₋₃ (for example, C₁, C₂ or C₃) alkyl;
n_{N1} represents 0, 1, 2 or 3, n_{N2} represents 0 or 1, and 0 ≤ n_{N1} + n_{N2} ≤ 3, when n_{N1} = 2 or 3, ring 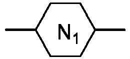 can be the same or different, Z_{N1} can be the same or different; and
n_{N3} represents 0, 1, 2 or 3.

In some embodiments of the present invention, L_{N1} and L_{N2} both represent -H.

In some embodiments of the present invention, the compound of general formula N is selected from a group consisting of the following compounds: 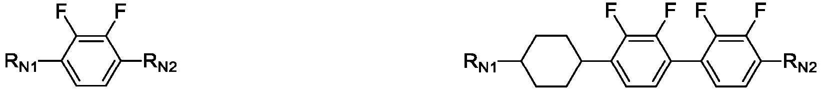 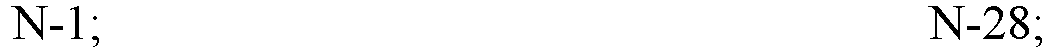 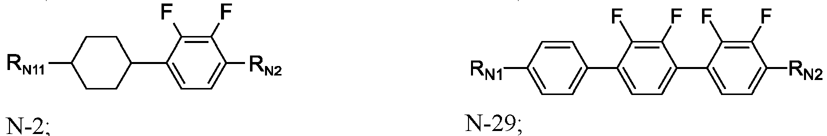 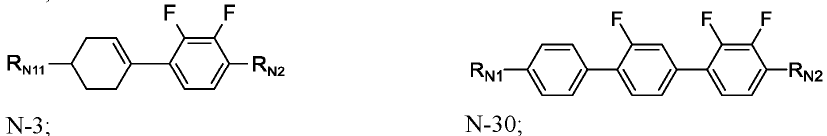 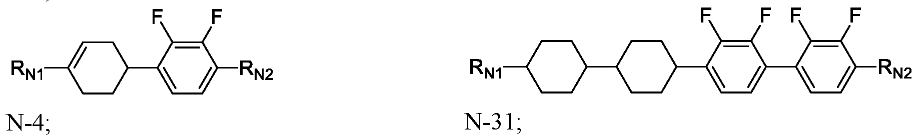 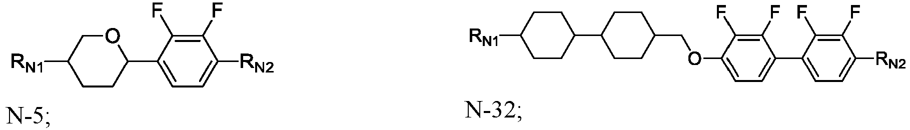 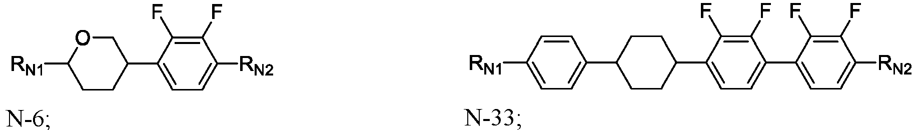 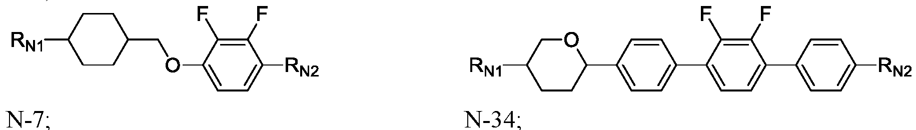 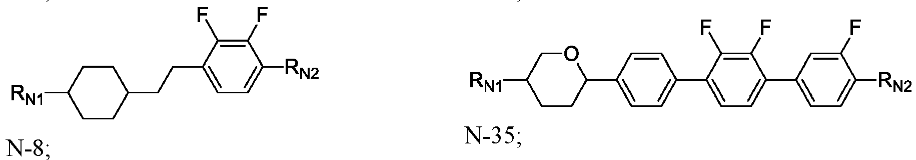 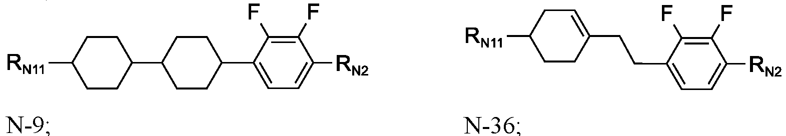 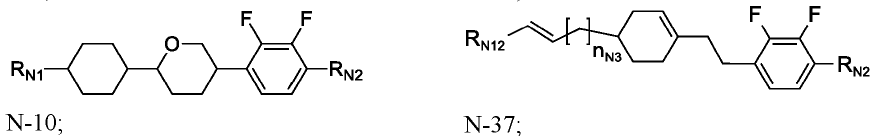 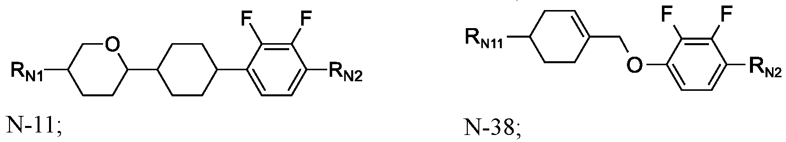 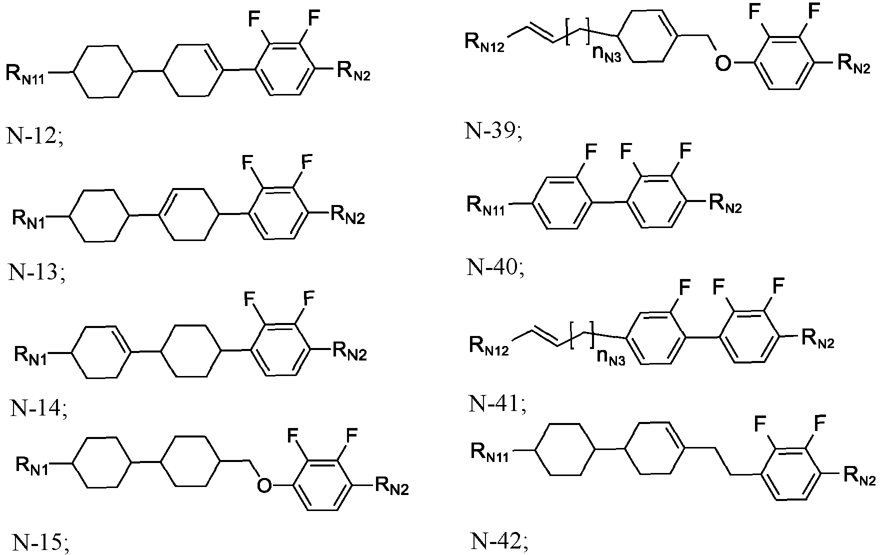 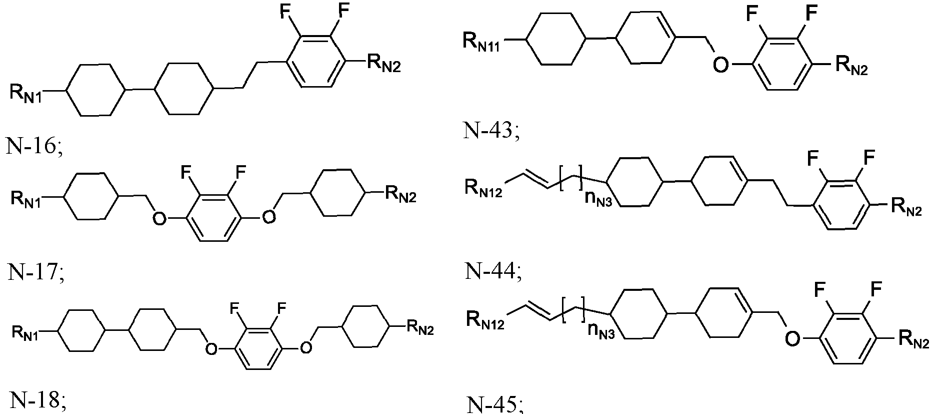 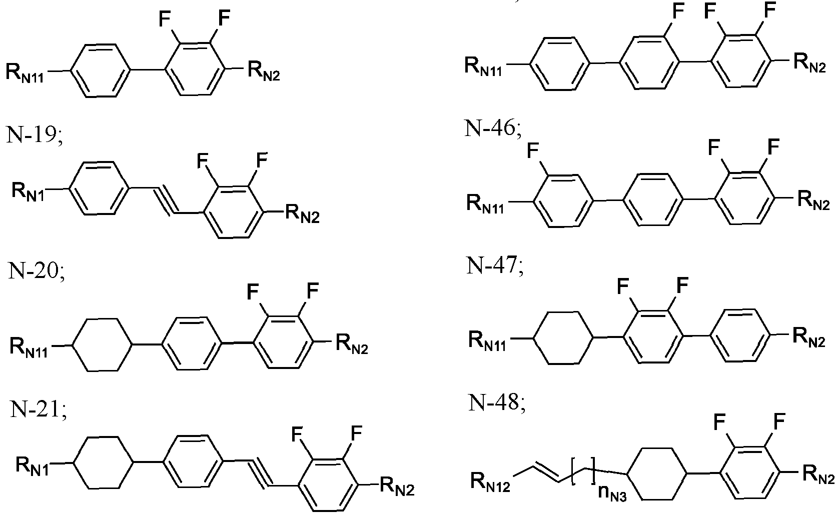 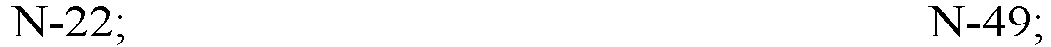 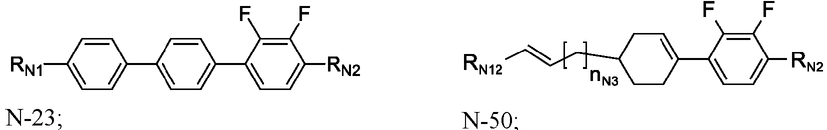 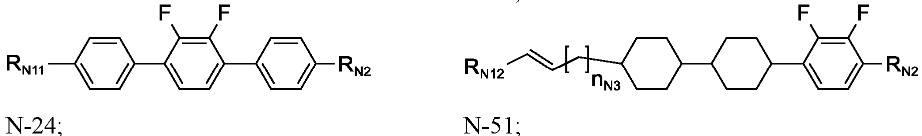 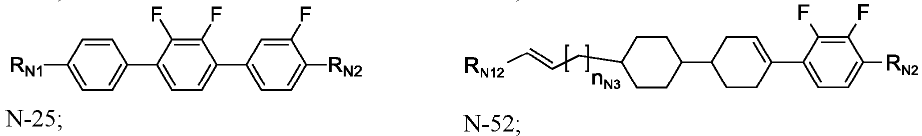 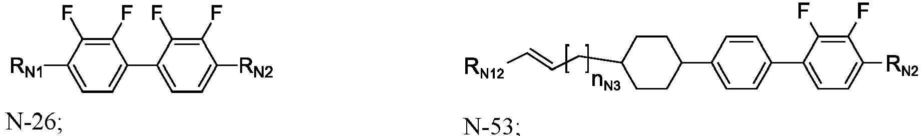 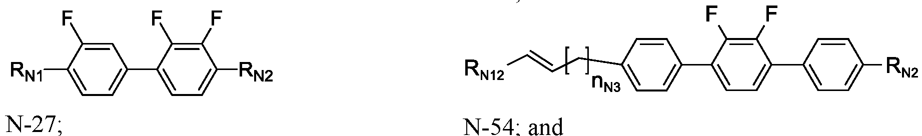 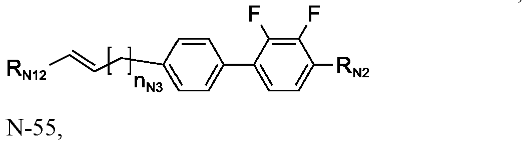 wherein,
R_{N11} represents C₁₋₅ linear alkyl, 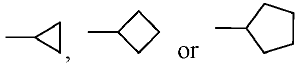 , one or more than two nonadjacent -CH₂- in the C₁₋₅ linear alkyl can each be independently replaced by -O-, -CO-, -CO-O- or -O-CO-;
R_{N12} represents -H, C₁₋₅ linear alkyl, 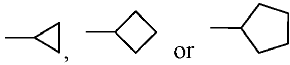 , one or more than two nonadjacent -CH₂- in the C₁₋₅ linear alkyl can each be independently replaced by -CH=CH-, -C≡C-, -O-, -CO-, -CO-O- or -O-CO-; and
n_{N3} represents 0, 1, 2 or 3.

In some embodiments of the present invention, preferably, R_{N1} and R_{N2} each independently represents C₁₋₁₀ linear alkyl, C₃₋₁₀ branched alkyl, C₁₋₉ linear alkoxy, C₃₋₉ branched alkoxy, C₂₋₁₀ linear alkenyl or C₃₋₁₀ branched alkenyl; further preferably, R_{N1} and R_{N2} each independently represents C₁₋₈ linear alkyl, C₁₋₇ linear alkoxy or C₂₋₈ linear alkenyl.

In some embodiments of the present invention, in order to achieve an appropriate clearing point, an appropriate optical anisotropy, a larger absolute value of dielectric anisotropy, a larger K value , an appropriate rotational viscosity, a lower residue concentration, a smaller roughness and a better alignment effect, the compound of general formula N is selected from a group consisting of the compound of general formula N-2, the compound of general formula N-3, the compound of general formula N-7, the compound of general formula N-9, the compound of general formula N-12, the compound of general formula N-15, the compound of general formula N-16, the compound of general formula N-19, the compound of general formula N-21.

In some embodiments of the present invention, the compound of general formula N includes at least two (for example, may be three, four or more) compounds selected from a group consisting of the compound of general formula N-19, the compound of general formula N-21 and the compound of general formula N-23.

In some embodiments of the present invention, it is preferred to adjust the content of the compound of general formula N, such that the liquid crystal composition of the present invention has a lower residue concentration, a smaller roughness and a better alignment effect while maintaining an appropriate clearing point, an appropriate optical anisotropy, a larger absolute value of dielectric anisotropy, a larger K value and an appropriate rotational viscosity.

In some embodiments of the present invention, the compound of general formula N provides (in percentage by weight) 0.1%-70% (including any of the numerical values or sub-ranges therebetween) of the liquid crystal composition, for example, 0.1%, 1%, 4%, 6%, 8%, 10%, 12%, 14%, 16%, 18%, 20%, 22%, 24%, 26%, 28%, 30%, 32%, 34%, 36%, 38%, 40%, 42%, 44%, 46%, 48%, 50%, 52%, 54%, 56%, 58%, 60%, 62%, 64%, 66%, 68%, 70%, or a range between any two numerical values.

In some embodiments of the present invention, the liquid crystal composition of the present invention comprises at least one compound of general formula B: 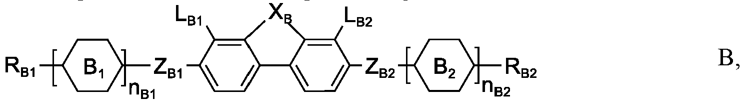 wherein,
R_{B1} and R_{B2} each independently represents halo, C₁₋₁₂ (for example, C₂, C₃, C₄, C₅, C₆, C₇, C₈, C₉, C₁₀ or C₁₁) halogenated or unhalogenated linear alkyl, C₃₋₁₂ (for example, C₄, C₅, C₆, C₇, C₈, C₉, C₁₀ or C₁₁) halogenated or unhalogenated branched alkyl, 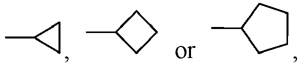 wherein one or more than two nonadjacent -CH₂- in the C₁₋₁₂ halogenated or unhalogenated linear alkyl, C₃₋₁₂ halogenated or unhalogenated branched alkyl, 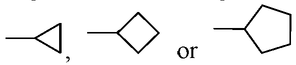 can each be independently replaced by -CH=CH-, -CH=CF-, -C≡C-, -O-, -CO-, -CO-O- or -O-CO-, wherein at most one single bond in 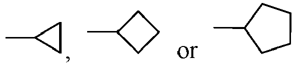 can be replaced by double bond;
ring 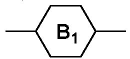 and ring 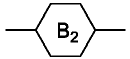 each independently represents 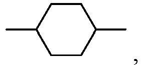 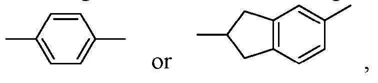 wherein one or more -CH₂- in 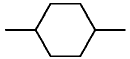 and 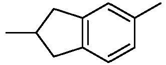 can be replaced by -O-, and one or at most two single bonds in the rings can be replaced by double bond, wherein one or more -H on 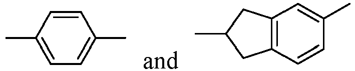 can each be independently substituted by -CN, -F or -Cl, and one or more -CH= in the rings can be replaced by -N=;
X_{B} represents -O-, -S- or -CO-;
L_{B1} and L_{B2} each independently represents -H, -F, -Cl, -CF₃ or -OCF₃;
Z_{B1} and Z_{B2} each independently represents -CO-O-, -O-CO-, -OCH₂-, -CH=CH-, -C≡C-, -CH₂CH₂-, -CF₂CF₂-, -(CH₂)n_{B3}-, -(CH₂)n_{B3}O, -(CH₂)n_{B3}S-, -CF₂O- or -OCF₂-, wherein n_{B3} represents an integer of 0-5 (for example, 1, 2, 3 or 4); and
n_{B1} and n_{B2} each independently represents 0 1 or 2, wherein when n_{B1} represents 2, ring 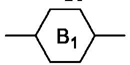 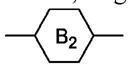 can be the same or different.

In some embodiments of the present invention, the compound of general formula B is selected from a group consisting of the following compounds: 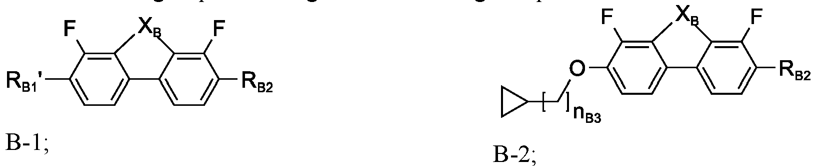 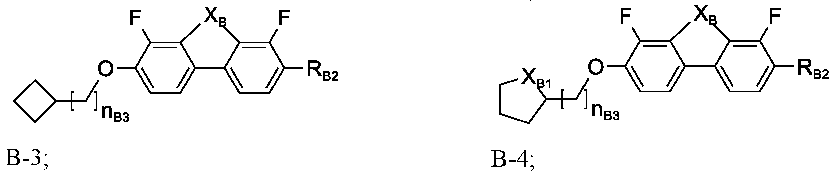 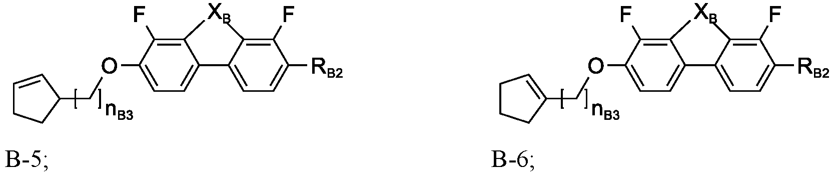 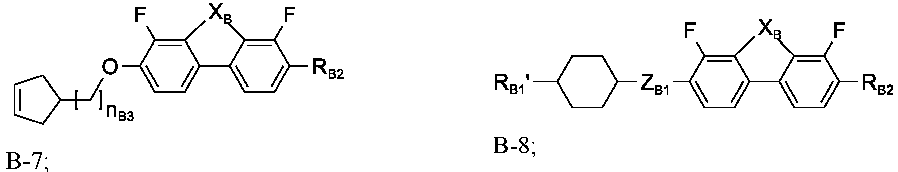 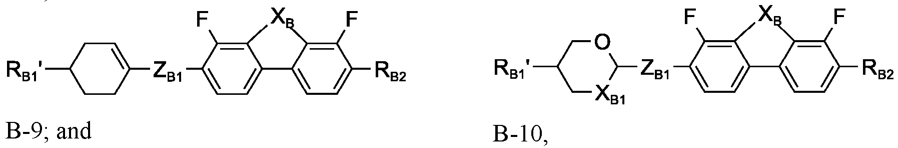
wherein,R_{B1}' represents C₁₋₈ linear alkyl or alkoxy, C₂₋₈ linear alkenyl or alkenoxy; and
X_{B1} represents -O- or -CH₂-.

In some embodiments of the present invention, in order to achieve an appropriate clearing point, an appropriate optical anisotropy, an appropriate absolute value of dielectric anisotropy, a larger K value (K₁₁ and K₃₃), a smaller rotational viscosity, a smaller polymer residue, a smaller roughness and a better alignment effect, the compound of general formula B is selected from a group consisting of the compound of general formula B-1, the compound of general formula B-4, the compound of general formula B-5, the compound of general formula B-6 and the compound of general formula B-7.

In some embodiments of the present invention, the compound of general formula B-1 is selected from a group consisting of the following compounds: 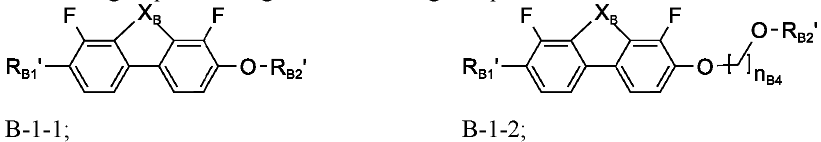 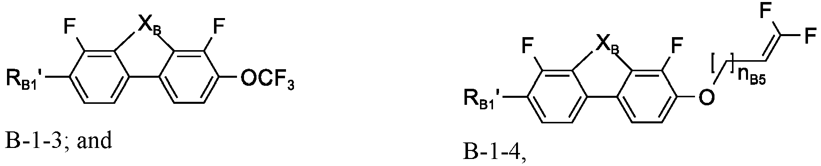 wherein, R_{B2}' represents C₁₋₅ linear alkyl; n_{B4} represents an integer of 1-5 (for example, 1, 2, 3 or 4); and n_{B5} represents an integer of 0-5 (for example, 1, 2, 3 or 4).

In some embodiments of the present invention, preferably, X_{B} represents -S-.

In some embodiments of the present invention, the compound of general formula B is selected from a group consisting of the compound of general formula B-1-1, the compound of general formula B-1-4, the compound of general formula B-4, the compound of general formula B-5, the compound of general formula B-6 and the compound of general formula B-7.

In some embodiments of the present invention, the compound of general formula B includes at least two (for example, may be three, four or more) compounds selected from a group consisting of the compound of general formula B-1-1, the compound of general formula B-5, the compound of general formula B-6 and the compound of general formula B-7.

In some embodiments of the present invention, it is preferred to adjust the content of the compound of general formula B, such that the liquid crystal composition of the present invention has an appropriate clearing point, an appropriate optical anisotropy, an appropriate absolute value of dielectric anisotropy, a larger K value (K₁₁ and K₃₃), a smaller rotational viscosity, a smaller polymer residue, a smaller roughness, a smaller contact angle and a better alignment effect.

In some embodiments of the present invention, the compound of general formula B provides (in percentage by weight) 0.1%-30% (including any of the numerical values or sub-ranges therebetween) of the liquid crystal composition, for example, 0.1%, 1%, 4%, 6%, 8%, 10%, 12%, 14%, 16%, 18%, 20%, 22%, 24%, 26%, 28%, 30%, or a range between any two numerical values.

In some embodiments of the present invention, the liquid crystal composition of the present invention comprises at least one polymerizable compound of general formula RM: 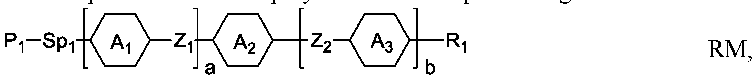 wherein,
R₁ represents -H, halo, -CN, -Sp₂-P₂, C₁₋₁₂ (for example, may be C₂, C₃, C₄, C₅, C₆, C₇, C₈, C₉, C₁₀ or C₁₁) linear alkyl, C₃₋₁₂ for example, may be C_{3,} C_{4,} C_{5,} C₆, C₇, C₈, C₉, C₁₀ or C₁₁) branched alkyl, 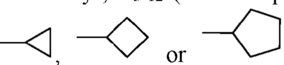 , wherein one or more than two nonadjacent -CH₂-in the C₁₋₁₂ linear alkyl, C₃₋₁₂ branched alkyl, 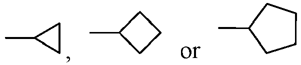 can each be independently replaced by -CH=CH-, -C≡C-, -O-, -CO-, -CO-O- or -O-CO-, and one or more -H can each be independently substituted by -F or -Cl;
ring 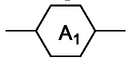 and ring 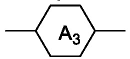 each independently represents 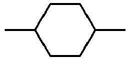 or 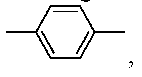 wherein one or more -CH₂- in 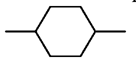 can be replaced by -O-, and one or at most two single bonds in the ring can be replaced by double bond, wherein one or more -H on 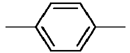 can each be independently substituted by -F, -Cl, -CN, -Sp₃-P₃, C₁₋₁₂ halogenated or unhalogenated linear alkyl, C₁₋₁₁ halogenated or unhalogenated linear alkoxy, 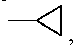 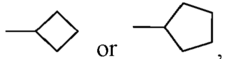 and one or more -CH= in the rings can be replaced by -N=;
ring 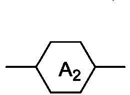 represents 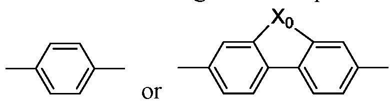 , wherein one or more -H on 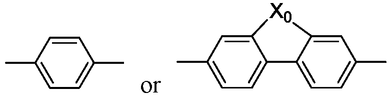 can each be independently substituted by -F, -Cl, -CN, -Sp₃-P₃, C₁₋₁₂ halogenated or unhalogenated linear alkyl, C₁₋₁₁ halogenated or unhalogenated linear alkoxy, 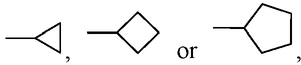 and one or more -CH= in the rings can be replaced by -N=;
P₁, P₂ and P₃ each independently represents polymerizable group;
X₀ represents -O-, -S- and -CO-;
Sp₁, Sp₂ and Sp₃ each independently represents spacer group or single bond;
Z₁ and Z₂ each independently represents -O-, -S-, -CO-, -CO-O-, -O-CO-, -O-CO-O-, -CH₂O-, -OCH₂-, -CH₂S-, -SCH₂-, -CF₂O-, -OCF₂-, -CF₂S-, -SCF₂-, -(CH₂)_{d}-, -CF₂CH₂-, -CH₂CF₂-, -(CF₂)_{d}-, -CH=CH-, -CF=CF-, -CH=CF-, -CF=CH-, -C≡C-, -CH=CH-CO-O-, -O-CO-CH=CH-, -CH₂CH₂-CO-O-, -O-CO-CH₂CH₂-, -CHR¹-, -CR¹R²- or single bond, wherein R¹ and R² each independently represents C₁₋₁₂ linear alkyl or C₃₋₁₂ branched alkyl, and d represents an integer of 1-4;
a represents 0, 1 or 2, b represents 0 or 1, wherein when a represents 2, ring 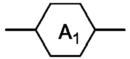 can be the same or different, Z₁ can be the same or different.

In some embodiments of the present invention, the polymerizable compound of general formula RM is selected from a group consisting of the following compounds: 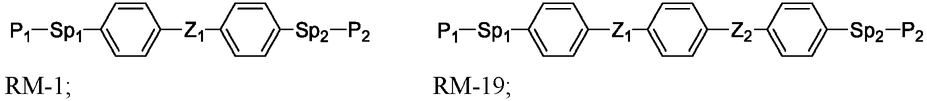 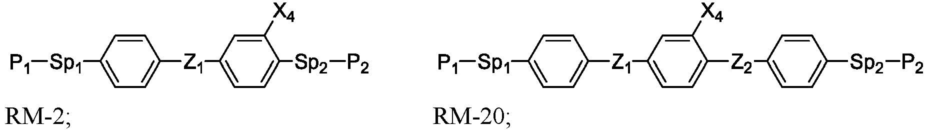 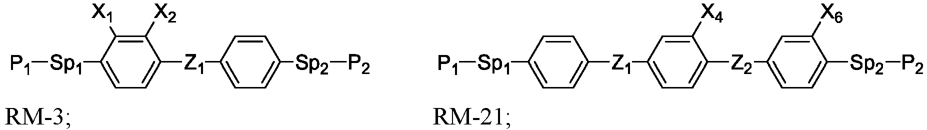 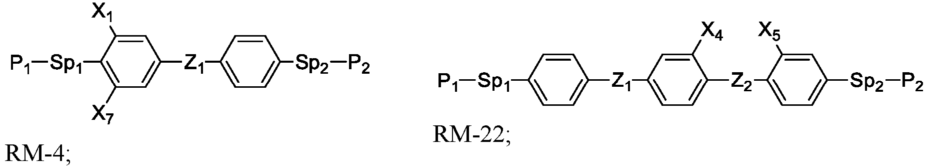 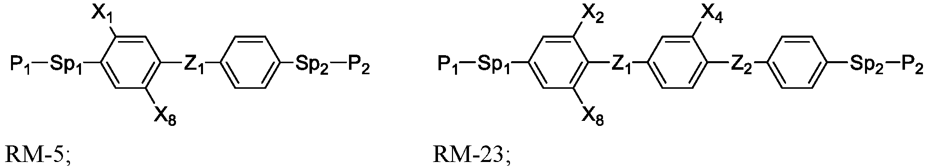 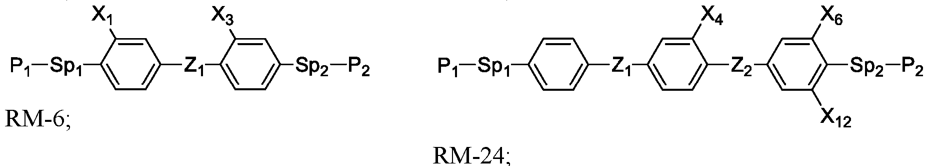 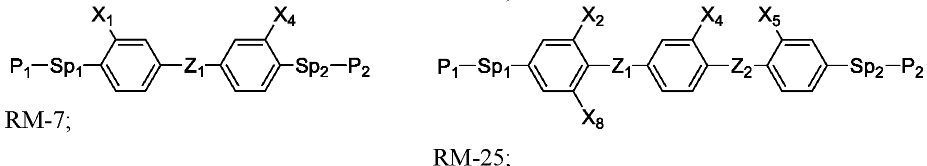 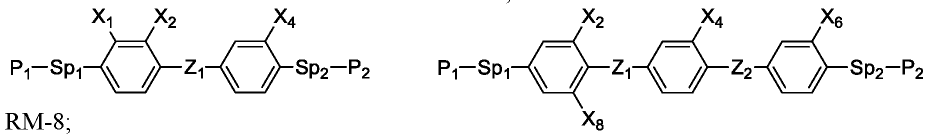 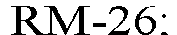 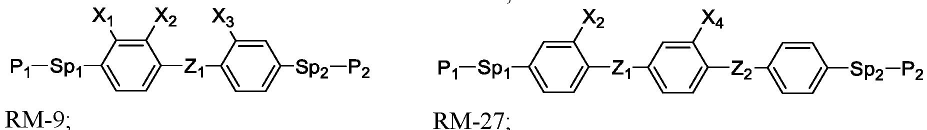 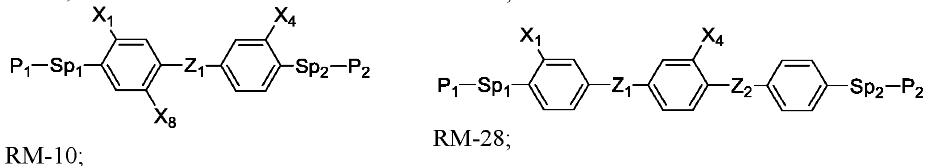 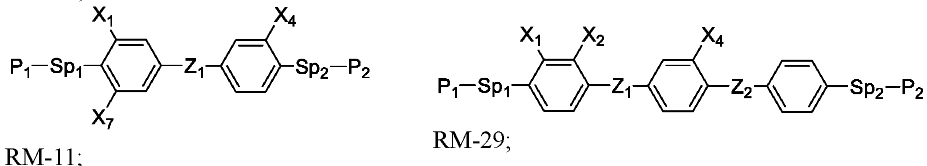 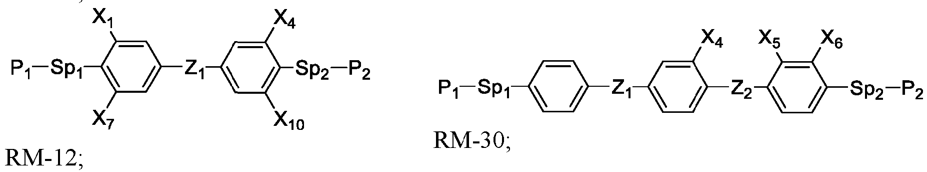 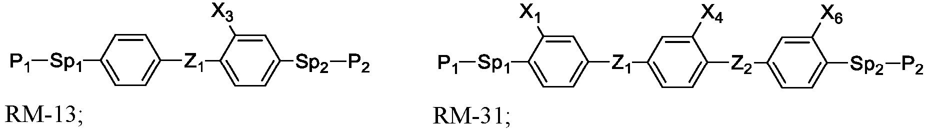 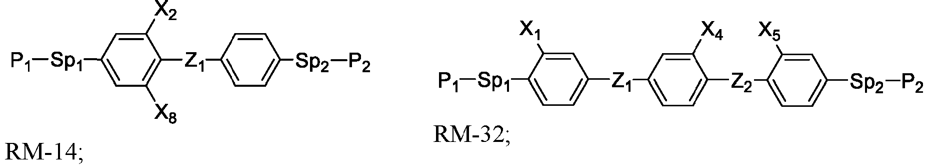 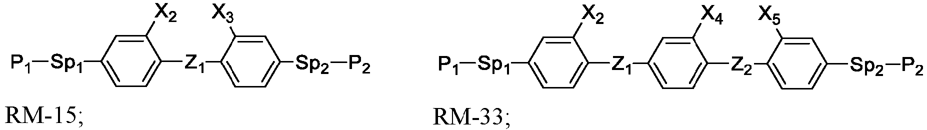 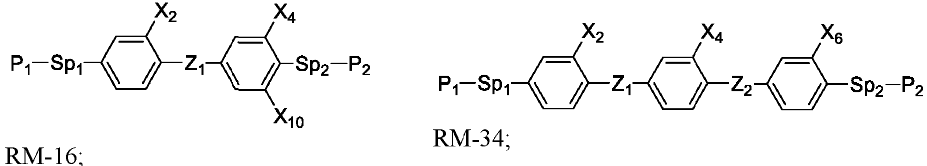 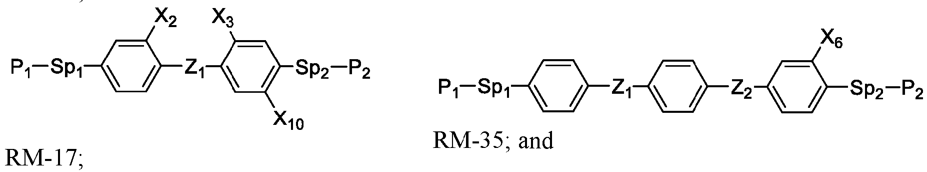 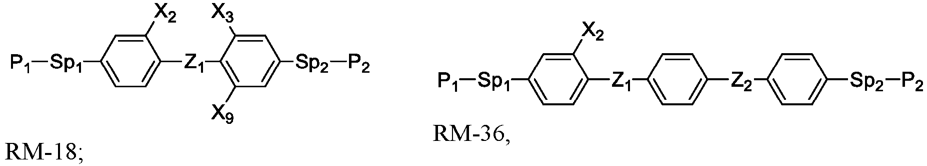 wherein, X₁-X₁₀ and X₁₂ each independently represents -F, -Cl, -Sp₃-P₃, C₁₋₅ linear alkyl or alkoxy,

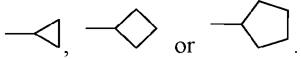

In some embodiments of the present invention, X₁-X₁₀ and X₁₂ each independently represents -F, -Cl, -Sp₃-P₃, -CH₃ or -OCH₃.

In some embodiments of the present invention, Sp₁ and Sp₂ both represent single bond.

In some embodiments of the present invention, in order to achieve an appropriate clearing point, an appropriate optical anisotropy, an appropriate absolute value of dielectric anisotropy, a larger K value , a smaller rotational viscosity, a lower residue concentration, a smaller roughness and a better alignment effect, the polymerizable compound of general formula RM is selected from a group consisting of the compound of general formula RM-1, the compound of general formula RM-2 and the compound of general formula RM-20.

The polymerizable groups involved in the present invention are groups suitable for polymerization reactions (for example, radical or ionic bond polymerization, addition polymerization or condensation polymerization), or groups suitable for addition or condensation on the polymer backbone. For chain polymerization, a polymerizable group containing -CH=CH- or -C≡C- is particularly preferred, and for ring-opening polymerization, for example, an oxetane or epoxy group is particularly preferred.

In some embodiments of the present invention, the polymerizable groups P₁, P₂ and P₃ each independently represents 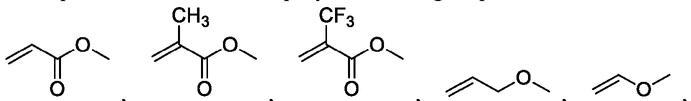 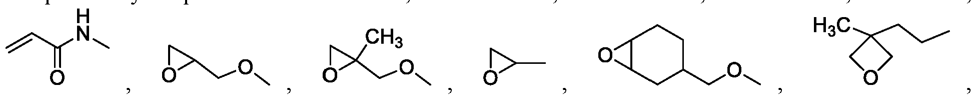 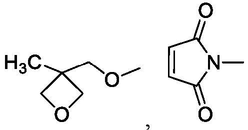 or -SH; preferably, the polymerizable groups P₁ P₂ and P₃ each independently represents 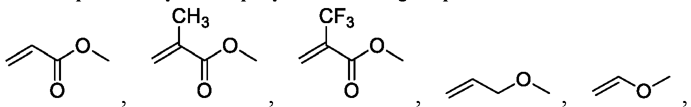 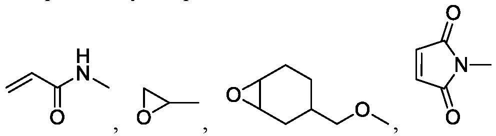 or -SH; further preferably, the polymerizable groups P₁, P₂ and P₃ each independently represents

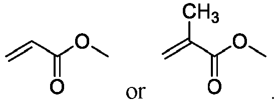

In some embodiments of the present invention, the polymerizable compound of general formula RM-1 is selected from a group consisting of the following compounds:

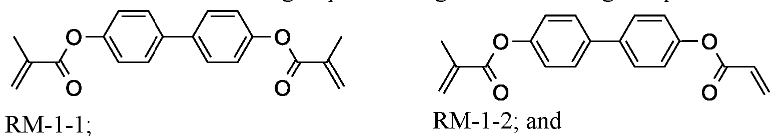

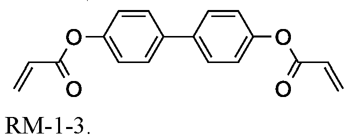

In some embodiments of the present invention, the polymerizable compound of general formula RM-2 is selected from a group consisting of the following compounds:

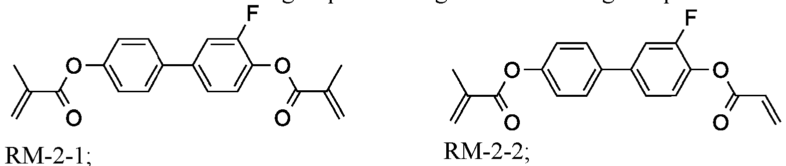

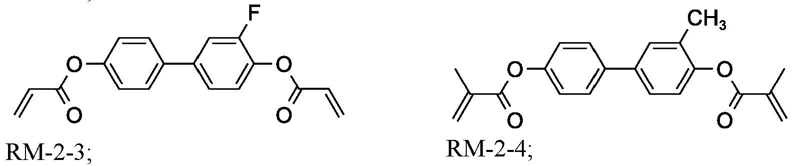

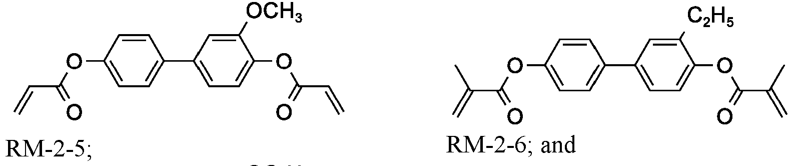

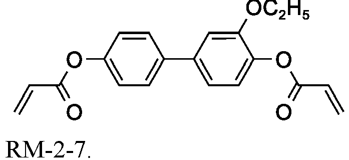

In some embodiments of the present invention, the polymerizable compound of general formula RM-19 is selected from a group consisting of the following compounds:

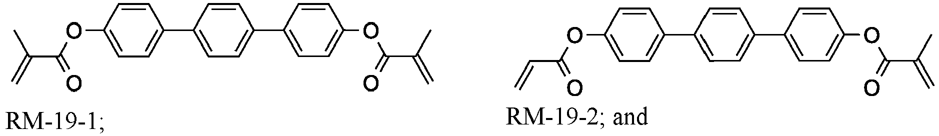

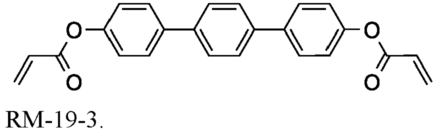

In some embodiments of the present invention, the polymerizable compound of general formula RM-20 is selected from a group consisting of the following compounds:

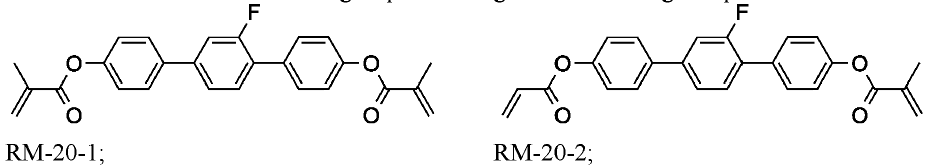

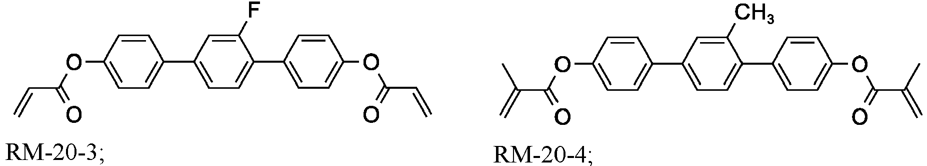

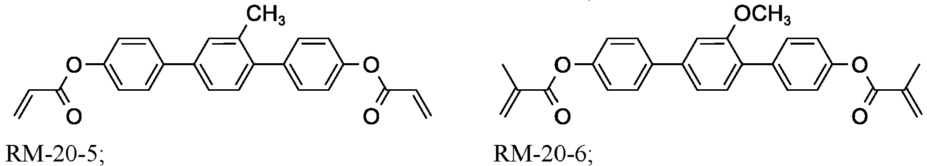

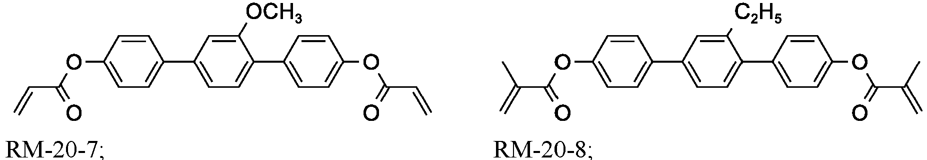

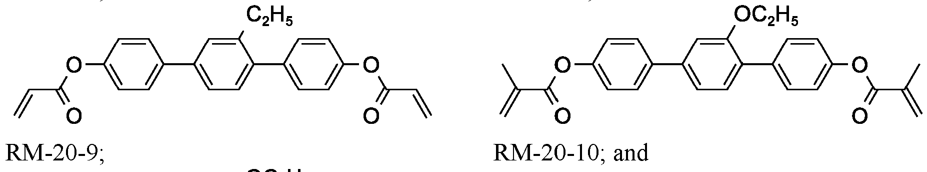

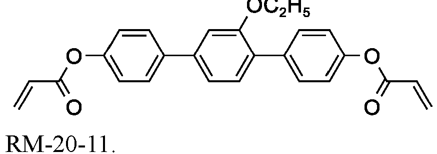

The term "spacer group" as used herein, is known to the person skilled in the art and is described in the literature, for example, Pure Appl. Chem. 2001, 73(5), 888 and C. Tschierske, G. Pelzl, S. Diele, Angew. Chem. 2004, 116, 6340-6368. As used herein, the term "spacer group" means a flexible group which connects the mesogenic group and the polymerizable group(s) in a polymerizable compound. Representative spacer group for example is -(CH₂)ₚ₁-, -(CH₂)ₚ₁-O-, -O-(CH₂)ₚ₁-, -(CH₂)ₚ₁-O-CO-, -(CH₂)ₚ₁-CO-O-, -(CH₂)ₚ₁-O-CO-O-, -(CH₂CH₂O)_{q1}-CH₂CH₂-, -(CH₂CH₂S)_{q1}-CH₂CH₂-, -(CH₂CH₂NH)_{q1}-CH₂CH₂-, -CR⁰R⁰⁰-(CH₂)ₚ₁- or -(SiR⁰R⁰⁰-O)ₚ₁-, wherein, p₁ represents an integer of 1-10 (for example, 1, 2, 3, 4, 5, 6, 7, 8 or 9), q₁ represents an integer of 1-3 (for example, 1, 2 or 3), R⁰ and R⁰⁰ each independently represents -H, C₁₋₁₀ (for example, C₁, C₂, C₃, C₄, C₅, C₆, C₇, C₈ or C₉) linear alkyl, C₃₋₁₀ (for example, C₃, C₄, C₅, C₆, C₇, C₈ or C₉) branched alkyl or C₃₋₁₀ (for example, C₃, C₄, C₅, C₆, C₇, C₈ or C₉) cycloalkyl. The particularly preferred spacer group is -(CH₂)ₚ₁-, -(CH₂)ₚ₁-O-, -(CH₂)ₚ₁-O-CO-, -(CH₂)ₚ₁-CO-O-, -(CH₂)ₚ₁-O-CO-O- or -CR⁰R⁰⁰-(CH₂)ₚ₁-.

In some embodiments of the present invention, it is preferred to adjust the content of the compound of general formula RM, such that the liquid crystal composition of the present invention has a smaller polymer residue, a smaller roughness and a better alignment effect.

In some embodiments of the present invention, the polymerizable compound of general formula RM provides (in percentage by weight) 0.001%-5% (including any of the numerical values or sub-ranges therebetween) of the liquid crystal composition, for example, 0.001%, 0.002%, 0.004%, 0.005%, 0.006%, 0.008%, 0.01%, 0.02%, 0.04%, 0.06%, 0.08%, 0.1%, 0.2%, 0.25%, 0.26%, 0.27%, 0.28%, 0.29%, 0.3%, 0.32%, 0.33%, 0.34%, 0.35%, 0.4%, 0.5%, 0.6%, 0.8%, 1%, 1.2%, 1.6%, 1.8%, 2%, 2.5%, 3%, 3.5%, 4%, 4.5%, 5%, or a range between any two numerical values.

As used herein, both -CO- and -C(O)- represent carbonyl group.

As used herein, the term "C₁₋ᵣ" (wherein r is an integer greater than 1) may refer to any integer number (between 1 and r, including the end values 1 and r) of carbon atom(s), e.g., C₂, C₍ᵣ₋₁₎ or Cr. For example, "C1-12" may include C₁, C₂, C₃, C₄, C₅, C₆, C₇, C₈, C₉, C₁₀, C₁₁ or C₁₂.

As used herein, the term "an integer of y₁-y₂" may refer to any integer within this rang (including the end values y₁ and y₂). For example, "an integer of 0-12" may be, such as, 0, 1, 2, 3, 4, 5, 6, 7, 8, 9, 10, 11 or 12.

In some embodiments of the present invention, the liquid crystal composition of the present invention further comprises at least one compound selected from a group consisting of the compound of general formula A-1 and the compound of general formula A-2: 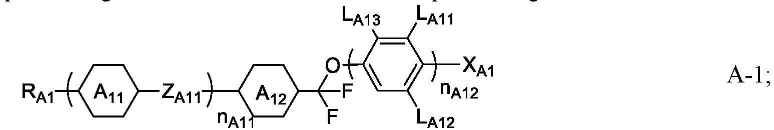 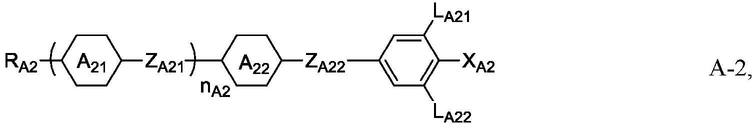 wherein,
R_{A1} and R_{A2} each independently represents C₁₋₁₂ (for example, C₁, C₂, C₃, C₄, C₅, C₆, C₇, C₈, C₉, C₁₀, C₁₁ or C₁₂) linear alkyl, C₃₋₁₂ (for example, C₃, C₄, C₅, C₆, C₇, C₈, C₉, C₁₀, C₁₁ or C₁₂) branched alkyl, 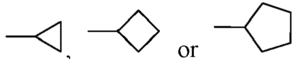 wherein one or more than two nonadjacent -CH₂-in the C₁₋₁₂ linear alkyl, C₃₋₁₂ branched alkyl, 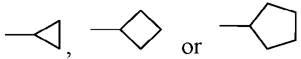 can each be independently replaced by -CH=CH-, -C≡C-, -O-, -CO-, -CO-O- or -O-CO-, and one or more -H in the C₁₋₁₂ linear alkyl or C₃₋₁₂ branched alkyl can each be independently substituted by -F or -Cl;
ring 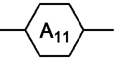 , ring 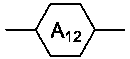 , ring 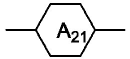 and ring 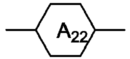 each independently represents 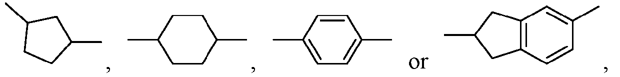 wherein one or more -CH₂- in 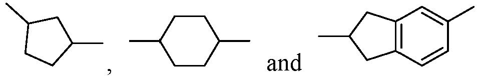 can be replaced by -O-, and one or at most two single bonds in the rings can be replaced by double bond, wherein one or more -H on 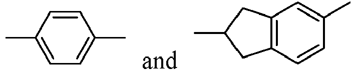 can each be independently substituted by -F, -Cl or -CN, and one or more -CH= in the rings can be replaced by -N=;
Z_{A11} represents single bond, -CH₂CH₂-, -CF₂CF₂-, -CO-O-, -O-CO-, -O-CO-O-, -CH=CH-, -CF=CF-, -CF₂O-, -OCF₂-, -CH₂O- or -OCH₂-;
Z_{A21} and Z_{A22} each independently represents single bond, -CH₂CH₂-, -CF₂CF₂-, -CO-O-, -O-CO-, -O-CO-O-, -CH=CH-, -CF=CF-, -CH₂O- or -OCH₂-;
L_{A11}, L_{A12}, L_{A13}, L_{A21} and L_{A22} each independently represents -H, halo or C₁₋₃ alkyl;
X_{A1} and X_{A2} each independently represents halo, C₁₋₅ halogenated alkyl or halogenated alkoxy, C₂₋₅ halogenated alkenyl or halogenated alkenoxy;
n_{A11} represents 0, 1, 2 or 3, wherein when n_{A11} = 2 or 3, ring 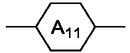 can be the same or different, Z_{A11} can be the same or different;
n_{A12} represents 1 or 2, wherein when n_{A12} = 2, ring 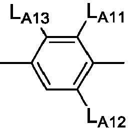 can be the same or different; and
n_{A2} represents 0, 1, 2 or 3, wherein when n_{A2} = 2 or 3, ring 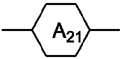 can be the same or different, Z_{A21} can be the same or different.

In some embodiments of the present invention, the compound selected from a group consisting of the compound of general formula A-1 and the compound of general formula A-2 provides (in percentage by weight) 0.1%-60% (including any value within this range) of the liquid crystal composition, for example, 0.1%, 1%, 4%, 6%, 8%, 10%, 12%, 14%, 16%, 18%, 20%, 22%, 24%, 26%, 28%, 30%, 32%, 34%, 36%, 38%, 40%, 42%, 44%, 46%, 48%, 50%, 52%, 54%, 56%, 58%, 60%, or a range between any two numerical values.

In some embodiments of the present invention, the compound of general formula A-1 is selected from a group consisting of the following compounds: 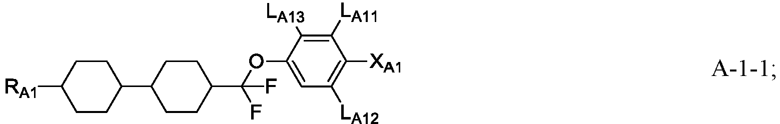 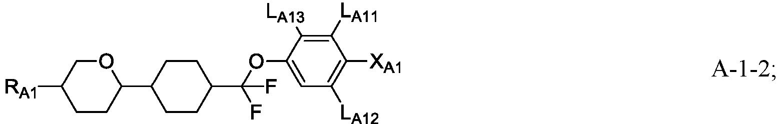 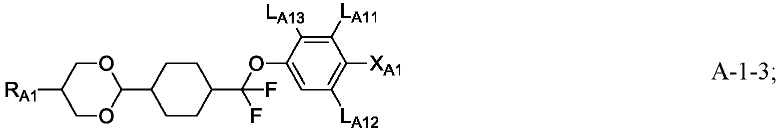 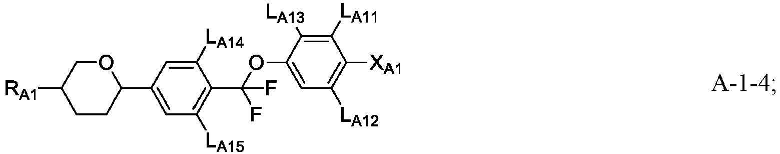 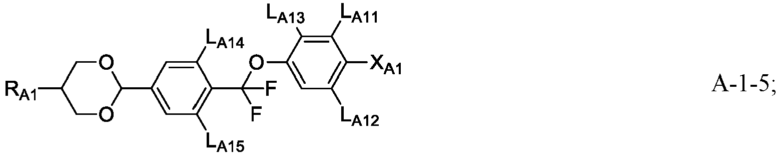 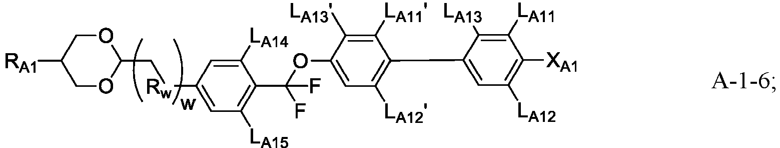 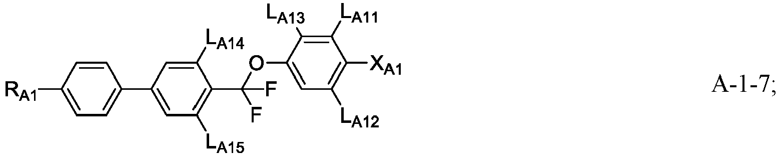 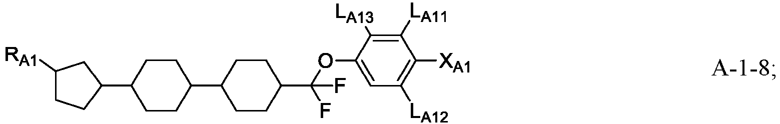 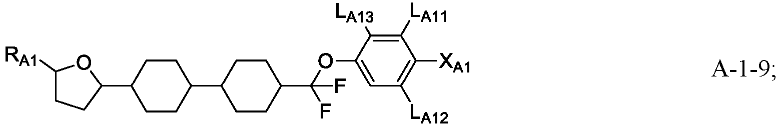 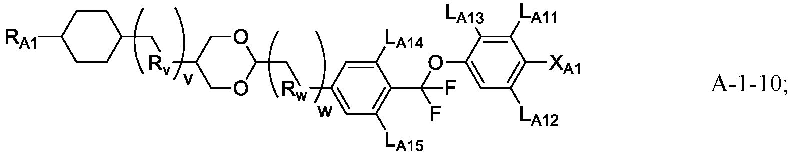 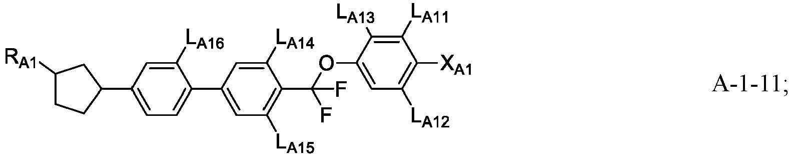 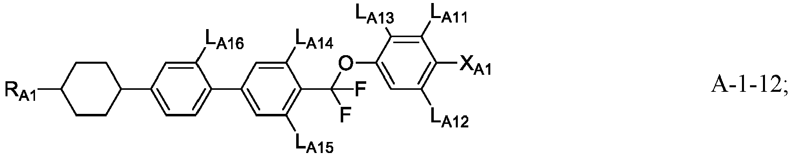 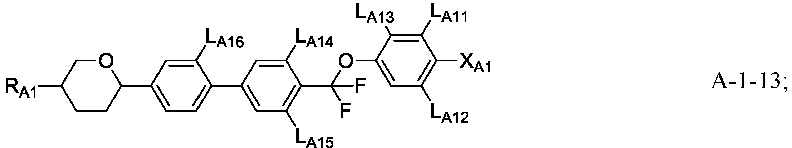 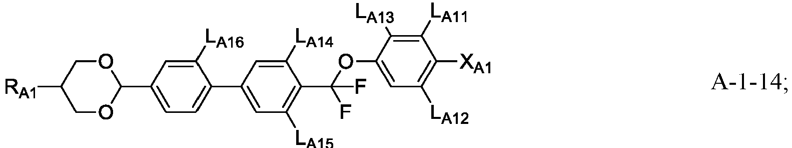 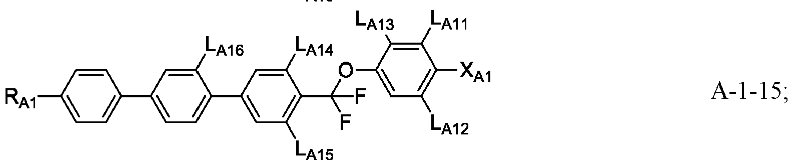 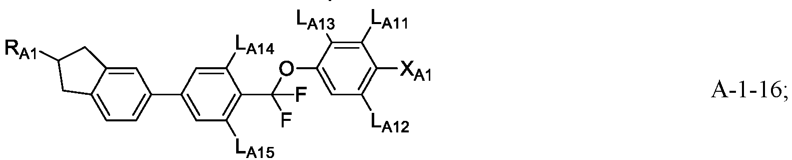 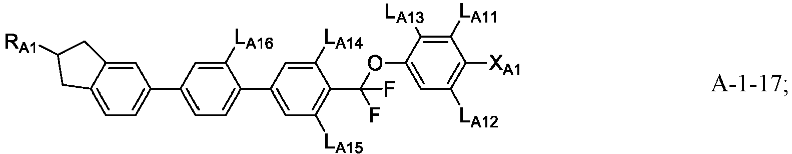 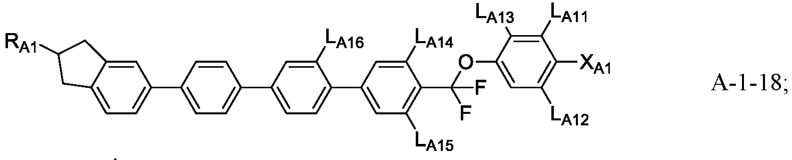 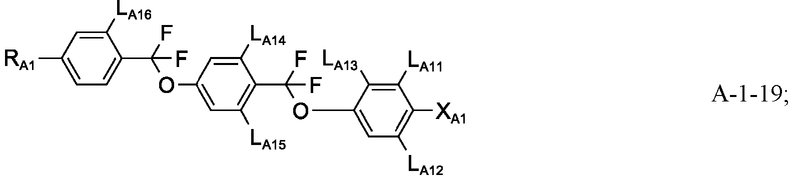 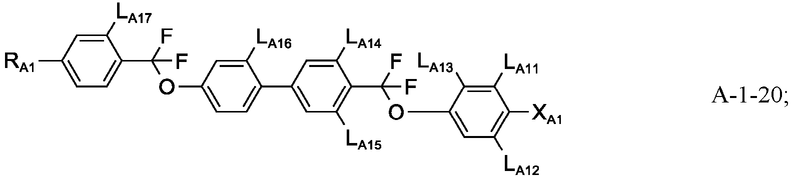 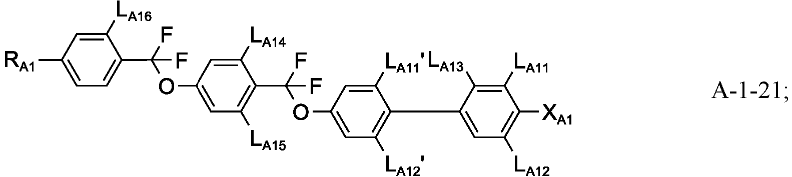 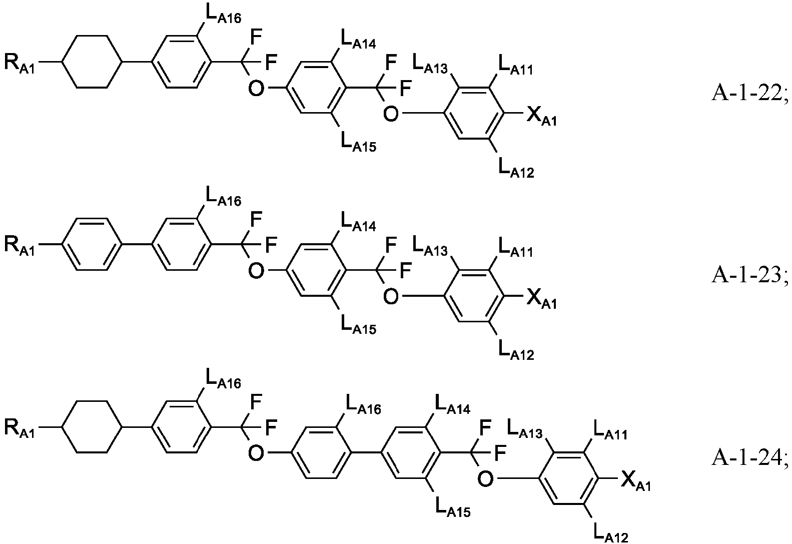 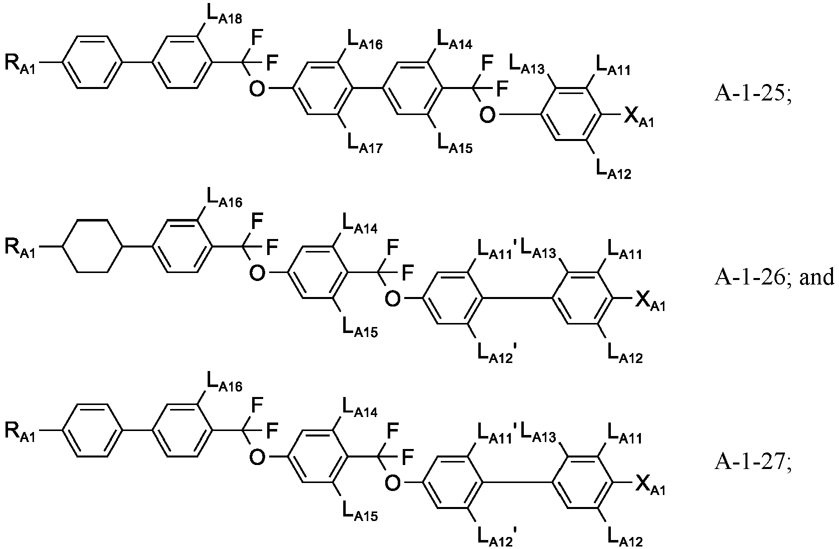 wherein,
R_{A1} represents C₁₋₈ linear alkyl, 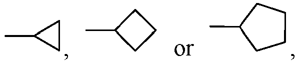 wherein one or more than two nonadjacent -CH₂- in the C₁₋₈ linear alkyl can each be independently replaced by -CH=CH-, -C≡C-, -O-, -CO-, -CO-O- or -O-CO-, and one or more -H present in these groups can each be independently substituted by -F or -Cl;
Rᵥ and R_{w} each independently represents -CH₂- or -O-;
L_{A11}, L_{A12}, L_{A11}', L_{A12}', L_{A14}, L_{A15}, L_{A16}, L_{A17} and L_{A18} each independently represents -H or -F;
L_{A13} and L_{A13}' each independently represents -H or -CH₃;
X_{A1} represents -F, -CF₃ or -OCF₃; and
v and w each independently represents 0 or 1.

In some embodiments of the present invention, the compound of general formula A-1 provides (in percentage by weight) 0.1%-50% (including any of the numerical values or sub-ranges therebetween) of the liquid crystal composition, for example, 0.1%, 1%, 4%, 6%, 8%, 10%, 12%, 14%, 16%, 18%, 20%, 22%, 24%, 26%, 28%, 30%, 32%, 34%, 36%, 38%, 40%, 42%, 44%, 46%, 48%, 50%, or a range between any two numerical values.

In some embodiments of the present invention, the compound of general formula A-2 is selected from a group consisting of the following compounds: 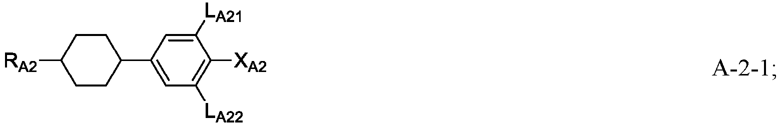 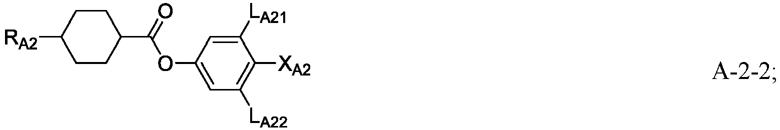 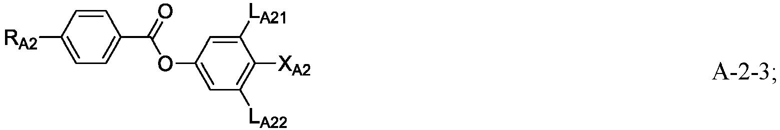 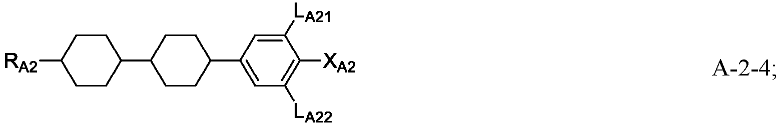 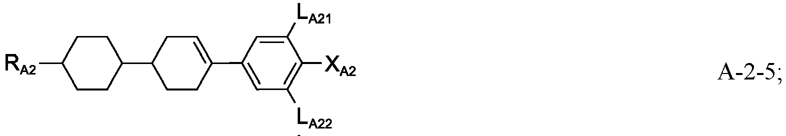 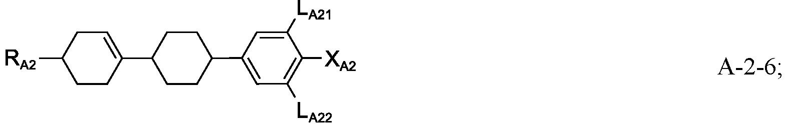 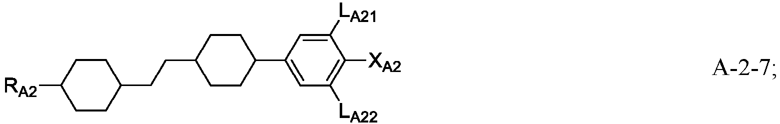 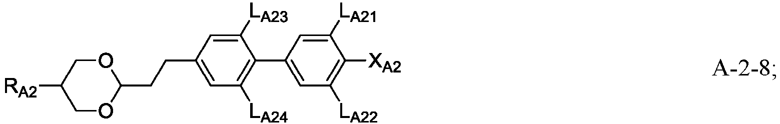 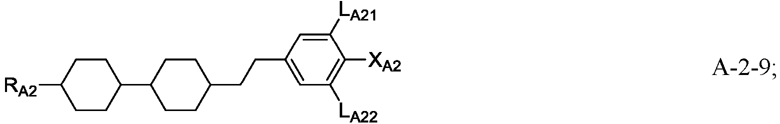 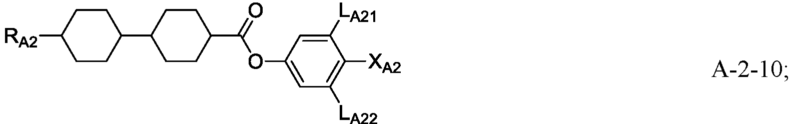 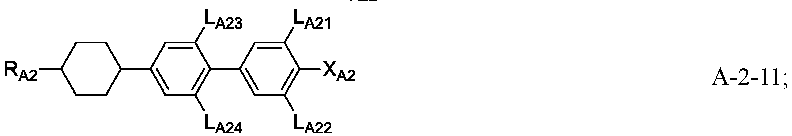 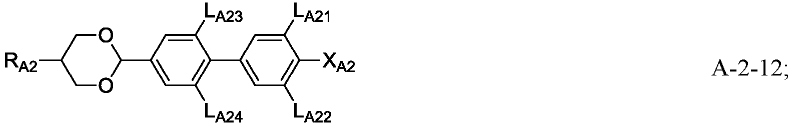 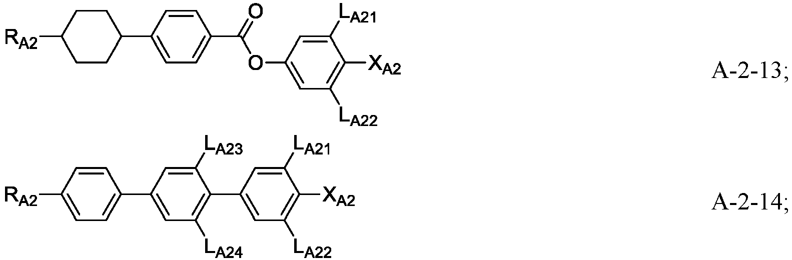 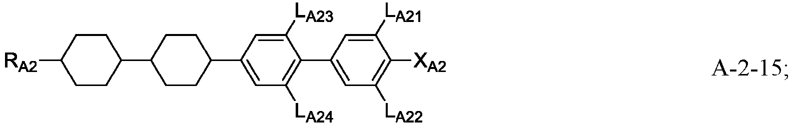 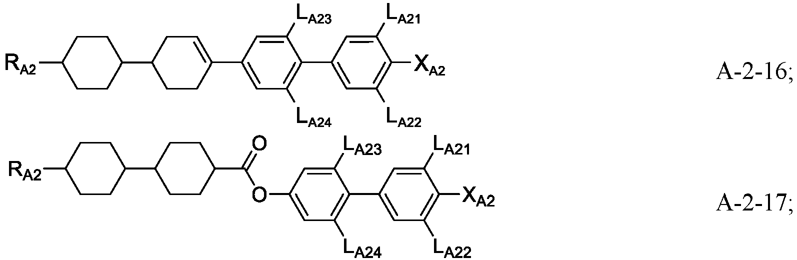 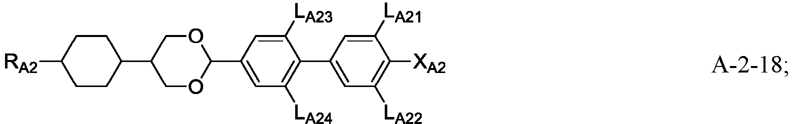 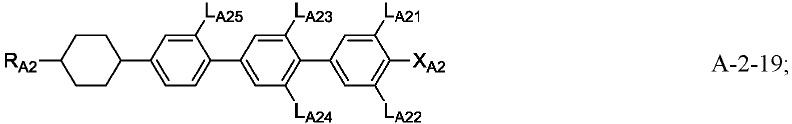 and 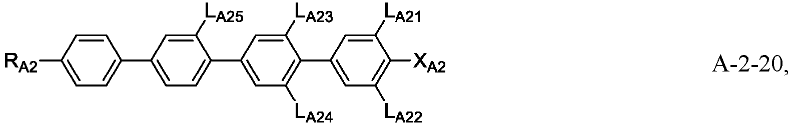 wherein,
R_{A2} represents C₁₋₈ linear alkyl, one or more than two nonadjacent -CH₂- in the C₁₋₈ linear alkyl can each be independently replaced by -CH=CH-, -C≡C-, -O-, -CO-, -CO-O- or -O-CO-, and one or more -H present in these group can each be independently substituted by -F or -Cl;
L_{A21}, L_{A22}, L_{A23}, L_{A24} and L_{A25} each independently represents -H or -F; and
X_{A2} represents -F, -CF₃, -OCF₃ or -CH₂CH₂CH=CF₂.

In some embodiments of the present invention, the compound of general formula A-2 provides (in percentage by weight) 0.1%-50% (including any value within this range) of the liquid crystal composition, for example, 0.1%, 1%, 4%, 6%, 8%, 10%, 12%, 14%, 16%, 18%, 20%, 22%, 24%, 26%, 28%, 30%, 32%, 34%, 36%, 38%, 40%, 42%, 44%, 46%, 48%, 50%, or a range between any two numerical values.

In some embodiments of the present invention, the liquid crystal composition further comprises at least one additive.

In addition to the above compounds, the liquid crystal composition of the present invention may also contain conventional nematic liquid crystal compound, smectic liquid crystal compound, cholesteric liquid crystal compound, dopants, antioxidant, ultraviolet absorber, infrared absorber, polymerizable monomer or light stabilizer and so forth.

Dopants which may be preferably added to the liquid crystal composition according to the present invention are shown below:

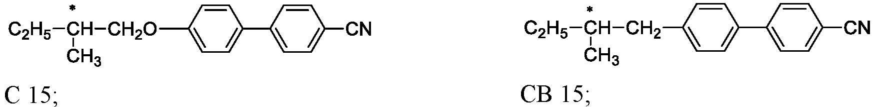

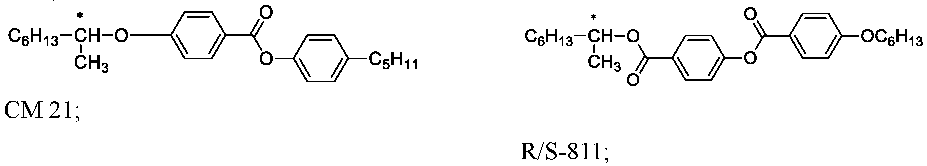

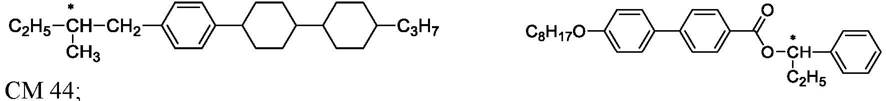

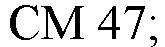

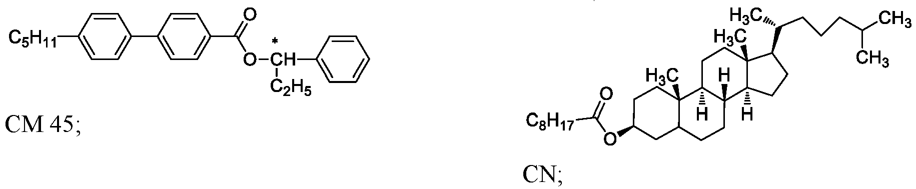

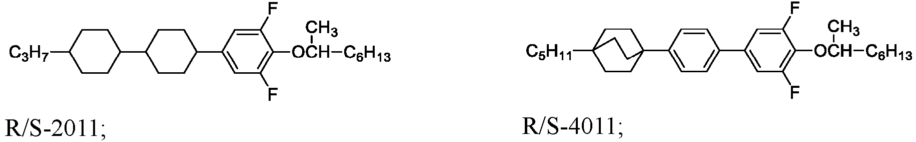

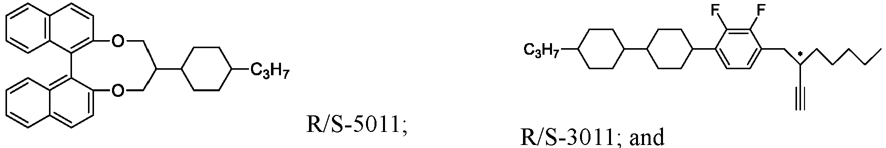

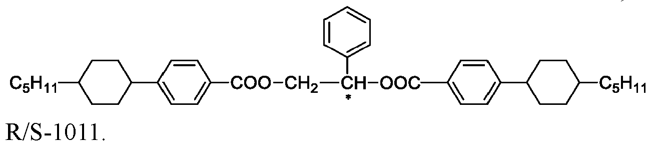

In some embodiments of the present invention, the dopant provides (in percentage by weight) 0%-5% of the liquid crystal composition; preferably, the dopant provides (in percentage by weight) 0.01%-1% of the liquid crystal composition.

Further, additives (such as antioxidant, light stabilizer, UV absorber and the forth) used in the liquid crystal composition of the present invention are preferably selected from the following substances: 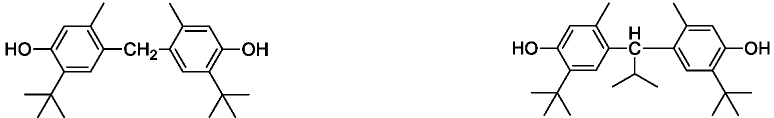 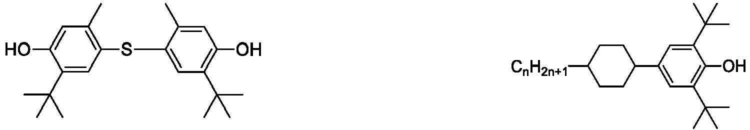 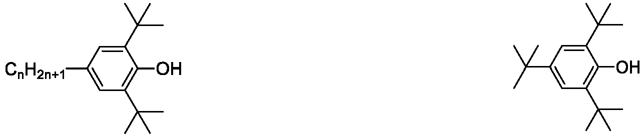 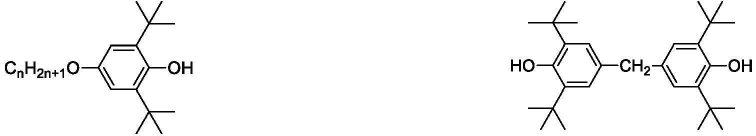 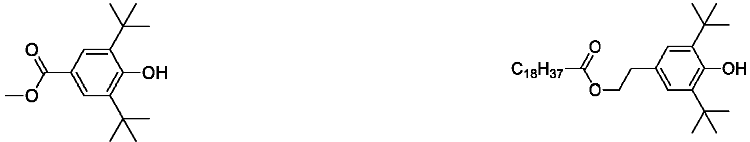 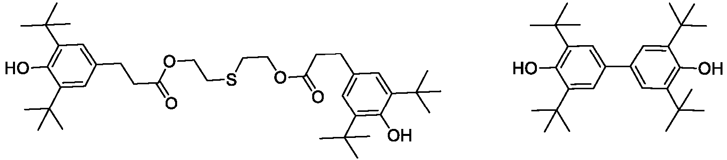 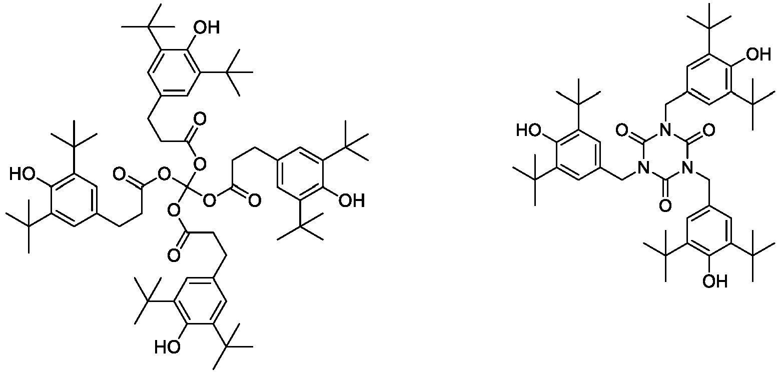 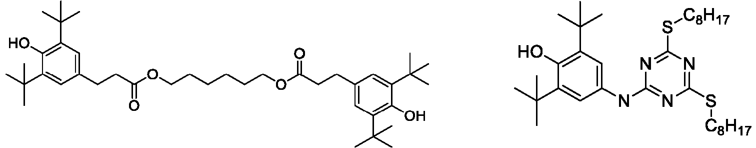 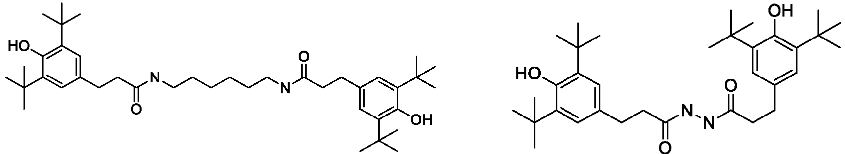 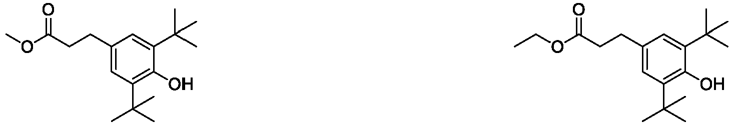 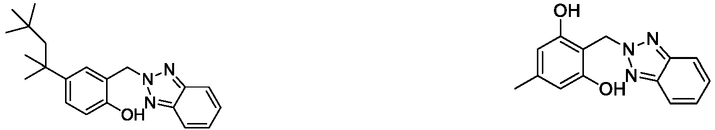 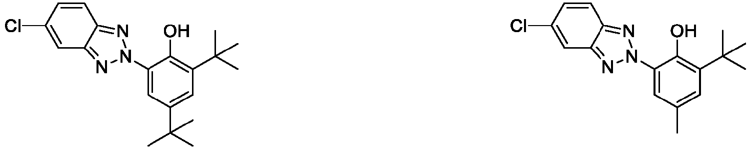 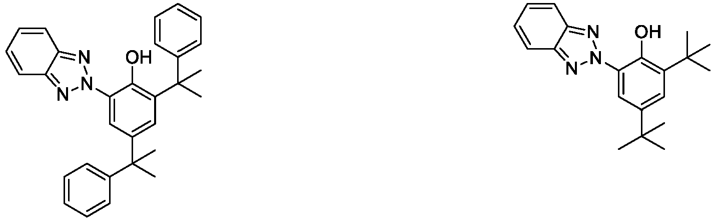 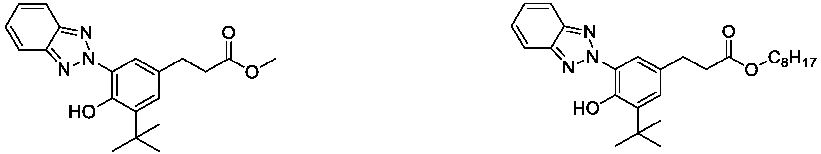 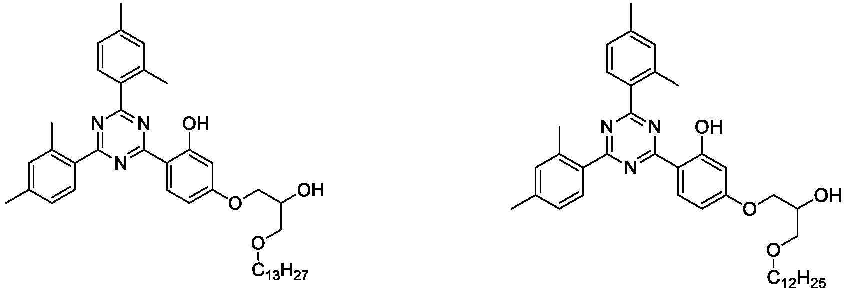 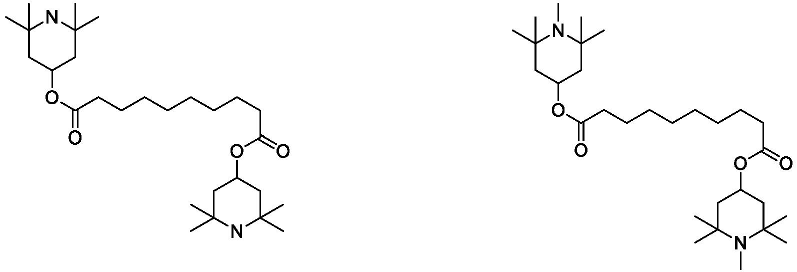 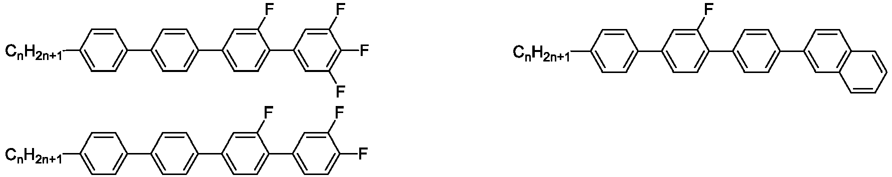 wherein, n represents a positive integer of 1-12.

Preferably, the antioxidant is selected from the compounds set forth below: 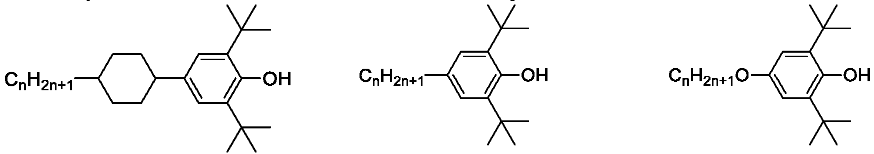 wherein, n represents a positive integer of 1-12.

In some embodiments of the present invention, the additive provides (in percentage by weight) 0%-5% of the liquid crystal composition; preferably, the additive provides (in percentage by weight) 0.01%-1% of the liquid crystal composition.

The liquid crystal composition containing polymerizable compound of the present invention can be polymerized even in the absence of a polymerization initiator. However, polymerization initiator(s) may also be contained therein to promote polymerization. Regarding the polymerization initiators, the following can be listed: benzoin ethers, benzophenones, acetophenones, benzyl ketals, acylphosphine oxides, and the like.

In another aspect, the present invention further provides a liquid crystal display device, the liquid crystal display device comprises the liquid crystal composition described above.

In some embodiments of the present invention, the above liquid crystal composition is particularly suitable for PSA-VA, PSA-OCB, PSA-IPS, PSA-FFS and PSA-TN type liquid crystal display devices.

**Beneficial effects:** As compared with the prior art, the liquid crystal composition comprising the self-aligning agent of general formula O in the present invention has a lower residue concentration, a smaller roughness, a better alignment effect as well as a better low-temperature storage stability and a better pre-tilt angle stability while maintaining an appropriate clearing point, an appropriate optical anisotropy, an appropriate absolute value of dielectric anisotropy, a larger K value (K₁₁ and K₃₃) and a smaller rotational viscosity.

### Detailed Embodiments

The present invention will be illustrated by combining the detailed embodiments below. It should be noted that, the following examples are exemplary embodiments of the present invention, which are only used to illustrate the present invention, not to limit it. Other combinations and various modifications within the conception of the present invention are possible without departing from the subject matter or scope of the present invention.

In the present application, unless otherwise specified, the proportions used herein are weight ratios, and temperatures are Celsius temperatures.

For the convenience of the expression, the group structures of each compound in the following Examples are represented by the codes listed in Table 1:

**Table 1. Codes of the group structures of the compounds**

| Unit structure of group | Code | Name of group |
|---|---|---|
| 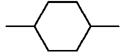 | C | 1,4-cyclohexylidene |
| 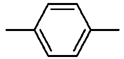 | P | 1,4-phenylene |
| 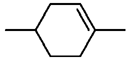 | L | 1,4-cyclohexenylene |
| 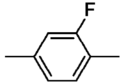 | G | 2-fluoro-1,4-phenylene |
| 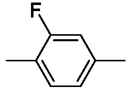 | G' | 3-fluoro-1,4-phenylene |
| 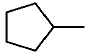 | C(5) | 1-cyclopentyl |
| 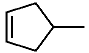 | C(5,V) | 1-cyclopentene |
| 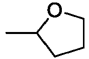 | THF | tetrahydrofuran-2-yl |
| 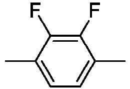 | W | 2,3-difluoro-1,4-phenylene |
| 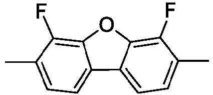 | B(O) | 4,6-difluoro-dibenzo[b,d]furan-3,7-diyl |
| 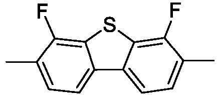 | B(S) | 4,6-difluoro-dibenzo[b,d]thiophene-3,7-diyl |
| 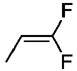 | V(2F) | difluorovinyl |
| -F | F | fluorine substituent |
| -O- | O | oxygen bridge group |
| -CH=CH- or -CH=CH₂ | V | vinylidene or vinyl |
| -CH₂O- | 1O | methyleneoxy |
| -CH₂CH₂- | 2 | ethyl bridge bond |
| -CₙH₂ₙ₊₁ or -CₙH₂ₙ- | n (n represents a positive integer of 1-12) | alkyl or alkylene |

Take the compound with the following structural formula as an example:

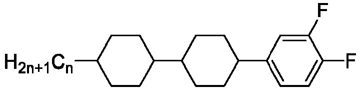

Represented by the codes listed in Table 1, this structural formula can be expressed as nCCGF, in which, n in the code represents the number of carbon atoms of the alkyl on the left, for example, n is "3", meaning that the alkyl is -C₃H₇; C in the code represents 1,4-cyclohexylidene, G represents 2-fluoro-1,4-phenylene, and F represents fluoro substituent.

The abbreviated codes of the test items in the following Examples are as follows:

| | |
|---|---|
| Cp | clearing point (nematic-isotropic phases transition temperature, °C) |
| Δn | optical anisotropy (589 nm, 20°C) |
| Δε | dielectric anisotropy (1 KHz, 20°C) |
| K₁₁ | splay elastic constant (20°C) |
| K₃₃ | bend elastic constant (20°C) |
| Rₐ | surface roughness (nm) |
| γ₁ | rotational viscosity (mPa·s, 20°C) |
| t_{-20°C} | low-temperature storage time (day, -20°C) |
| PTA | pre-tilt angle (°, 20°C) |
| ΔPTA | stability of pre-tilt angle (change in pre-tilt angle after applying voltage for a fixed time, °) |

wherein,
Cp: measured with melting point apparatus.
Δn: measured with an Abbe refractometer under sodium lamp (589 nm) light source at 20°C.
Δε=ε_{//}-ε_{⊥}, in which, ε_{//} is the dielectric constant parallel to the molecular axis, ε_{⊥} is the dielectric constant perpendicular to the molecular axis, the test conditions: 20°C, 1 KHz, VA type test cell with a cell gap of 6 µm.
γ₁: measured using a LCM-2 type liquid crystal physical property evaluation system; the test conditions: 20°C, 160 V-260 V, test cell gap 20 µm.
K₁₁ and K₃₃: calculated from the C-V curve of the liquid crystal measured using LCR meter and a VA test cell, the test conditions: cell gap 6 µm, V = 0.1~20 V, 20°C.
t_{-20°C}: placing the nematic liquid crystal media in glass vials, storing at -20 °C, and recording the time when crystal precipitation is observed, wherein, 7D NG represents crystal precipitation is observed after stored at -20°C for 7 days, 10D OK represents no crystal precipitation is observed after stored at -20°C for 10 days.
Ra: after UV photopolymerization of the polymerizable compound contained in the liquid crystal composition, the liquid crystal molecules are rinsed and then the surface roughness of the polymerized polymer layer is tested with an atomic force microscope (AFM).

Alignment effect: the liquid crystal containing the self-aligning agent and polymerizable compound is filled into a test cell with ITO on both sides (without PI layer, cell gap 3.2 µm). The test cell filled with the liquid crystal is placed in an oven at 120°C and heated for 1 hour. The test cell is cooled to room temperature at room temperature and then placed in a fixture with upper and lower linear polarizers attached thereto (the light transmission axes of the upper and lower polarizers are orthogonal at 90°). The alignment effect of the liquid crystal on the white backlight panel is observed. If it is all black, it indicates a good alignment effect. If there is light leakage in the corner areas around the test cell, the alignment effect is average. If there is also light leakage in the middle area of the test cell, the alignment effect is poo.

Residue concentration: after applying UV1 (5.5 mw/cm⁻², 313 nm) irradiation for 180 s and UV2 (0.25 mw/cm⁻², 313 nm) irradiation for 90 min, the liquid crystal eluted from the liquid crystal test cell is detected by high-performance liquid chromatography (HPLC), and the concentration of polymerizable compound and self-aligning agent therein is called residue concentration (ppm).

PTA: measured by crystal rotation method. Liquid crystals are filled into VA type test cell (cell gap 3.5 µm), and irradiated with ultraviolet light as described in UV1 step with the application of a voltage (15 V, 60 Hz), causing polymerization of the polymerizable compound and generation of pre-tilt angle PTA1, the liquid crystal composition with the generated pre-tilt angle PTA1 are subsequently irradiated with ultraviolet light as described in UV2 step to remove residual polymerizable compounds in the PTA1 state, and at this time the pre-tilt angle formed by the polymerizable compound is PTA2. In the present invention, the polymerization rate of the polymerizable compound is investigated through the comparation of the pre-tilt angles formed after the same exposure time of UV1 irradiation (the smaller the pre-tilt angle, the faster the polymerization rate) or the times for forming the same pre-tilt angle (the shorter the time required for forming the same pre-tilt angle, the fast the polymerization rate).

ΔPTA: after the test cell used in the measurement of PTA is subjected to UV1 and UV2 steps to form a pre-tilt angle of 88±0.2°, 60 Hz SW wave, AC voltage (20 V) and DC voltage (2 V) are applied to the test cell at 40°C with the presence of backlight. After a fixed time period, the pre-tilt angle of the test cell is tested, and ΔPTA (165 h) = PTA₍ᵢₙᵢₜᵢₐₗ₎ - PTA_{(165 h)}, the smaller the ΔPTA (165 h), the better the stability of the pre-tilt angle.

The self-aligning agent of general formula O provided in the present invention may be prepared by conventional organic synthesis methods, wherein the methods for introducing a target terminal group, a cyclic structure and a linking group into a starting material can be found in the following literatures: Organic Synthesis (John Wiley & Sons Inc.), Organic Reactions (John Wiley & Sons Inc.), Comprehensive Organic Synthesis (Pergamon Press), and the like.

The synthetic methods of the linking groups in the self-aligning agent of general formula O can refer to the following schemes, wherein MSG¹ or MSG² is a monovalent organic group having at least one ring, and a plurality of MSG¹ (or MSG²) used in the following schemes can be the same or different.

### (1) Synthesis of single bond

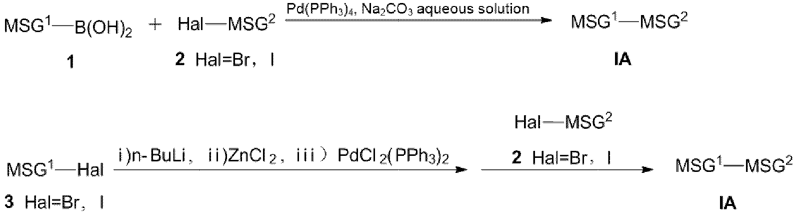

Compound **IA** with a single bond is prepared by allowing an aryl boronic acid **1** to react, in the presence of an aqueous carbonate solution and a catalyst such as Tetrakis(triphenylphosphine)palladium (Pd(PPh₃)₄), with Compound **2** prepared according to a well-known method. Compound **IA** with a single bond may also be prepared by allowing Compound **3** prepared according to a well-known method to react with n-butyllithium (n-BuLi) and subsequently with zinc chloride, and further with Compound **2** in the presence of a catalyst such as dichlorobis(triphenylphosphine)palladium (PdCl₂(PPh₃)₂).

### (2) Synthesis of -CO-O- and -O-CO-

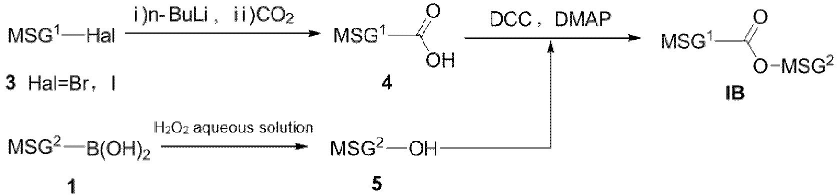

A carboxylic acid **4** is obtained by allowing Compound **3** to react with n-butyllithium and then with carbon dioxide. In the presence of 1,3-dicyclohexyl carbodiimide (DCC) and 4-dimethylaminopyridine (DMAP), Compound **4** and Compound **5** synthesized by a well-known method are dehydrated to synthesize Compound **IB** having -COO-. A compound having -OCO-can also be synthesized by this method.

### (3) Synthesis of -CF₂O- and -OCF₂-

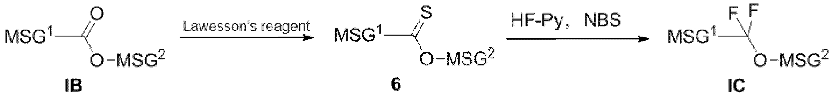

Compound **6** is obtained by treating Compound **IB** with a thiation agent such as Lawesson's reagent. Compound **IC** having -CF₂O- is prepared by fluorinating Compound 6 with a hydrogen fluoride-pyridine (HF-Py) and N-bromosuccinimide (NBS). A compound having -OCF₂- can also be synthesized by this method.

### (4) Synthesis of -CH=CH-

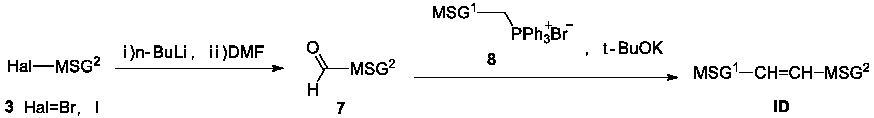

Compound 7 is obtained by allowing Compound 3 to react with n-butyllithium and then with formamide such as N,N-dimethylformamide (DMF). Compound **ID** is prepared by allowing phosphorus ylide, which is generated by reacting a phosphonium salt **8** prepared according to a well-known method with potassium t-butoxide (t-BuOK), to react with Compound 7. A cis isomer is generated by the above method depending on reaction conditions. It should be understood that the cis isomer may be isomerized into a trans isomer according to a well-known method, when necessary.

### (5) Synthesis of -CH₂CH₂-

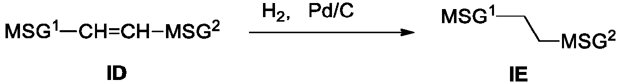

Compound **IE** may be prepared by hydrogenating Compound **ID** with a catalyst such as palladium on carbon (Pd/C).

### (6) Synthesis of -CH₂O- or -OCH₂-

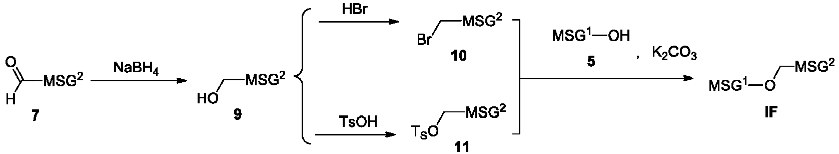

Compound **9** is obtained by reducing Compound 7 with sodium boron hydride (NaBH₄). Compound **10** is then obtained by halogenating Compound **9** with hydrobromic acid. Alternatively, Compound **11** is obtained by protecting the hydroxyl group of Compound **9** with p-toluenesulfonic acid (TsOH). Then, Compound **IF** is prepared by allowing Compound **10** or Compound **11** to react with Compound **5** in the presence of potassium carbonate. A compound having -OCH₂- may also be synthesized according to these methods.

### (7) Synthesis of -CH=CF₂

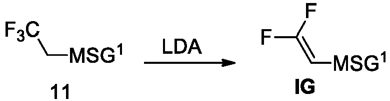

Compound **IG** is prepared by removing hydrofluoric acid from the terminal chain of Compound **11** using a tetrahydrofuran solution of lithium diisopropylamide (LDA).

For ring structures such as 1,4-cyclohexylene, 1,3-dioxane-2,5-diyl, 1,4-phenylene, 2-fluoro-1,4-phenylene, 2,3-difluoro-1,4-phenylene, 2,5-difluoro-1,4-phenylene, 2,6-difluoro-1,4-phenylene, 2,3,5,6-tetrafluoro-1,4-phenylene, the starting materials are already commercially available or their synthesis methods are well-known in the art.

### Synthetic Preparation Example 1

The preparation process of Compound of formula O-1-2-1-1 is as follows:

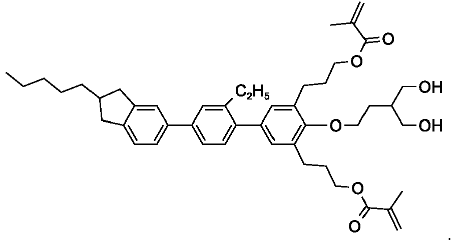

### Step 1. Synthesis of Compound of formula 1-c

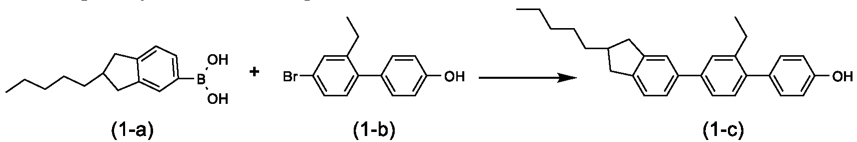

46.4 g Compound of formula 1-a ((2-pentyl-2,3-dihydro-1H-inden-5-yl)boronic acid), 55.4 g Compound of formula 1-b (4-(4-bromo-2-ethylphenyl)phenol) and 33.2 g potassium carbonate were added into a reaction flask, and fully dissolved in toluene. 1 g Pd(dppf)₂Cl₂ was added under N₂ protection, heated to reflux under N₂ protection and reacted for 4 h. Spot detection on the plate shows the disappearance of the raw materials. The reaction was extracted with toluene and chromatographed, and the solvent was removed by dry spinning. The residue was recrystallized 3 times with 800 mL mixed solvent of toluene and ethanol (the volume ratio of toluene to ethanol was 3:1) to afford 56.9 g Compound of formula 1-c (4-[2-ethyl-4-(2-pentyl-2,3-dihydro-1H-inden-5-yl)phenyl]phenol) as white solid, yield 74%.

### Step 2. Synthesis of Compound of formula 1-d

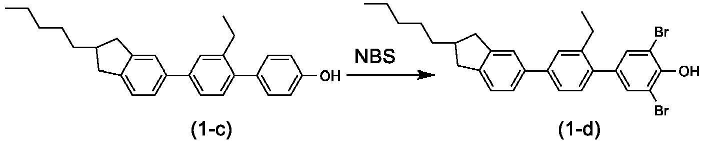

56.9 g Compound of formula 1-c and 3.0 g Diisopropylamine were added into a reaction flask, and fully dissolved in tetrahydrofuran. 49 g N-bromosuccinimide (NBS) was added in batches at a controlled temperature of 0~-5°C. The reaction was naturally warmed and reacted for 6 h. 0.5 L sodium sulfite aqueous solution was added to neutralize the reaction solution until a neutral pH and the solution was separated. The aqueous layer was twice extracted with 300 mL Dichloromethane, and the organic phase was combined, washed twice with 0.5 L water, dried, purified by 30 g silica gel column, eluted with 1 L Dichloromethane, and recrystallized with 0.5 L mixed solvent of n-heptane and ethanol (the volume ratio of n-heptane and ethanol is 10: 1) to afford 70.0 g yellow Compound of formula 1-d (2,6-dibromo-4-[2-ethyl-4-(2-pentyl-2,3-dihydro-1H-inden-5-yl)phenyl]phenol), yield: 87%.

### Step 3. Synthesis of Compound of formula 1-e

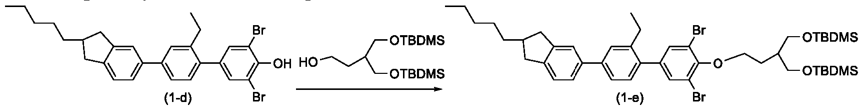

Under N₂ protection, 70.0 g Compound of formula 1-d and 45.0 g Compound 4-[(tert-butyldimethyl)oxy]-3-[(tert-butyldimethyl)oxy]methyl]butan-1-ol were added into a reaction flask, and fully dissolved in Diethyl azodicarboxylate (DEAD). In N₂ atmosphere, 0.3 g Triphenylphosphine was added, and the mixture was reacted at room temperature for 2 h. The reaction was purified, eluted with 2 L n-heptane, and recrystallized with 0.5 L mixed solvent of toluene and n-heptane (the volume ratio of toluene and n-heptane is 1:3) to afford 109.2 g Compound of 1-e (6-(2-{2,6-dibromo-4-[2-ethyl-4-(2-pentyl-2,3-dihydro-1H-inden-5-yl)phenyl]phenoxy}ethyl)-2 ,2,3,3,9,9,10,10-octamethyl-4,8-dioxa-3,9-disiloxane) as white solid, yield: 97%.

### Step 4. Synthesis of Compound of formula 1-f

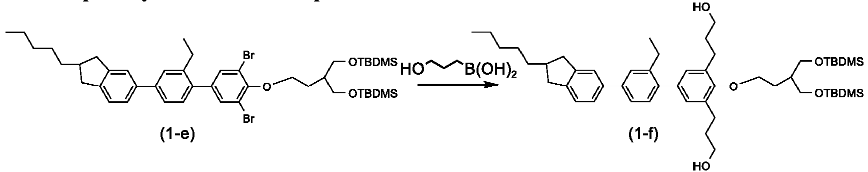

Under N₂ protection, 13 g Compound (3-hydroxypropyl)boronic acid, 109.2 g Compound of formula 1-e and 34.6 g anhydrous potassium carbonate were added into a reaction flask, and fully dissolved in N,N-dimethylformamide. Under N₂ protection, 0.3 g Tetrakis(triphenylphosphine)palladium was added, and the mixture was reacted at 70°C for 3 h. The reaction was purified, eluted with 2 L n-hexane, recrystallized with 0.2 L ethanol to afford 82 g Compound of formula 1-f (3-(2-{4-[(tert-butyldimethylsilyl)oxy]-3-{[(tert-butyldimethylsilyl)oxy]methyl}butoxy}-5-[2-et hyl-4-(2-pentyl-2,3-dihydro-1H-inden-5-yl)phenyl]-3-(3-hydroxypropyl)phenyl)propan-1-ol) as white solid, yield: 79.3%.

### Step 5. Synthesis of Compound of formula 1-g

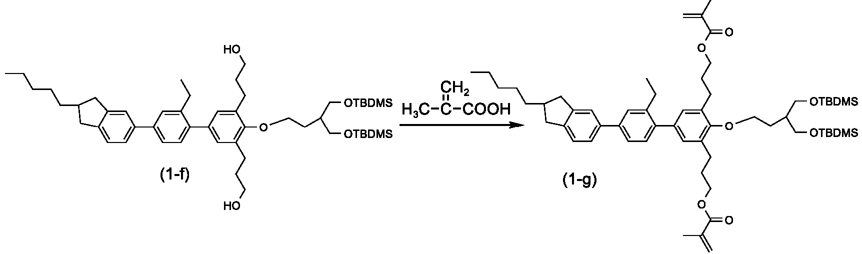

In a reaction flask, 35 g Dicyclohexyl carbodiimide (DCC) was fully dissolved in 100 mL Dichloromethane and set aside for later use. 82 g Compound of formula 1-f and 8.6 g Compound 2-methylprop-2-enoic acid were added into the reaction flask at room temperature and fully dissolved in Dichloromethane. 1.5 g 4-dimethylaminopyridine (DMAP) was added under stirring. The temperature was controlled at 0~10°C and 100 mL solution of Dicyclohexyl carbodiimide in Dichloromethane prepared above was added dropwise into the reaction system and the mixture was reacted overnight. The reaction was purified, eluted with 2 L n-hexane, recrystallized with 0.2 L acetonitrile to afford 89.1g Compound of formula 1-g (Propyl 3-(2-{4-[(tert-butyldimethylsilyl)oxy]-3-{[(tert-butyldimethylsilyl)oxy]methyl}butoxy}-5-[2-eth yl-4-(2-pentyl-2,3-dihydro-1H-inden-5-yl)phenyl]-3-{3-[(2-methylprop-2-enoyl)oxypropyl}ph enyl)2-methylprop-2-enoate) as white solid, yield: 93%.

### Step 6. Synthesis of Compound of formula O-1-2-1-1

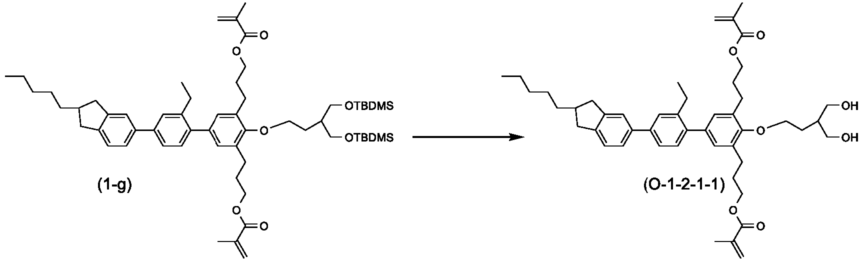

Under N₂ protection, 89.1 g Compound of formula 1-g and 7.3 g ammonium carbonate were added into a reaction flask, and fully dissolved in 0.5 L mixed solvent of acetic acid, water and tetrahydrofuran (the volume ratio of acetic acid, water and tetrahydrofuran is 10:5:2). The mixture was reacted at a controlled temperature of 70~80°C for 2 h, and extracted with 0.5 L toluene. The reaction was purified, eluted with 2 L toluene, recrystallized with 200 mL ethanol to afford 57.8 g Compound of formula 0-1-2-1-1 (Propyl 3-{5-[2-ethyl-4-(2-pentyl-2,3-dihydro-1H-inden-5-yl)phenyl]-2-[4-hydroxy-3-(hydroxymethyl)b utoxy]-3-{3-[(2-methylprop-2-enoyl)oxy]propyl}phenyl}2-methylprop-2-enoate) as white solid, yield: 85%.

Mass-to-charge ratio (m/z) of Compound of formula O-1-2-1-1 is 738.1 (M+), Elemental Analysis: C, 76.39; H, 8.46; O, 15.16;
H-NMR (300 MHz, CDCl3): 0.85-2.15 (m, 28H), 2.25-3.25 (m, 10H), 3.35-3.73 (m, 6H), 3.95-4.78 (m, 6H), 6.25-6.89 (m, 4H), 6.96-7.95 (m, 8H).

By adjusting the raw materials of Formula 1-b and using the same synthesis method as Synthetic Preparation Example 1, Compounds as shown in the following table can be synthesized accordingly.

| 1-b | Target Compound |
|---|---|
| 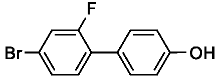 | 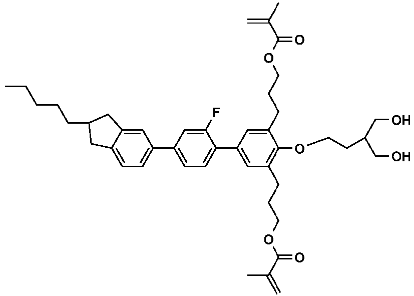 |
| | Compound of formula O-1-2-1-11 |
| | m/z is 728.1 (M+), Elemental Analysis: C, 74.15; H, 7.88; F, 2.61; O, 15.36. |
| | H-NMR (300 MHz, CDCl3): 0.85-2.15 (m, 25H), 2.25-3.25 (m, 8H), 3.35-3.73 (m, 6H), 3.95-4.78 (m, 6H), 6.25-6.89 (m, 4H), 6.96-7.95 (m, 8H). |
| 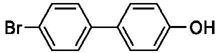 | 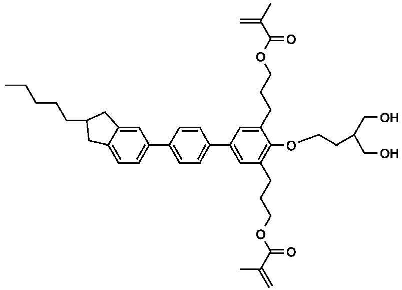 |
| | Compound of formula O-1-2-3-1 |
| | m/z is 710.1 (M+), Elemental Analysis: C, 76.02; H, 8.22; O, 15.75; H-NMR (300 MHz, CDCl3): 0.85-2.15 (m, 25H), 2.25-3.25 (m, 8H), 3.35-3.73 (m, 6H), 3.95-4.78 (m, 6H), 6.25-6.89 (m, 4H), 6.96-7.95 (m, 9H). |
| 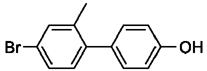 | 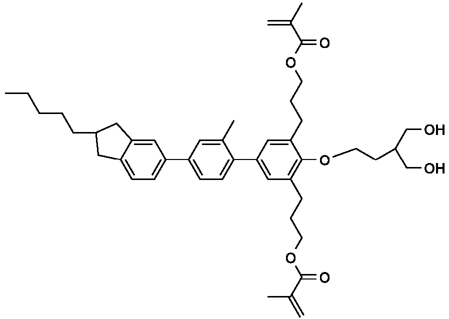 |
| | Compound of formula O-1-2-1-16 |
| | m/z is 724.1 (M+), Elemental Analysis: C, 76.21; H, 8.34; O, 15.45; H-NMR (300 MHz, CDCl3): 0.85-2.15 (m, 25H), 2.25-3.25 (m, 11H), 3.35-3.73 (m, 6H), 3.95-4.78 (m, 6H), 6.25-6.89 (m, 4H), 6.96-7.95 (m, 8H). |
| 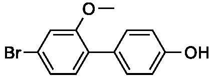 | 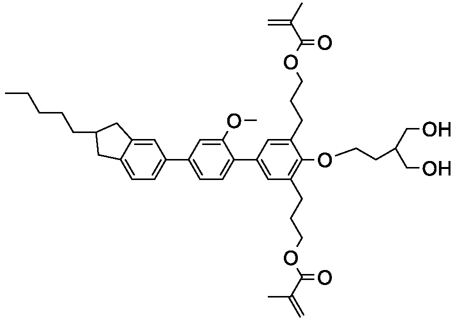 |
| | Compound of formula O-1-2-1-21 |
| | m/z is 740.1 (M+), Elemental Analysis: C, 74.56; H, 8.16; O, 17.27. |
| | H-NMR (300 MHz, CDCl3): 0.85-2.15 (m, 25H), 2.25-3.25 (m, 8H), 3.35-3.73 (m, 6H), 3.83 (s, 3H), 3.95-4.78 (m, 6H), 6.25-6.89 (m, 4H), 6.96-7.95 (m, 8H). |
| 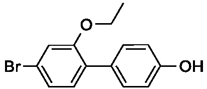 | 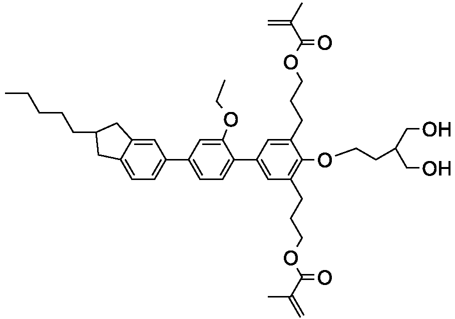 |
| | Compound of formula O-1-2-1-6 |
| | m/z is 754.1 (M+), Elemental Analysis: C, 74.77; H, 8.28; O, 16.95; H-NMR (300 MHz, CDCl3): 0.85-2.15 (m, 28H), 2.25-3.25 (m, 8H), 3.35-3.73 (m, 6H), 3.95-4.80 (m, 8H), 6.25-6.89 (m, 4H), 6.96-7.95 (m, 8H). |

### Synthetic Preparation Example 2

The preparation process of Compound of formula O-1-2-1-15 is as follows:

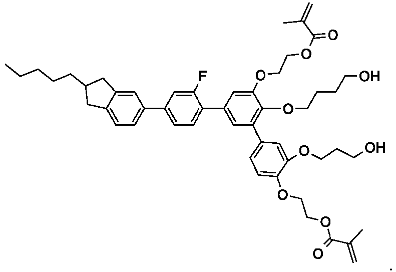

### Step 1. Synthesis of Compound of formula 2-c

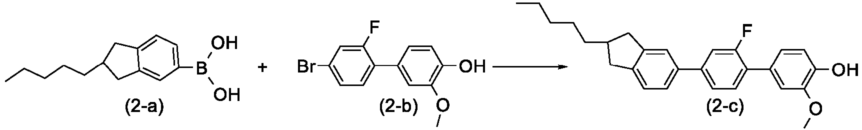

23.3 g Compound of formula 2-a, 29.7 g Compound of formula 2-b and 25.4 g anhydrous sodium carbonate were added into a reaction flask, and fully dissolved in toluene. In N₂ atmosphere, 0.1 g Pd-132 was added, and the mixture was reacted for 4 h. The mixture was adjusted to acidity with 1 M dilute hydrochloric acid, followed by liquid separation. The organic phase was washed with water, and the solvent was evaporated under reduced pressure. The crude product was purified by silica gel column chromatography using 2 L mixed solvent of n-heptane and toluene (the volume ratio of n-heptane and toluene was 1:1) and recrystallized to afford 36.4 g Compound of formula 2-c (4-[2-fluoro-4-(2-pentyl-2,3-dihydro-1H-inden-5-yl)phenyl]-2-methoxyphenol) as white crystal, yield 90%.

### Step 2. Synthesis of Compound of formula 2-d

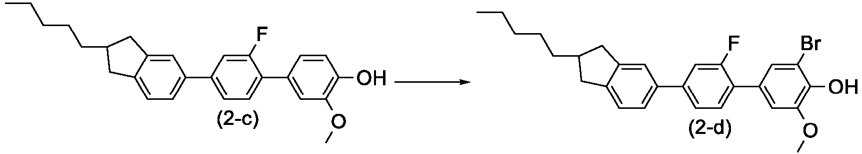

36.4 g white crystal of Compound of formula 2-c was added into a reaction flask, and fully dissolved in carbon tetrachloride. In N₂ atmosphere, 0.2 g Fe powder was added, followed by dropwise addition of 16 g liquid bromine over 1 h. After 30 min, the excess bromine was removed by washing with sodium bisulfite solution. The organic phase was washed with water, and the solvent was evaporated under reduced pressure. The crude product was recrystallized with 180 mL mixed solvent of n-heptane and ethanol (the volume ratio of n-heptane and ethanol is 5:1) to afford 35.8 g Compound of formula 2-d (2-bromo-4-[2-fluoro-4-(2-pentyl-2,3-dihydro-1H-inden-5-yl)phenyl]-6-methoxyphenol) as white crystal, yield 82%.

### Step 3. Synthesis of Compound of formula 2-e

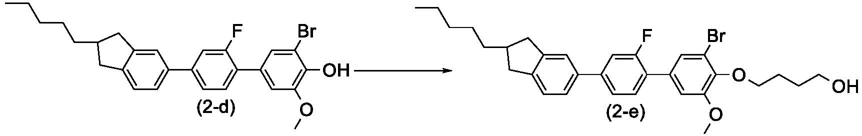

35.8 g white crystal of Compound of formula 2-d and 10.6 g sodium carbonate were added into a reaction flask, and fully dissolved in tetrahydrofuran. In N₂ atmosphere, 15.3 g 4-bromobutanol was added, and the mixture was reacted at 80°C for 6 h. The reaction mixture was poured into 500 mL water and extracted with 700 mL toluene. The organic phase was washed with 300 mL water, and the solvent was evaporated under reduced pressure. The crude product was recrystallized with 300 mL mixed solvent of n-heptane and ethanol (the volume ratio of n-heptane and ethanol was 4:1) to afford 35.6 g Compound of formula 2-e (4-{2-bromo-4-[2-fluoro-4-(2-pentyl-2,3-dihydro-1H-inden-5-yl)phenyl]-6-methoxyphenoxy}b utan-1-ol), yield 86%.

### Step 4. Synthesis of Compound of formula 2-f

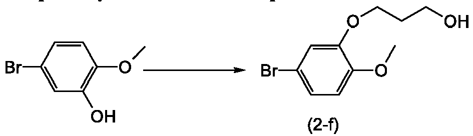

20.3 g 5-bromo-2-methoxyphenol and 13.8 g sodium carbonate were added into a reaction flask, and fully dissolved in tetrahydrofuran. In N₂ atmosphere, 20.7 g 3-bromopropanol was added at a controlled temperature of 80°C, and the mixture was reacted for 6 h and extracted with 200 mL toluene. The organic phase was washed with water, and the solvent was evaporated under reduced pressure. The crude product was recrystallized with 50 mL n-heptane to afford 19.8 g Compound of formula 2-f (3-(5-bromo-2-methoxyphenoxy)propan-1-ol), yield 76%.

### Step 5. Synthesis of Compound of formula 2-g

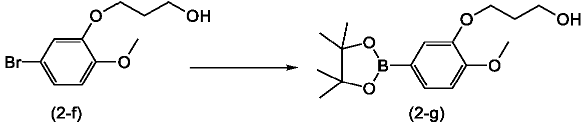

19.8 g Compound of formula 2-f, 25.3 g bis(pinacolato)diboron and 8.2 g anhydrous sodium carbonate were added into a reaction flask, and fully dissolved in toluene. In N₂ atmosphere, 0.4 g Tetrakis(triphenylphosphine)palladium was added, and the mixture was refluxed for 4 hours. The reaction mixture was washed with water and evaporated under reduced pressure. The crude product was recrystallized with 100 mL n-heptane to afford 19.5 g Compound of formula 2-g (3-[2-methoxy-5-(tetramethyl-1,3,2-dioxaborolan-2-yl)phenoxy]propan-1-ol) as pale-yellow crystal, yield 83%.

### Step 6. Synthesis of Compound of formula 2-h

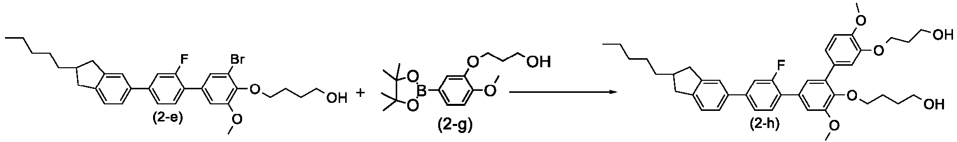

19.5 g pale-yellow crystal of Compound of formula 2-g, 35.6 g Compound of formula 2-e and 10.6 g anhydrous sodium carbonate were added into a reaction flask, and fully dissolved in toluene. In N₂ atmosphere, 0.5 g Tetrakis(triphenylphosphine)palladium was added, and the mixture was refluxed for 4 h. The reaction mixture was washed with water and evaporated under reduced pressure. The crude product was recrystallized with 600 mL mixed solvent of toluene and n-heptane (the volume ratio of toluene and n-heptane was 1:2) to afford 30.9 g Compound of formula 2-h (4-{4-[2-fluoro-4-(2-pentyl-2,3-dihydro-1H-inden-5-yl)phenyl]-2-[3-(3-hydroxypropoxy)-4-met hoxyphenyl]-6-methoxyphenoxy}butan-1-ol) as white crystal, yield 75%.

### Step 7. Synthesis of Compound of formula 2-i

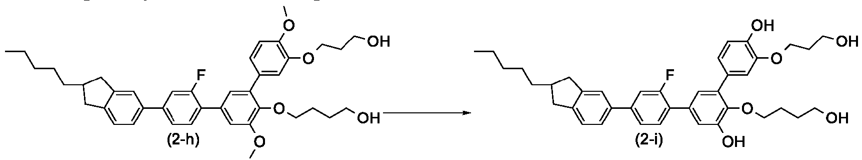

30.9 g white crystal of Compound of formula 2-h was added into a reaction flask, and fully dissolved in Dichloromethane. At -30°C, 35.8 g boron tribromide was added dropwise, and the mixture was maintained at this temperature and reacted for 3 h. The reaction mixture was washed with water, the organic phase was separated, and the solvent was evaporated under reduced pressure. The crude product was recrystallized with 100 mL mixed solvent of n-heptane and ethanol (the volume ratio of n-heptane and ethanol was 3:1) to afford 23.9 g Compound of formula 2-i (5-[2-fluoro-4-(2-pentyl-2,3-dihydro-1H-inden-5-yl)phenyl]-3-[4-hydroxy-3-(3-hydroxypropoxy )phenyl]-2-(4-hydroxybutoxy)phenol) as white solid, yield 81%.

### Step 8. Synthesis of Compound of formula 2-j

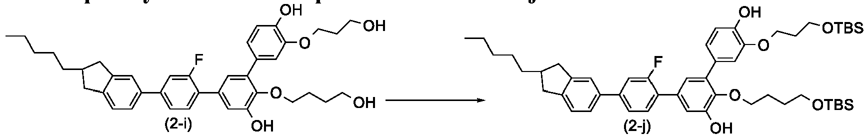

23.9 g white solid of Compound of formula 2-i and 2.8 g imidazole were added into a reaction flask, and fully dissolved in tetrahydrofuran. The solution was cooled to 0°C under nitrogen protection, and 5.9 g tert-butyldimethylsilyl chloride was added within 40 min, and the mixture was maintained at 0°C and reacted for 1.5 h. The reaction mixture was washed with 500 mL ammonium chloride solution, extracted with 500 mL methyl tert-butyl ether. The organic phase was separated, washed with water until neutral, dried, and rotary evaporated. The crude product was recrystallized with 100 mL mixed solvent of n-heptane and ethanol (the volume ratio of n-heptane and ethanol was 3:1) to afford 20.6 g Compound of formula 2-j (2-{4-[(tert-butyldimethylsilyl)oxy]butoxy}-3-(3-{3-[(tert-butyldimethylsilyl)oxy]propoxy}-4-h ydroxyphenyl)-5-[2-fluoro-4-(2-pentyl-2,3-dihydro-1H-inden-5-yl)phenyl]phenol) as white solid, yield 63%.

### Step 9. Synthesis of Compound of formula 2-k

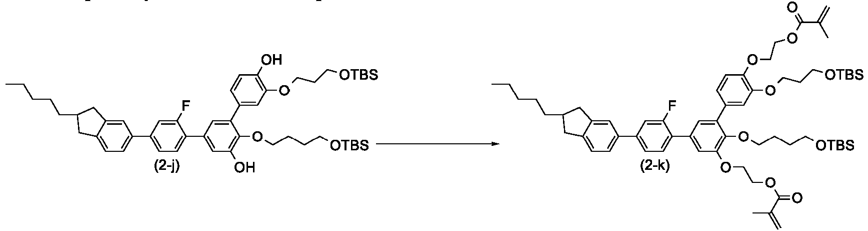

20.6 g white solid of Compound of formula 2-j and 13.8 g potassium carbonate were added into a reaction flask, and fully dissolved in tetrahydrofuran. In N₂ atmosphere, 19.3 g 2-bromoethyl methacrylate was added, and the mixture was reacted for 6 h at a controlled temperature of 70°C. 300 mL water was added and the mixture was extracted with 300 mL toluene. The organic phase was washed with water, dried and recrystallized with 50 mL mixed solvent of toluene and ethanol (the volume ratio of toluene and ethanol was 1:3), to afford 13 g Compound of formula 2-k (2-[4-(2-{4-[(tert-butyldimethylsilyl)oxy]butoxy}-5-[2-fluoro-4-(2-pentyl-2,3-dihydro-1H-inden -5-yl)phenyl]-3-{2-[(2-methylprop-2-enoyl)oxy]ethoxy}phenyl)-2-{3-[(tert-butyldimethylsilyl)o xy]propoxy}phenoxy]ethyl 2-methylprop-2-enoate), yield 50%.

### Step 10. Synthesis of Compound of formula O-1-2-1-15

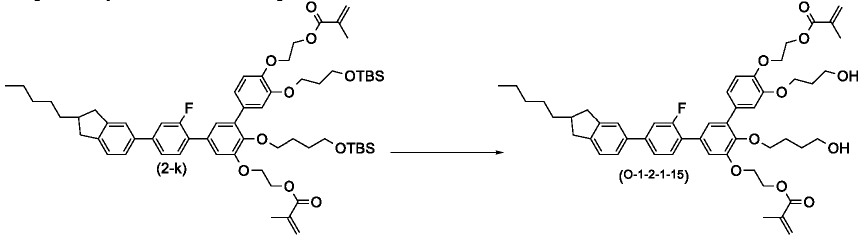

13 g Compound of formula 2-k was added into a reaction flask, and fully dissolved in tetrahydrofuran. The solution was cooled to 0°C, 7.5 mL 2 M dilute hydrochloric acid was slowly added dropwise, and the mixture was reacted at room temperature under stirring for 3 h. The temperature was controlled at 0°C, and 200 mL saturated sodium bicarbonate aqueous solution was added, and the mixture was extracted with 300 mL methyl tert-butyl ether. The organic phase was separated, washed with water until neutral, dried, and rotary evaporated. The crude product was recrystallized with 50 mL mixed solvent of n-heptane and ethanol (the volume ratio of n-heptane and ethanol was 4:1) to afford 7.2 g Compound of formula O-1-2-1-15 (ethyl 2-(4-{5-[2-fluoro-4-(2-pentyl-2,3-dihydro-1H-inden-5-yl)phenyl]-2-(4-hydroxybutoxy)-3-{2-[(2 -methylprop-2-enoyl)oxy]ethoxy}phenyl}-2-(3-hydroxypropoxy)phenoxy)2-methylprop-2-enoa te) as white solid, yield 70%.
m/z of Compound of formula O-1-2-1-15 is 852.1 (M+), Elemental Analysis: C, 71.81; H, 7.21; F, 2.23; O, 18.76;
H-NMR (300 MHz, CDCl3): 0.85-2.15 (m, 24H), 2.25-3.25 (m, 4H), 3.35-3.73 (m, 6H), 3.95-4.78 (m, 12H), 6.25-6.89 (m, 4H), 6.96-7.95 (m, 11H).

By adjusting the raw materials of Formula 2-b and using the same synthesis method as Synthetic Preparation Example 2, Compounds as shown in the following table can be synthesized accordingly.

| 2-b | Target Compound |
|---|---|
| 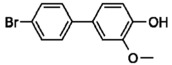 | 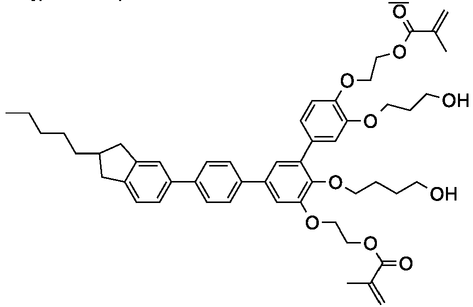 |
| | Compound of formula O-1-2-3-5 |
| | m/z is 834.1 (M+), Elemental Analysis: C, 73.36; H, 7.48; F, 2.23; O, 19.16; |
| | H-NMR (300 MHz, CDCl3): 0.85-2.15 (m, 24H), 2.25-3.25 (m, 4H), 3.35-3.73 (m, 6H), 3.95-4.78 (m, 12H), 6.25-6.89 (m, 4H), 6.96-7.95 (m, 12H). |
| 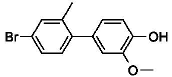 | 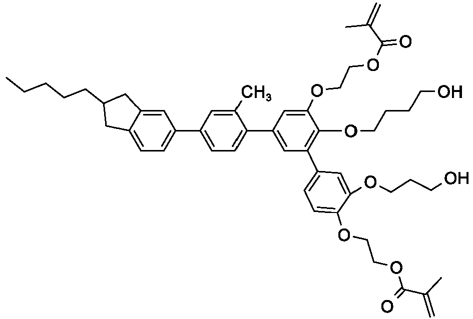 |
| | Compound of formula O-1-2-1-20 |
| | m/z is 848.1 (M+), Elemental Analysis: C, 73.56; H, 7.60; O, 18.84; |
| | H-NMR (300 MHz, CDCl3): 0.85-2.15 (m, 24H), 2.25-3.25 (m, 7H), 3.35-3.73 (m, 6H), 3.95-4.78 (m, 12H), 6.25-6.89 (m, 4H), 6.96-7.95 (m, 11H). |
| 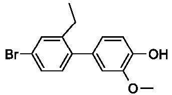 | 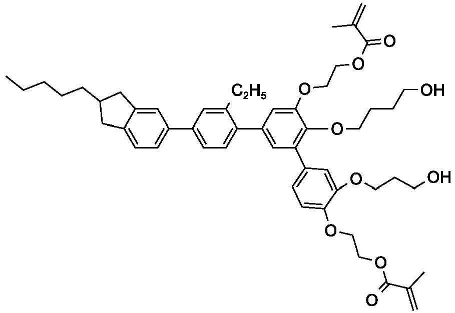 |
| | Compound of formula O-1-2-1-5 |
| | m/z is 862.1 (M+), Elemental Analysis: C, 73.75; H, 7.71; O, 18.54; |
| | H-NMR (300 MHz, CDCl3): 0.85-2.15 (m, 27H), 2.25-3.25 (m, 6H), 3.35-3.73 (m, 6H), 3.95-4.78 (m, 12H), 6.25-6.89 (m, 4H), 6.96-7.95 (m, 11H). |

The components used in the following Examples can either be synthesized by method known in the art or be obtained commercially. These synthetic techniques are conventional, and each of the obtained liquid crystal compounds is tested to meet the standards of electronic compounds.

The liquid crystal compositions are prepared in accordance with the ratios specified in the following Examples. The preparation of the liquid crystal compositions is proceeded by mixing in accordance with the ratios through conventional methods in the art, such as heating, ultrasonic wave, suspension and the like.

The structures of the polymerizable compounds used in each of the following Examples are shown in Table 2 below.

**Table 2 Polymerizable compounds used in Examples**

| Structural formula | General formula code |
|---|---|
| 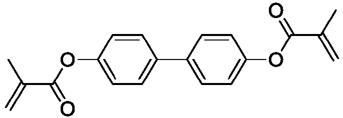 | RM-1-1 |
| 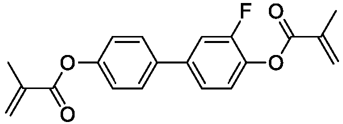 | RM-2-1 |
| 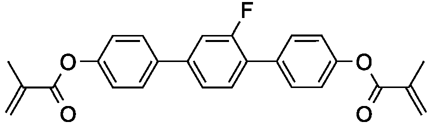 | RM-20-1 |

The structures of self-aligning agents used in each of the following Examples are shown in Table 3 below.

**Table 3 Self-aligning agents used in Examples**

| Component number | Structural formula | General formula code |
|---|---|---|
| D-1 | 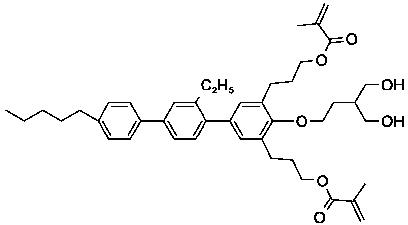 | |
| D-2 | 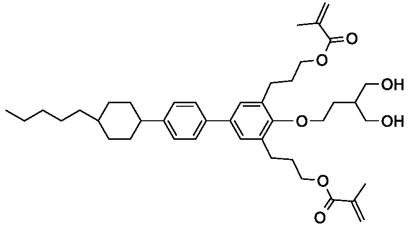 | |
| D-2' | 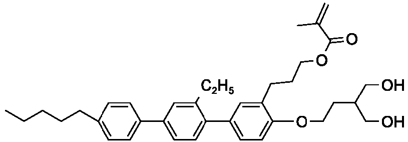 | |
| D-3 | 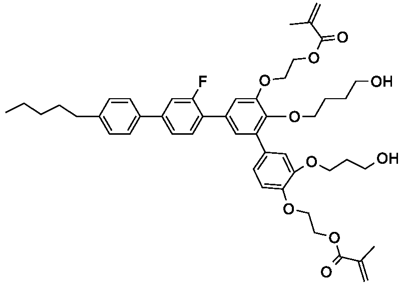 | |
| D-3' | 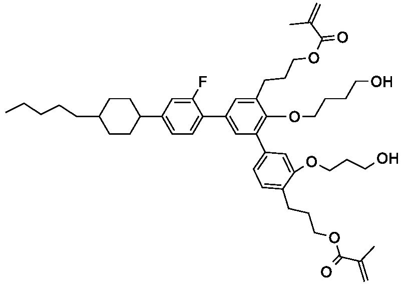 | |
| D-4 | 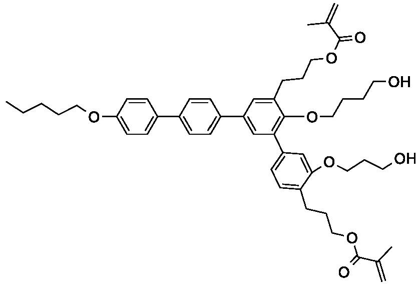 | |
| AD-1 | 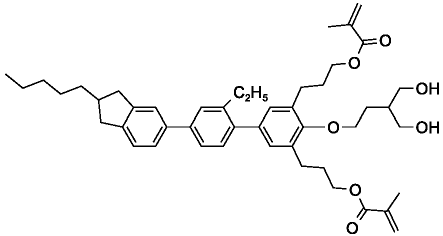 | 0-1-2-1-1 |
| AD-2 | 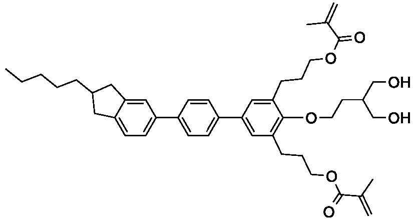 | 0-1-2-3-1 |
| AD-3 | 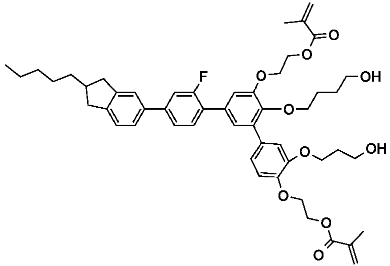 | 0-1-2-1-15 |
| AD-3' | 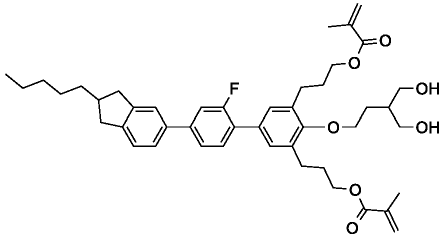 | 0-1-2-1-11 |
| AD-4 | 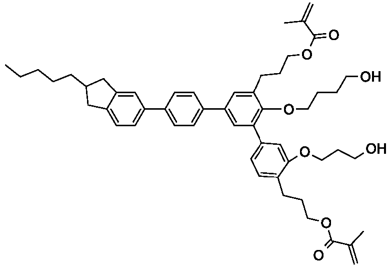 | 0-1-2-3-4 |
| AD-5 | 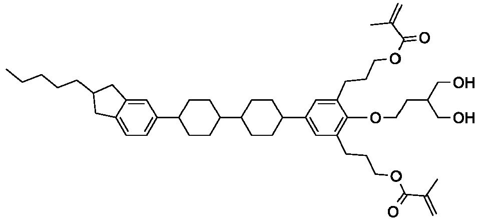 | 0-1-6-2 |

Host liquid crystal compositions Host-1, Host-2, Host-3, Host-4, Host-5 and Host-6 are prepared according to each compound and weight percentage listed in Table 4 and are tested by filling the same between two substrates of a liquid crystal display device.

**Table 4 Formulations and test results for performance parameters of host liquid crystal compositions**

| Component code | General formula code | Weight percentage | | | | | |
|---|---|---|---|---|---|---|---|
| | | Host-1 | Host-2 | Host-3 | Host-4 | Host-5 | Host-6 |
| 3CPWO4 | N-21 | 9 | | | 8 | | |
| 3C1OWO2 | N-7 | 7.5 | | | | | |
| 2CPWO2 | N-21 | | | | 5 | | 5 |
| 3CPWO2 | N-21 | | 2.5 | 7.5 | | 2.5 | |
| 3CPWO3 | N-21 | 9 | | | | | |
| 1VCPWO2 | N-53 | | | | 9 | | 9 |
| 2OPWO2 | N-19 | | 6 | | | 6 | |
| 3PWO2 | N-19 | | 9 | 15 | 10.5 | 9 | 10.5 |
| 2CC1OWO2 | N-15 | 7 | | | | | |
| 3CC1OWO2 | N-15 | 7 | | | | | |
| 4CC1OWO2 | N-15 | 8 | | | | | |
| 4C1OWO2 | N-7 | 5 | | | | | |
| 3CWO2 | N-2 | | 13 | 13 | 6 | 13 | |
| 3CWO4 | N-2 | | 2 | | | 2 | |
| 5CWO2 | N-2 | | | 5 | | | |
| 2CCWO2 | N-9 | | 6.5 | 5 | 4 | | 4 |
| 3CCWO2 | N-9 | | 13.5 | 12 | 13 | 13.5 | 13 |
| 3LWO2 | N-3 | | | | | | 6 |
| 2CLWO2 | N-12 | | | | | 6.5 | |
| 3CPO2 | M2-5 | 6 | | | | | |
| 3CC2 | M-1-2 | 15 | 14 | 17 | | 14 | |
| 5CC2 | M-1-9 | 9 | 4 | | | 4 | |
| 3CC4 | M-1-4 | 9 | 6 | | | 6 | |
| 3CCV | M-1-5 | | | | 28.5 | | 28.5 |
| 3CCV1 | M-1-6 | | 10.5 | 9.5 | 8 | 10.5 | 8 |
| 3CCP1 | M-11-1 | 2 | | | | | |
| 3CCP2 | M-11-2 | | 13 | 9 | | 8 | 4 |
| 3CPP1 | M-13-1 | | | 7 | | | |
| 3CPP2V1 | M-13-9 | | | | | 5 | |
| 3CPP2V | M-13-8 | | | | | | 4 |
| 5PP1 | M-4 | 6.5 | | | 8 | | 4 |
| 3PPO2 | M-4 | | | | | | 4 |
| Total | | 100 | 100 | 100 | 100 | 100 | 100 |
| Cp | | 76.4 | 74.9 | 75.7 | 76.2 | 76.8 | 77 |
| Δn | | 0.089 | 0.1 | 0.109 | 0.109 | 0.102 | 0.11 |
| Δε | | -3.4 | -3.1 | -3.3 | -3 | -3.1 | -2.6 |
| K₁₁ | | 14.5 | 11.7 | 14.6 | 14.2 | 12.1 | 14.5 |
| K₃₃ | | 13.8 | 14.9 | 15.6 | 15.7 | 15.3 | 16.1 |
| γ₁ | | 105 | 101 | 106 | 84 | 103 | 78 |

### Comparative Examples 1-3 and Examples 1-3

0.3 parts by weight of polymerizable compound RM-1-1 and 0.7 parts by weight of D-1, D-2 and D-3 respectively are added into 100 parts by weight of host liquid crystal composition Host-1 to prepare the liquid crystal compositions of Comparative Examples 1-3 respectively, and 0.3 parts by weight of polymerizable compound RM-1-1 and 0.7 parts by weight of AD-1, AD-2 and AD-3 are added into 100 parts by weight of host liquid crystal composition Host-1 to prepare the liquid crystal compositions of Examples 1-3 respectively. The physical property values of each of the obtained liquid crystal compositions showed negligible changes relative to perspective host liquid crystal composition. The obtained liquid crystal compositions are tested for performance by filling each liquid crystal composition into a "non-aligned" test cell (cell gap d is 3.5 µm, with ITO coatings (structured ITO for multi-domain switching mode) on both substrates, no alignment layer, and no passivation layer). The test results for relevant performances of the liquid crystal compositions of Comparative Examples 1-3 and Examples 1-3 are shown in Table 5 below.

**Table 5 Test results for performances of the liquid crystal compositions of Comparative Examples 1-3 and Examples1-3**

| | Comp. Ex. 1 | Comp. Ex. 2 | Comp. Ex. 3 | Ex. 1 | Ex. 2 | Ex. 3 |
|---|---|---|---|---|---|---|
| Residue conc. | 155 | 183 | 168 | 105 | 118 | 109 |
| Ra | 15.1 | 16.4 | 15.8 | 12.3 | 11.8 | 12.5 |
| Alignment effect | Fair | Fair | Poor | Good | Good | Good |

It can be seen from the comparison between Example 1 and Comparative Example 1, the comparison between Example 2 and Comparative Example 2 and the comparison between Example 3 and Comparative Example 3 that, the liquid crystal composition comprising the self-aligning agent of general formula O in the present invention has a lower residue concentration (105-118 ppm VS 155-183 ppm), a smaller roughness (11.8-12.5 nm VS 15.1-16.4 nm) and a better alignment effect while maintaining an appropriate clearing point, an appropriate optical anisotropy, an appropriate absolute value of dielectric anisotropy, a larger K value (K₁₁ and K₃₃) and a smaller rotational viscosity.

### Comparative Examples 4-6 and Examples 4-6

0.3 parts by weight of polymerizable compound RM-1-1 and 1 parts by weight of D-1, D-2 and D-3 respectively are added into 100 parts by weight of host liquid crystal composition Host-2 to prepare the liquid crystal compositions of Comparative Examples 4-6 respectively, and 0.3 parts by weight of polymerizable compound RM-1-1 and 1 parts by weight of AD-1, AD-2 and AD-3 are added into 100 parts by weight of host liquid crystal composition Host-2 to prepare the liquid crystal compositions of Examples 4-6 respectively. The physical property values of each of the obtained liquid crystal compositions showed negligible changes relative to perspective host liquid crystal composition. The obtained liquid crystal compositions are tested for performance by filling each liquid crystal composition into a "non-aligned" test cell (cell gap d is 3.5 µm, with ITO coatings (structured ITO for multi-domain switching mode) on both substrates, no alignment layer, and no passivation layer). The test results for relevant performances of the liquid crystal compositions of Comparative Examples 4-6 and Examples4-6 are shown in Table 6 below.

**Table 6 Test results for performances of the liquid crystal compositions of Comparative Examples 4-6 and Examples 4-6**

| | Comp. Ex. 4 | Comp. Ex. 5 | Comp. Ex. 6 | Ex. 4 | Ex. 5 | Ex. 6 |
|---|---|---|---|---|---|---|
| Residue conc. | 168 | 197 | 179 | 113 | 126 | 119 |
| Ra | 15.3 | 15.9 | 15.5 | 11.6 | 11.9 | 12.3 |
| Alignment effect | Fair | Fair | Poor | Good | Good | Good |

It can be seen from the comparison between Example 4 and Comparative Example 4, the comparison between Example 5 and Comparative Example 5 and the comparison between Example 6 and Comparative Example 6 that, the liquid crystal composition comprising the self-aligning agent of general formula O in the present invention has a lower residue concentration (113-126 ppm VS 168-197 ppm), a smaller roughness (11.6-12.3 nm VS 15.3-15.9 nm) and a better alignment effect while maintaining an appropriate clearing point, an appropriate optical anisotropy, an appropriate absolute value of dielectric anisotropy, a larger K value (K₁₁ and K₃₃) and a smaller rotational viscosity.

### Comparative Examples 7-9 and Example7-9

0.3 parts by weight of polymerizable compound RM-2-1 and 0.9 parts by weight of D-1, D-2 and D-3 respectively are added into 100 parts by weight of host liquid crystal composition Host-3 to prepare the liquid crystal compositions of Comparative Examples 7-9 respectively, and 0.3 parts by weight of polymerizable compound RM-2-1 and 0.9 parts by weight of AD-1, AD-2 and AD-3 are added into 100 parts by weight of host liquid crystal composition Host-3 to prepare the liquid crystal compositions of Examples 7-9 respectively. The physical property values of each of the obtained liquid crystal compositions showed negligible changes relative to perspective host liquid crystal composition. The obtained liquid crystal compositions are tested for performance by filling each liquid crystal composition into a "non-aligned" test cell (cell gap d is 3.5 µm, with ITO coatings (structured ITO for multi-domain switching mode) on both substrates, no alignment layer, and no passivation layer). The test results for relevant performances of the liquid crystal compositions of Comparative Examples 7-9 and Examples 7-9 are shown in Table 7 below.

**Table 7 Test results for performances of the liquid crystal compositions of Comparative Examples 7-9 and Examples 7-9**

| | Comp. Ex. 7 | Comp. Ex. 8 | Comp. Ex. 9 | Ex. 7 | Ex. 8 | Ex. 9 |
|---|---|---|---|---|---|---|
| Residue conc. | 163 | 189 | 172 | 110 | 123 | 117 |
| Ra | 15.5 | 16.1 | 15.4 | 11.8 | 12 | 12.4 |
| Alignment effect | Fair | Fair | Poor | Good | Good | Good |

It can be seen from the comparison between Example 7 and Comparative Example 7, the comparison between Example 8 and Comparative Example 8 and the comparison between Example 9 and Comparative Example 9 that, the liquid crystal composition comprising the self-aligning agent of general formula O in the present invention has a lower residue concentration (110-123 ppm VS 163-189 ppm), a smaller roughness (11.8-12.4 nm VS 15.4-16.1 nm) and a better alignment effect while maintaining an appropriate clearing point, an appropriate optical anisotropy, an appropriate absolute value of dielectric anisotropy, a larger K value (K₁₁ and K₃₃) and a smaller rotational viscosity.

### Comparative Examples 10-12 and Examples 10-12

0.3 parts by weight of polymerizable compound RM-1-1 and 0.8 parts by weight of D-1, D-2 and D-3 respectively are added into 100 parts by weight of host liquid crystal composition Host-4 to prepare the liquid crystal compositions of Comparative Examples 10-12 respectively, and 0.3 parts by weight of polymerizable compound RM-1-1 and 0.8 parts by weight of AD-1, AD-2 and AD-3 are added into 100 parts by weight of host liquid crystal composition Host-4 to prepare the liquid crystal compositions of Examples 10-12 respectively. The physical property values of each of the obtained liquid crystal compositions showed negligible changes relative to perspective host liquid crystal composition. The obtained liquid crystal compositions are tested for performance by filling each liquid crystal composition into a "non-aligned" test cell (cell gap d is 3.5 µm, with ITO coatings (structured ITO for multi-domain switching mode) on both substrates, no alignment layer, and no passivation layer). The test results for relevant performances of the liquid crystal compositions of Comparative Examples 10-12 and Examples 10-12 are shown in Table 8 below.

**Table 8 Test results for performances of the liquid crystal compositions of Comparative Examples 10-12 and Examples 10-12**

| | Comp. Ex. 10 | Comp. Ex. 11 | Comp. Ex. 12 | Ex. 10 | Ex. 11 | Ex. 12 |
|---|---|---|---|---|---|---|
| Residue conc. | 189 | 223 | 207 | 126 | 144 | 132 |
| Ra | 14.8 | 15.6 | 15.1 | 11.5 | 11.7 | 12 |
| Alignment effect | Fair | Fair | Poor | Good | Good | Good |

It can be seen from the comparison between Example 10 and Comparative Example 10, the comparison between Example 11 and Comparative Example 11 and the comparison between Example 12 and Comparative Example 12 that, the liquid crystal composition comprising the self-aligning agent of general formula O in the present invention has a lower residue concentration (126-144 ppm VS 189-223 ppm), a smaller roughness (11.5-12 nm VS 14.8-15.6 nm) and a better alignment effect while maintaining an appropriate clearing point, an appropriate optical anisotropy, an appropriate absolute value of dielectric anisotropy, a larger K value (K₁₁ and K₃₃) and a smaller rotational viscosity.

### Examples 13-17

The liquid crystal compositions of Examples 13-17 are prepared according to the parts by weight of each compound listed in Table 9. The obtained liquid crystal compositions are tested for performance by filling each liquid crystal composition into a "non-aligned" test cell (cell gap d is 3.5 µm, with ITO coatings (structured ITO for multi-domain switching mode) on both substrates, no alignment layer, and no passivation layer). The test results for relevant performances of the liquid crystal compositions of Examples 13-17 are shown in Table 10 below.

**Table 9 Formulations of the liquid crystal compositions of Examples 13-17**

| | Ex. 13 | Ex. 14 | Ex. 15 | Ex. 16 | Ex. 17 |
|---|---|---|---|---|---|
| Host-5 | 100 | 100 | | | |
| Host-6 | | | 100 | 100 | 100 |
| RM-1-1 | 0.3 | 0.3 | 0.3 | | |
| RM-2-1 | | | | 0.28 | 0.3 |
| RM-20-1 | | | | 0.02 | |
| AD-1 | 0.8 | 0.5 | | | |
| AD-2 | | | 0.8 | 0.8 | 0.5 |
| AD-3 | | 0.3 | | | 0.3 |

**Table 10 Test results for performances of the liquid crystal compositions of Examples 13-17**

| | Ex. 13 | Ex. 14 | Ex. 15 | Ex. 16 | Ex. 17 |
|---|---|---|---|---|---|
| Residue conc. | 108 | 105 | 141 | 138 | 133 |
| Ra | 11.8 | 11.4 | 11.5 | 11.7 | 11.6 |
| Alignment effect | Good | Good | Good | Good | Good |

It can be seen from the performance parameters of Examples 13-17 that, the liquid crystal composition comprising the self-aligning agent of general formula O in the present invention has a lower residue concentration (105-141 ppm), a smaller roughness (11.4-11.8 nm) and a better alignment effect while maintaining an appropriate clearing point, an appropriate optical anisotropy, an appropriate absolute value of dielectric anisotropy, a larger K value (K₁₁ and K₃₃) and a smaller rotational viscosity.

Host liquid crystal compositions Host-7, Host-8, Host-9, Host-10 and Host-11 are prepared according to each compound and weight percentage listed in Table 11, and are tested by filling the same between two substrates of a liquid crystal display device.

**Table 11 Formulations and test results for performance parameters of host liquid crystal compositions**

| Component code | General formula code | Weight percentage | | | | |
|---|---|---|---|---|---|---|
| | | Host-7 | Host-8 | Host-9 | Host-10 | Host-11 |
| 2CPWO2 | N-21 | 7.5 | 3.5 | 11 | | 7.5 |
| 3CPWO2 | N-21 | 9 | 10.3 | | | 9 |
| 3CCWO2 | N-9 | 3 | | | | 3 |
| 3CLWO2 | N-12 | 4 | 7 | 4 | 6.3 | 4 |
| 2OPWO2 | N-19 | 6 | 5.5 | | 6 | 6 |
| 3CC2 | M-1-2 | | | | 3.2 | |
| 3CCV | M-1-5 | 37 | 29 | 25.5 | 29 | 37 |
| 1PP2V1 | M-4 | 5 | | | 4 | 5 |
| VCCP1 | M-11-1 | 5 | | | | 5 |
| 3CCP1 | M-11-1 | | 11 | 12.5 | 11.5 | |
| 3CCP3 | M-11-3 | | | 3.5 | | |
| 3CPP2 | M-13-2 | | 1.2 | 6.5 | | |
| 1PWO2 | N-19 | 9 | 11 | 11 | 10.5 | 9 |
| 2PWO2 | N-19 | | | 10 | | |
| 3PWO2 | N-19 | | 9 | | | |
| 4OB(S)O2 | B-1-1 | | | 4 | | |
| 5OB(S)O2 | B-1-1 | | | 2.5 | | |
| C(5)1OB(S)O4 | B-4 | 2.5 | | | | 2.5 |
| C(5)1OB(S)O2 | B-4 | 2.5 | | | | 2.5 |
| C(5,V)1OB(S)O4 | B-7 | | | | 8 | |
| C(5,V)1OB(S)O2 | B-7 | | | | 4 | |
| 4OB(S)OV(2F) | B-1-4 | | 4 | | | |
| 3CCV1 | M-1-6 | 5 | 8.5 | 9.5 | 8.5 | 5 |
| 3CPWO4 | N-21 | 4.5 | | | | 4.5 |
| 1VCPWO2 | N-53 | | | | 9 | |
| Total | | 100 | 100 | 100 | 100 | 100 |
| Cp | | 75.3 | 75 | 75.2 | 75.3 | 75.2 |
| Δn | | 0.1145 | 0.1145 | 0.1146 | 0.1145 | 0.115 |
| Δε | | -2.88 | -2.83 | -3.2 | -2.97 | -2.83 |
| K₁₁ | | 15.1 | 14.7 | 14.1 | 15.6 | 14.8 |
| K₃₃ | | 16.4 | 15.7 | 16.3 | 16.8 | 16.0 |
| γ₁ | | 68 | 69 | 70 | 75 | 70 |

Host liquid crystal compositions Host-12, Host-13, Host-14 and Host-15 are prepared according to each compound and weight percentage listed in Table 12, and are tested by filling the same between two substrates of a liquid crystal display device.

**Table 12 Formulations and test results for performance parameters of host liquid crystal compositions**

| Component code | General formula code | Weight percentage | | | |
|---|---|---|---|---|---|
| | | Host-12 | Host-13 | Host-14 | Host-15 |
| 2CPWO2 | N-21 | 3.5 | 11 | 11 | 5 |
| 3CPWO2 | N-21 | 10.3 | | | |
| 3CLWO2 | N-12 | 7 | 4 | 4 | 5 |
| 2OPWO2 | N-19 | 5.5 | | | |
| 3PWO2 | N-19 | 9 | | | 9 |
| 2PWO2 | N-19 | | 10 | 10 | |
| 1PWO2 | N-19 | 11 | 11 | 11 | |
| 3PPWO2 | N-23 | | | | 2 |
| 3CPP2 | M-13-2 | 1.2 | 6.5 | 6.5 | 6 |
| 1VCPP2 | M-13-2 | | | | 3 |
| 3CCP1 | M-11-1 | 11 | 12.5 | 12.5 | |
| 3CCP3 | M-11-3 | | 3.5 | 3.5 | |
| 3CC2 | M-1-2 | | | | |
| 3CCV | M-1-5 | 29 | 25.5 | 25.5 | 36.5 |
| 3CCV1 | M-1-6 | 8.5 | 9.5 | 9.5 | 6.5 |
| 1PP2V1 | M-4 | | | | 8 |
| 4OB(S)O2 | B-1-1 | | 4 | | |
| 4OB(O)O2 | B-1-1 | | | 4 | |
| 5OB(O)O2 | B-1-1 | | 2.5 | 2.5 | |
| C(5,V)1OB(S)O4 | B-7 | | | | 5 |
| C(5,V)1OB(S)O2 | B-7 | | | | 4 |
| C(5,V)1OB(O)O4 | B-7 | | | | |
| C(5,V)1OB(O)O2 | B-7 | | | | |
| 4OB(O)OV(2F) | B-1-4 | 4 | | | 3 |
| 1VCPWO2 | N-53 | | | | 7 |
| Total | | 100 | 100 | 100 | 100 |
| Cp | | 74.9 | 75.1 | 75.1 | 73.7 |
| Δn | | 0.1147 | 0.1148 | 0.1151 | 0.121 |
| Δε | | -2.79 | -3.13 | -3.13 | -2.3 |
| K₁₁ | | 14.5 | 14 | 13.9 | 14.7 |
| K₃₃ | | 15.4 | 16.2 | 16.1 | 15.2 |
| γ₁ | | 71 | 71 | 72 | 67 |

### Comparative Examples 13-15 and Examples 18-22

The liquid crystal compositions of Comparative Examples 13-15 and Examples 18-22 are prepared according to the parts by weight of each compound listed in Table 13. The obtained liquid crystal compositions are tested for performance by filling each liquid crystal composition into a "non-aligned" test cell (cell gap d is 3.5 µm, with ITO coatings (structured ITO for multi-domain switching mode) on both substrates, no alignment layer, and no passivation layer). The test results for relevant performances of the liquid crystal compositions of Comparative Examples 13-15 and Examples 18-22 are shown in Table 14 below.

**Table 13 Formulations of the liquid crystal compositions of Comparative Examples 13-15 and Examples 18-22**

| | Comp. Ex. 13 | Comp. Ex. 14 | Comp. Ex. 15 | Ex. 18 | Ex. 19 | Ex. 20 | Ex. 21 | Ex. 22 |
|---|---|---|---|---|---|---|---|---|
| Host-7 | 100 | 100 | 100 | 100 | 100 | 100 | 100 | 100 |
| RM-1-1 | 0.4 | 0.4 | 0.4 | 0.4 | 0.4 | 0.4 | 0.4 | 0.4 |
| D-1 | 0.7 | | | | | | | |
| D-2' | | 0.7 | | | | | | |
| D-3 | | | 0.7 | | | | | |
| AD-2 | | | | 0.7 | | | | |
| AD-1 | | | | | 0.7 | | | |
| AD-3' | | | | | | 0.7 | | |
| AD-4 | | | | | | | 0.7 | |
| AD-5 | | | | | | | | 0.7 |

**Table 14 Test results for performances of the liquid crystal compositions of Comparative Examples 13-15 and Examples 18-22**

| | Comp. Ex. 13 | Comp. Ex. 14 | Comp. Ex. 15 | Ex. 18 | Ex. 19 | Ex. 20 | Ex. 21 | Ex. 22 |
|---|---|---|---|---|---|---|---|---|
| Residue conc. | 235 | 203 | 252 | 185 | 202 | 156 | 179 | 158 |
| Ra | 13.8 | 14.7 | 14.4 | 10.9 | 11.1 | 11.4 | 11.5 | 11.9 |
| Alignment effect | Fair | Fair | Poor | Good | Good | Good | Good | Good |

It can be seen from the comparison between Examples 18-22 and Comparative Examples 13-15 that, through structural optimization of the self-aligning agent, the liquid crystal composition of the present invention has a smaller polymer residue (156-202 ppm VS 203-252 ppm), a smaller roughness (10.9-11.9 nm VS 13.8-14.7 nm) and a better alignment effect while maintaining an appropriate clearing point, an appropriate optical anisotropy, an appropriate absolute value of dielectric anisotropy, a larger K value (K₁₁ and K₃₃) and a smaller rotational viscosity.

### Comparative Examples 16-18 and Examples 23-27

The liquid crystal compositions of Comparative Examples 16-18 and Examples 23-27 are prepared according to the parts by weight of each compound listed in Table 15. The obtained liquid crystal compositions are tested for performance by filling each liquid crystal composition into a "non-aligned" test cell (cell gap d is 3.5 µm, with ITO coatings (structured ITO for multi-domain switching mode) on both substrates, no alignment layer, and no passivation layer). The test results for relevant performances of the liquid crystal compositions of Comparative Examples 16-18 and Examples 23-27 are shown in Table 16 below.

**Table 15 Formulations of the liquid crystal compositions of Comparative Examples 16-18 and Examples 23-27**

| | Comp. Ex. 16 | Comp. Ex. 17 | Comp. Ex. 18 | Ex. 23 | Ex. 24 | Ex. 25 | Ex. 26 | Ex. 27 |
|---|---|---|---|---|---|---|---|---|
| Host-8 | 100 | 100 | 100 | 100 | 100 | 100 | 100 | 100 |
| RM-1-1 | 0.4 | 0.4 | 0.4 | 0.4 | 0.4 | 0.4 | 0.4 | 0.4 |
| D-1 | 0.8 | | | | | | | |
| D-2' | | 0.8 | | | | | | |
| D-3 | | | 0.8 | | | | | |
| AD-2 | | | | 0.8 | | | | |
| AD-1 | | | | | 0.8 | | | |
| AD-3' | | | | | | 0.8 | | |
| AD-4 | | | | | | | 0.8 | |
| AD-5 | | | | | | | | 0.8 |

**Table 16 Test results for performances of the liquid crystal compositions of Comparative Examples 16-18 and Examples 23-27**

| | Comp. Ex. 16 | Comp. Ex. 17 | Comp. Ex. 18 | Ex. 23 | Ex. 24 | Ex. 25 | Ex. 26 | Ex. 27 |
|---|---|---|---|---|---|---|---|---|
| Residue conc. | 213 | 185 | 238 | 162 | 183 | 143 | 158 | 146 |
| Ra | 13.9 | 14.8 | 14.5 | 10.7 | 10.8 | 11.1 | 11.3 | 11.6 |
| Alignment effect | Fair | Fair | Poor | Good | Good | Good | Good | Good |

It can be seen from the comparison between Examples 23-27 and Comparative Examples 16-18 that, through structural optimization of the self-aligning agent, the liquid crystal composition of the present invention has a smaller polymer residue (143-183 ppm VS 185-238 ppm), a smaller roughness (10.7-11.6 nm VS 13.9-14.8 nm) and a better alignment effect while maintaining an appropriate clearing point, an appropriate optical anisotropy, an appropriate absolute value of dielectric anisotropy, a larger K value (K₁₁ and K₃₃) and a smaller rotational viscosity.

### Comparative Examples 19-21 and Examples 28-32

The liquid crystal compositions of Comparative Examples 19-21 and Examples 28-32 are prepared according to the parts by weight of each compound listed in Table 17. The obtained liquid crystal compositions are tested for performance by filling each liquid crystal composition into a "non-aligned" test cell (cell gap d is 3.5 µm, with ITO coatings (structured ITO for multi-domain switching mode) on both substrates, no alignment layer, and no passivation layer). The test results for relevant performances of the liquid crystal compositions of Comparative Examples 19-21 and Examples 28-32 are shown in Table 18 below.

**Table 17 Formulations of the liquid crystal compositions of Comparative Examples 19-21 and Examples 28-32**

| | Comp. Ex. 19 | Comp. Ex. 20 | Comp. Ex. 21 | Ex. 28 | Ex. 29 | Ex. 30 | Ex. 31 | Ex. 32 |
|---|---|---|---|---|---|---|---|---|
| Host-9 | 100 | 100 | 100 | 100 | 100 | 100 | 100 | 100 |
| RM-2-1 | 0.4 | 0.4 | 0.4 | 0.4 | 0.4 | 0.4 | 0.4 | 0.4 |
| D-1 | 1 | | | | | | | |
| D-2' | | 1 | | | | | | |
| D-3 | | | 1 | | | | | |
| AD-2 | | | | 1 | | | | |
| AD-1 | | | | | 1 | | | |
| AD-3' | | | | | | 1 | | |
| AD-4 | | | | | | | 1 | |
| AD-5 | | | | | | | | 1 |

**Table 18 Test results for performances of the liquid crystal compositions of Comparative Examples 19-21 and Examples 28-32**

| | Comp. Ex. 19 | Comp. Ex. 20 | Comp. Ex. 21 | Ex. 28 | Ex. 29 | Ex. 30 | Ex. 31 | Ex. 32 |
|---|---|---|---|---|---|---|---|---|
| Residue conc. | 198 | 175 | 217 | 161 | 172 | 135 | 155 | 142 |
| Ra | 14.0 | 14.9 | 14.5 | 10.7 | 10.9 | 11.2 | 11.3 | 11.7 |
| Alignment effect | Fair | Fair | Poor | Good | Good | Good | Good | Good |

It can be seen from the comparison between Examples 28-32 and Comparative Examples 19-21 that, through structural optimization of the self-aligning agent, the liquid crystal composition of the present invention has a smaller polymer residue (135-172 ppm VS 175-219 ppm), a smaller roughness (10.7-11.7 nm VS 14.0-14.9 nm) and a better alignment effect while maintaining an appropriate clearing point, an appropriate optical anisotropy, an appropriate absolute value of dielectric anisotropy, a larger K value (K₁₁ and K₃₃) and a smaller rotational viscosity.

### Comparative Examples 22-24 and Examples 33-37

The liquid crystal compositions of Comparative Examples 22-24 and Examples 33-37 are prepared according to the parts by weight of each compound listed in Table 19. The obtained liquid crystal compositions are tested for performance by filling each liquid crystal composition into a "non-aligned" test cell (cell gap d is 3.5 µm, with ITO coatings (structured ITO for multi-domain switching mode) on both substrates, no alignment layer, and no passivation layer). The test results for relevant performances of the liquid crystal compositions of Comparative Examples 22-24 and Examples 33-37 are shown in Table 20 below.

**Table 19 Formulations of the liquid crystal compositions of Comparative Examples 22-24 and Examples 33-37**

| | Comp. Ex. 22 | Comp. Ex. 23 | Comp. Ex. 24 | Ex. 33 | Ex. 34 | Ex. 35 | Ex. 36 | Ex. 37 |
|---|---|---|---|---|---|---|---|---|
| Host-10 | 100 | 100 | 100 | 100 | 100 | 100 | 100 | 100 |
| RM-1-1 | 0.4 | 0.4 | 0.4 | 0.4 | 0.4 | 0.4 | 0.4 | 0.4 |
| D-1 | 0.9 | | | | | | | |
| D-2 | | 0.9 | | | | | | |
| D-3 | | | 0.9 | | | | | |
| AD-2 | | | | 0.9 | | | | |
| AD-1 | | | | | 0.9 | | | |
| AD-3' | | | | | | 0.9 | | |
| AD-4 | | | | | | | 0.9 | |
| AD-5 | | | | | | | | 0.9 |

**Table 20 Test results for performances of the liquid crystal compositions of Comparative Examples 22-24 and Examples 33-37**

| | Comp. Ex. 22 | Comp. Ex. 23 | Comp. Ex. 24 | Ex. 33 | Ex. 34 | Ex. 35 | Ex. 36 | Ex. 37 |
|---|---|---|---|---|---|---|---|---|
| Residue conc. | 192 | 174 | 209 | 155 | 166 | 128 | 149 | 135 |
| Ra | 14.2 | 15.1 | 14.6 | 10.6 | 10.7 | 11.0 | 11.2 | 11.5 |
| Alignment effect | Fair | Fair | Poor | Good | Good | Good | Good | Good |

It can be seen from the comparison between Examples 33-37 and Comparative Examples 22-24 that, through structural optimization of the self-aligning agent, the liquid crystal composition of the present invention has a smaller polymer residue (128-166 ppm VS 174-209 ppm), a smaller roughness (10.6-11.5 nm VS 14.2-15.1 nm) and a better alignment effect while maintaining an appropriate clearing point, an appropriate optical anisotropy, an appropriate absolute value of dielectric anisotropy, a larger K value (K₁₁ and K₃₃) and a smaller rotational viscosity.

### Examples 38-47

The liquid crystal compositions of Examples 38-47 are prepared according to the parts by weight of each compound listed in Table 21. The obtained liquid crystal compositions are tested for performance by filling each liquid crystal composition into a "non-aligned" test cell (cell gap d is 3.5 µm, with ITO coatings (structured ITO for multi-domain switching mode) on both substrates, no alignment layer, and no passivation layer). The test results for relevant performances of the liquid crystal compositions of Examples 38-47 are shown in Table 22 below.

**Table 21 Formulations of the liquid crystal compositions of Examples 38-47**

| | Ex. 38 | Ex. 39 | Ex. 40 | Ex. 41 | Ex. 42 | Ex. 43 | Ex. 44 | Ex. 45 | Ex. 46 | Ex. 47 |
|---|---|---|---|---|---|---|---|---|---|---|
| Host-11 | 100 | 100 | 100 | 100 | 100 | | | | | |
| Host-12 | | | | | | 100 | 100 | 100 | 100 | 100 |
| RM-1-1 | 0.4 | 0.4 | 0.4 | 0.4 | 0.4 | 0.8 | 0.8 | 0.8 | 0.8 | 0.8 |
| AD-2 | 0.7 | | | | | 0.8 | | | | |
| AD-1 | | 0.7 | | | | | 0.8 | | | |
| AD-3' | | | 0.7 | | | | | 0.8 | | |
| AD-4 | | | | 0.7 | | | | | 0.8 | |
| AD-5 | | | | | 0.7 | | | | | 0.8 |

**Table 22 Test results for performances of the liquid crystal compositions of Examples 38-47**

| | Ex. 38 | Ex. 39 | Ex. 40 | Ex. 41 | Ex. 42 | Ex. 43 | Ex. 44 | Ex. 45 | Ex. 46 | Ex. 47 |
|---|---|---|---|---|---|---|---|---|---|---|
| Residue conc. | 193 | 213 | 164 | 187 | 165 | 169 | 191 | 150 | 165 | 154 |
| Ra | 11.6 | 11.9 | 12.2 | 12.4 | 12.7 | 11.6 | 11.7 | 11.9 | 12.2 | 12.5 |
| Alignment effect | Good | Good | Good | Good | Good | Good | Good | Good | Good | Good |

It can be seen from the performance parameters of Examples 38-47 that, the liquid crystal composition comprising the self-aligning agent of general formula O in the present invention has a lower residue concentration (150-213 ppm), a smaller roughness (11.6-12.7 nm) and a better alignment effect while maintaining an appropriate clearing point, an appropriate optical anisotropy, an appropriate absolute value of dielectric anisotropy, a larger K value (K₁₁ and K₃₃) and a smaller rotational viscosity.

### Examples 48-57

The liquid crystal compositions of Examples 48-57 are prepared according to the parts by weight of each compound listed in Table 23. The obtained liquid crystal compositions are tested for performance by filling each liquid crystal composition into a "non-aligned" test cell (cell gap d is 3.5 µm, with ITO coatings (structured ITO for multi-domain switching mode) on both substrates, no alignment layer, and no passivation layer). The test results for relevant performances of the liquid crystal compositions of Examples 48-57 are shown in Table 24 below.

**Table 23 Formulations of the liquid crystal compositions of Examples 48-57**

| | Ex. 48 | Ex. 49 | Ex. 50 | Ex. 51 | Ex. 52 | Ex. 53 | Ex. 54 | Ex. 55 | Ex. 56 | Ex. 57 |
|---|---|---|---|---|---|---|---|---|---|---|
| Host-13 | 100 | 100 | 100 | 100 | 100 | | | | | |
| Host-14 | | | | | | 100 | 100 | 100 | 100 | 100 |
| RM-2-1 | 0.4 | 0.4 | 0.4 | 0.4 | 0.4 | 0.4 | 0.4 | 0.4 | 0.4 | 0.4 |
| AD-2 | 1 | | | | | 1 | | | | |
| AD-1 | | 1 | | | | | 1 | | | |
| AD-3' | | | 1 | | | | | 1 | | |
| AD-4 | | | | 1 | | | | | 1 | |
| AD-5 | | | | | 1 | | | | | 1 |

**Table 24 Test results for performances of the liquid crystal compositions of Example 48-57**

| | Ex. 48 | Ex. 49 | Ex. 50 | Ex. 51 | Ex. 52 | Ex. 53 | Ex. 54 | Ex. 55 | Ex. 56 | Ex. 57 |
|---|---|---|---|---|---|---|---|---|---|---|
| Residue conc. | 165 | 178 | 140 | 160 | 146 | 172 | 184 | 146 | 167 | 152 |
| Ra | 11.1 | 11.3 | 11.5 | 11.7 | 12.1 | 11.6 | 11.7 | 12.0 | 12.2 | 12.6 |
| Alignment effect | Good | Good | Good | Good | Good | Good | Good | Good | Good | Good |

It can be seen from the performance parameters of Examples 48-57 that, the liquid crystal composition comprising the self-aligning agent of general formula O in the present invention has a lower residue concentration (140-184 ppm), a smaller roughness (11.1-12.6 nm) and a better alignment effect while maintaining an appropriate clearing point, an appropriate optical anisotropy, an appropriate absolute value of dielectric anisotropy, a larger K value (K₁₁ and K₃₃) and a smaller rotational viscosity.

### Examples 58-62

The liquid crystal compositions of Examples 58-62 are prepared according to the parts by weight of each compound listed in Table 25. The obtained liquid crystal compositions are tested for performance by filling each liquid crystal composition into a "non-aligned" test cell (cell gap d is 3.5 µm, with ITO coatings (structured ITO for multi-domain switching mode) on both substrates, no alignment layer, and no passivation layer). The test results for relevant performances of the liquid crystal compositions of Examples 58-62 are shown in Table 26 below.

**Table 25 Formulations of the liquid crystal compositions of Examples 58-62**

| | Ex. 58 | Ex. 59 | Ex. 60 | Ex. 61 | Ex. 62 |
|---|---|---|---|---|---|
| Host-15 | 100 | 100 | 100 | 100 | 100 |
| | | | | | |
| RM-1-1 | 0.4 | 0.4 | 0.4 | 0.4 | 0.4 |
| AD-2 | 0.8 | | | | |
| AD-1 | | 0.8 | | | |
| AD-3' | | | 0.8 | | |
| AD-4 | | | | 0.8 | |
| AD-5 | | | | | 0.8 |

**Table 26 Test results for performances of the liquid crystal compositions of Examples 58-62**

| | Ex. 58 | Ex. 59 | Ex. 60 | Ex. 61 | Ex. 62 |
|---|---|---|---|---|---|
| Residue conc. | 144 | 156 | 119 | 139 | 126 |
| Ra | 10.5 | 10.7 | 11.0 | 11.2 | 11.5 |
| Alignment effect | Good | Good | Good | Good | Good |

It can be seen from the performance parameters of Examples 58-62 that, the liquid crystal composition comprising the self-aligning agent of general formula O in the present invention has a lower residue concentration (119-175 ppm), a smaller roughness (10.5-12.5 nm) and a better alignment effect while maintaining an appropriate clearing point, an appropriate optical anisotropy, an appropriate absolute value of dielectric anisotropy, a larger K value (K₁₁ and K₃₃) and a smaller rotational viscosity.

Host liquid crystal compositions Host-16, Host-17, Host-18, Host-19, Host-20 and Host-21 are prepared according to each compound and weight percentage listed in Table 27, and are tested by filling the same between two substrates of a liquid crystal display device.

**Table 27 Formulations and test results for performance parameters of host liquid crystal compositions**

| Component code | General formula code | Weight percentage | | | | | |
|---|---|---|---|---|---|---|---|
| | | Host-16 | Host-17 | Host-18 | Host-19 | Host-20 | Host-21 |
| 3PWO2 | N-19 | | 15 | 3 | 9 | 9 | 8 |
| V2PWO4 | N-55 | | | 6 | | | |
| 2OPWO2 | N-19 | | | 6 | 6 | 6 | |
| 3CPWO2 | N-21 | 5 | 7.5 | 2.5 | 2.5 | | 8 |
| 1VCPWO2 | N-53 | | | | | 2.5 | |
| 3C1OWO2 | N-7 | 11 | | | | | |
| 2CC1OWO2 | N-15 | 8.5 | | | | | |
| 3CC1OWO2 | N-15 | 11 | | | | | |
| 3CWO2 | N-2 | | 13 | 13 | 13 | | 12 |
| 3CWO4 | N-2 | | | 2 | 2 | 2 | |
| 5CWO2 | N-2 | | 5 | | | | 5 |
| VCWO2 | N-49 | | | | | 13 | |
| 2CCWO2 | N-9 | | 5 | 6.5 | | 6.5 | |
| 3CCWO2 | N-9 | 3 | 12 | 13.5 | 13.5 | 13.5 | 6 |
| VCCWO2 | N-51 | | | | | | 6 |
| 2CLWO2 | N-12 | | | | 6.5 | | |
| 2C1OWO2 | N-7 | 5 | | | | | |
| 2PWP3 | N-24 | | | | | | 5 |
| 2PWP2V1 | N-54 | | | | | | 5 |
| 3CPP2 | M-13-2 | 13 | 9 | 13 | 8 | 13 | 10 |
| 3CPP2V1 | M-13-9 | | | | 5 | | |
| 3CPP1 | M-13-1 | | 7 | | | | 10 |
| 3CPPC3 | M-24 | 1 | | | | | |
| 3CC2 | M-1-2 | 20 | 17 | 14 | 14 | 14 | 15 |
| 5CC2 | M-1-9 | | | 4 | 4 | 4 | |
| 3CC4 | M-1-4 | | | 6 | 6 | 6 | |
| 3CCV1 | M-1-6 | | 9.5 | 10.5 | 10.5 | 10.5 | 10 |
| 2CPP2 | M-13-2 | 8 | | | | | |
| 5PP1 | M-4 | 14.5 | | | | | |
| Total | | 100 | 100 | 100 | 100 | 100 | 100 |
| Cp | | 75 | 75.7 | 77.2 | 76.8 | 75.6 | 76.6 |
| Δn | | 0.1109 | 0.109 | 0.101 | 0.102 | 0.101 | 0.112 |
| Δε | | -2.71 | -3.3 | -3.0 | -3.1 | -3.0 | -2.9 |
| K₁₁ | | 14.5 | 14.6 | 15.1 | 12.1 | 15 | 15.1 |
| K₃₃ | | 15.7 | 15.6 | 14.7 | 15.3 | 14.8 | 14.7 |
| γ₁ | | 110 | 106 | 109 | 103 | 108 | 105 |

### Comparative Examples 25-27 and Examples 63-66

The liquid crystal compositions of Comparative Examples 25-27 and Examples 63-66 are prepared according to the parts by weight of each compound listed in Table 28. The obtained liquid crystal compositions are tested for performance by filling each liquid crystal composition into a "non-aligned" test cell (cell gap d is 3.5 µm, with ITO coatings (structured ITO for multi-domain switching mode) on both substrates, no alignment layer, and no passivation layer). The test results for relevant performances of the liquid crystal compositions of Comparative Examples 25-27 and Examples 63-66 are shown in Table 29 below.

**Table 28 Formulations of the liquid crystal compositions of Comparative Examples 25-27 and Examples 63-66**

| | Comp. Ex. 25 | Comp. Ex. 26 | Comp. Ex. 27 | Ex. 63 | Ex. 64 | Ex. 65 | Ex. 66 |
|---|---|---|---|---|---|---|---|
| Host-16 | 100 | 100 | 100 | 100 | 100 | 100 | 100 |
| RM-1-1 | 0.3 | 0.3 | 0.3 | 0.3 | | 0.3 | |
| RM-2-1 | | | | | 0.3 | | 0.3 |
| D-1 | 1 | | | | | | |
| D-4 | | 1 | | | | | |
| D-3' | | | 1 | | | | |
| AD-2 | | | | 1 | | | |
| AD-1 | | | | | 1 | | |
| AD-3 | | | | | | 1 | |
| AD-4 | | | | | | | 1 |

**Table 29 Test results for performances of the liquid crystal compositions of Comparative Examples 25-27 and Examples 63-66**

| | Comp. Ex. 25 | Comp. Ex. 26 | Comp. Ex. 27 | Ex. 68 | Ex. 69 | Ex. 70 | Ex. 71 |
|---|---|---|---|---|---|---|---|
| Residue conc. | 163 | 172 | 166 | 128 | 121 | 116 | 120 |
| Ra | 15.2 | 15.6 | 15.7 | 11.9 | 11.7 | 11.8 | 12.1 |
| t_{-10°C} | 8D NG | 7D NG | 7D NG | 10D OK | 10D OK | 10D OK | 10D OK |

| Alignment effect | Poor | Fair | Poor | Good | Good | Good | Good |
|---|---|---|---|---|---|---|---|
| PTA₍ᵢₙᵢₜᵢₐₗ₎ | 88.12 | 88.08 | 88.11 | 88.12 | 88.09 | 88.12 | 88.12 |
| PTA₍₁₆₅ₕ₎ | 87.77 | 87.73 | 87.75 | 87.86 | 87.85 | 87.87 | 87.87 |
| ΔPTA | 0.35 | 0.35 | 0.36 | 0.26 | 0.24 | 0.25 | 0.25 |

It can be seen from the comparison between Examples 63-66 and Comparative Examples 25-27 that, through structural optimization of the self-aligning agent, the liquid crystal composition of the present invention has a smaller polymer residue (116-128 ppm VS 163-172 ppm), a smaller roughness (11.7-12.1 nm VS 15.2-15.7 nm), a better low-temperature storage stability (10D OK VS 7-8D NG), a better alignment effect, and a better pre-tilt angle stability (0.24-0.26 VS 0.35-0.36) while maintaining an appropriate clearing point, an appropriate optical anisotropy, an appropriate absolute value of dielectric anisotropy, a larger K value (K₁₁ and K₃₃) and a smaller rotational viscosity.

### Comparative Examples 28-30 and Examples 67-70

The liquid crystal compositions of Comparative Examples 28-30 and Examples 67-70 are prepared according to the parts by weight of each compound listed in Table 30. The obtained liquid crystal compositions are tested for performance by filling each liquid crystal composition into a "non-aligned" test cell (cell gap d is 3.5 µm, with ITO coatings (structured ITO for multi-domain switching mode) on both substrates, no alignment layer, and no passivation layer). The test results for relevant performances of the liquid crystal compositions of Comparative Examples 28-30 and Examples 67-70 are shown in Table 31 below.

**Table 30 Formulations of the liquid crystal compositions of Comparative Examples 28-30 and Examples 67-70**

| | Comp. Ex. 28 | Comp. Ex. 29 | Comp. Ex. 30 | Ex. 67 | Ex. 68 | Ex. 69 | Ex. 70 |
|---|---|---|---|---|---|---|---|
| Host-17 | 100 | 100 | 100 | 100 | 100 | 100 | 100 |
| RM-2-1 | 0.3 | 0.3 | 0.3 | 0.3 | 0.3 | 0.3 | 0.3 |
| D-1 | 0.9 | | | | | | |
| D-4 | | 0.9 | | | | | |
| D-3' | | | 0.9 | | | | |
| AD-2 | | | | 0.9 | | | |
| AD-1 | | | | | 0.9 | | |
| AD-3 | | | | | | 0.9 | |
| AD-4 | | | | | | | 0.9 |

**Table 31 Test results for performances of the liquid crystal compositions of Comparative Examples 28-30 and Examples 67-70**

| | Comp. Ex. 28 | Comp. Ex. 29 | Comp. Ex. 30 | Ex. 67 | Ex. 68 | Ex. 69 | Ex. 70 |
|---|---|---|---|---|---|---|---|
| Residue conc. | 168 | 189 | 172 | 120 | 123 | 117 | 119 |
| Ra | 15.5 | 16.1 | 15.4 | 11.8 | 12 | 12.4 | 12.1 |
| t_{-10°C} | 8D NG | 8D NG | 7D NG | 10D OK | 10D OK | 10D OK | 10D OK |
| Alignmen t effect | Fair | Fair | Poor | Good | Good | Good | Good |
| PTA₍ᵢₙᵢₜᵢₐₗ₎ | 88.1 | 88.12 | 88.09 | 88.14 | 88.08 | 88.14 | 88.14 |
| PTA₍₁₆₅ₕ₎ | 87.74 | 87.77 | 87.74 | 87.89 | 87.81 | 87.87 | 87.88 |
| ΔPTA | 0.36 | 0.35 | 0.35 | 0.25 | 0.27 | 0.27 | 0.26 |

It can be seen from the comparison between Examples 67-70 and Comparative Examples 28-30 that, through structural optimization of the self-aligning agent, the liquid crystal composition of the present invention has a smaller polymer residue (117-123 ppm VS 168-189 ppm), a smaller roughness (11.8-12.4 nm VS 15.4-16.1 nm), a better low-temperature storage stability (10D OK VS 7-8D NG), a better alignment effect, and a better pre-tilt angle stability (0.25-0.27 VS 0.35-0.36) while maintaining an appropriate clearing point, an appropriate optical anisotropy, an appropriate absolute value of dielectric anisotropy, a larger K value (K₁₁ and K₃₃) and a smaller rotational viscosity.

### Comparative Examples 31-33 and Examples 71-74

The liquid crystal compositions of Comparative Examples 31-33 and Examples 71-74 are prepared according to the parts by weight of each compound listed in Table 32. The obtained liquid crystal compositions are tested for performance by filling each liquid crystal composition into a "non-aligned" test cell (cell gap d is 3.5 µm, with ITO coatings (structured ITO for multi-domain switching mode) on both substrates, no alignment layer, and no passivation layer). The test results for relevant performances of the liquid crystal compositions of Comparative Examples 31-33 and Examples 71-74 are shown in Table 33 below.

**Table 32 Formulations of the liquid crystal compositions of Comparative Examples 31-33 and Examples 71-74**

| | Comp. Ex. 31 | Comp. Ex. 32 | Comp. Ex. 33 | Ex. 71 | Ex. 72 | Ex. 73 | Ex. 74 |
|---|---|---|---|---|---|---|---|
| Host-18 | 100 | 100 | 100 | 100 | 100 | 100 | 100 |
| RM-1-1 | 0.3 | 0.3 | 0.3 | 0.3 | 0.3 | 0.3 | 0.3 |
| D-1 | 1 | | | | | | |
| D-4 | | 1 | | | | | |
| D-3' | | | 1 | | | | |
| AD-2 | | | | 1 | | | |
| AD-1 | | | | | 1 | | |
| AD-3 | | | | | | 1 | |
| AD-4 | | | | | | | 1 |

**Table 33 Test results for performances of the liquid crystal compositions of Comparative Examples 31-33 and Examples 71-74**

| | Comp. Ex. 31 | Comp. Ex. 32 | Comp. Ex. 33 | Ex. 71 | Ex. 72 | Ex. 73 | Ex. 74 |
|---|---|---|---|---|---|---|---|
| Residue conc. | 158 | 157 | 159 | 112 | 105 | 108 | 111 |
| Ra | 14.3 | 14.9 | 14.5 | 11.7 | 11.8 | 11.2 | 11.3 |
| t_{-10°C} | 8D NG | 7D NG | 8D NG | 10D OK | 10D OK | 10D OK | 10D OK |
| Alignment effect | Fair | Fair | Poor | Good | Good | Good | Good |
| PTA₍ᵢₙᵢₜᵢₐₗ₎ | 88.14 | 88.11 | 88.09 | 88.15 | 88.09 | 88.15 | 88.13 |
| PTA₍₁₆₅ₕ₎ | 87.81 | 87.78 | 87.86 | 87.92 | 87.85 | 87.92 | 87.89 |
| ΔPTA | 0.33 | 0.33 | 0.33 | 0.23 | 0.24 | 0.23 | 0.24 |

It can be seen from the comparison between Examples 71-74 and Comparative Examples 31-33 that, through structural optimization of the self-aligning agent, the liquid crystal composition of the present invention has a smaller polymer residue (105-112 ppm VS 157-159 ppm), a smaller roughness (11.2-11.8 nm VS 14.3-14.9 nm), a better low-temperature storage stability (10D OK VS 7-8D NG), a better alignment effect, and a better pre-tilt angle stability (0.23-0.24 VS 0.33) while maintaining an appropriate clearing point, an appropriate optical anisotropy, an appropriate absolute value of dielectric anisotropy, a larger K value (K₁₁ and K₃₃) and a smaller rotational viscosity.

### Comparative Examples 34-36 and Examples 75-78

The liquid crystal compositions of Comparative Examples 34-36 and Examples 75-78 are prepared according to the parts by weight of each compound listed in Table 34. The obtained liquid crystal compositions are tested for performance by filling each liquid crystal composition into a "non-aligned" test cell (cell gap d is 3.5 µm, with ITO coatings (structured ITO for multi-domain switching mode) on both substrates, no alignment layer, and no passivation layer). The test results for relevant performances of the liquid crystal compositions of Comparative Examples 34-36 and Examples 75-78 are shown in Table 35 below.

**Table 34 Formulations of the liquid crystal compositions of Comparative Examples 34-36 and Examples 75-78**

| | Comp. Ex. 34 | Comp. Ex. 35 | Comp. Ex. 36 | Ex. 75 | Ex. 76 | Ex. 77 | Ex. 78 |
|---|---|---|---|---|---|---|---|
| Host-19 | 100 | 100 | 100 | 100 | 100 | 100 | 100 |
| RM-1-1 | 0.3 | 0.3 | 0.3 | 0.3 | 0.3 | 0.3 | 0.3 |
| D-1 | 0.8 | | | | | | |
| D-4 | | 0.8 | | | | | |
| D-3' | | | 0.8 | | | | |
| AD-2 | | | | 0.8 | | | |
| AD-1 | | | | | 0.8 | | |
| AD-3 | | | | | | 0.8 | |
| AD-4 | | | | | | | 0.8 |

**Table 35 Test results for performances of the liquid crystal compositions of Comparative Examples 34-36 and Examples 75-78**

| | Comp. Ex. 34 | Comp. Ex. 35 | Comp. Ex. 36 | Ex. 75 | Ex. 76 | Ex. 77 | Ex. 78 |
|---|---|---|---|---|---|---|---|
| Residue conc. | 169 | 173 | 167 | 115 | 128 | 120 | 122 |
| Ra | 15.8 | 15.6 | 15.1 | 12.5 | 12.4 | 12.4 | 12.2 |
| t_{-10°C} | 8D NG | 6D NG | 7D NG | 10D OK | 10D OK | 10D OK | 10D OK |
| Alignment effect | Fair | Fair | Poor | Good | Good | Good | Good |
| PTA₍ᵢₙᵢₜᵢₐₗ₎ | 88.12 | 88.11 | 88.09 | 88.11 | 88.1 | 88.14 | 88.11 |
| PTA₍₁₆₅ₕ₎ | 87.76 | 87.75 | 87.74 | 87.85 | 87.84 | 87.9 | 87.86 |
| ΔPTA | 0.36 | 0.36 | 0.35 | 0.26 | 0.26 | 0.24 | 0.25 |

It can be seen from the comparison between Examples 75-78 and Comparative Examples 34-36 that, through structural optimization of the self-aligning agent, the liquid crystal composition of the present invention has a smaller polymer residue (115-128 ppm VS 167-173 ppm), a smaller roughness (12.2-12.5 nm VS 15.1-15.8 nm), a better low-temperature storage stability (10D OK VS 6-8D NG), a better alignment effect, and a better pre-tilt angle stability (0.24-0.26 VS 0.35-0.36) while maintaining an appropriate clearing point, an appropriate optical anisotropy, an appropriate absolute value of dielectric anisotropy, a larger K value (K₁₁ and K₃₃) and a smaller rotational viscosity.

### Comparative Examples 37-39 and Examples 79-82

The liquid crystal compositions of Comparative Examples 37-39 and Examples 79-82 are prepared according to the parts by weight of each compound listed in Table 36. The obtained liquid crystal compositions are tested for performance by filling each liquid crystal composition into a "non-aligned" test cell (cell gap d is 3.5 µm, with ITO coatings (structured ITO for multi-domain switching mode) on both substrates, no alignment layer, and no passivation layer). The test results for relevant performances of the liquid crystal compositions of Comparative Examples 37-39 and Examples 79-82 are shown in Table 37 below.

**Table 36 Formulations of the liquid crystal compositions of Comparative Examples 37-39 and Examples 79-82**

| | Comp. Ex. 37 | Comp. Ex. 38 | Comp. Ex. 39 | Ex. 79 | Ex. 80 | Ex. 81 | Ex. 82 |
|---|---|---|---|---|---|---|---|
| Host-20 | 100 | 100 | 100 | 100 | 100 | 100 | 100 |
| RM-2-1 | 0.3 | 0.3 | 0.3 | 0.3 | 0.3 | 0.3 | 0.3 |
| D-1 | 1 | | | | | | |
| D-4 | | 1 | | | | | |
| D-3 | | | 1 | | | | |
| AD-2 | | | | 1 | | | |
| AD-1 | | | | | 1 | | |
| AD-3 | | | | | | 1 | |
| AD-4 | | | | | | | 1 |

**Table 37 Test results for performances of the liquid crystal compositions of Comparative Examples 37-39 and Examples 79-82**

| | Comp. Ex. 37 | Comp. Ex. 38 | Comp. Ex. 39 | Ex. 79 | Ex. 80 | Ex. 81 | Ex. 82 |
|---|---|---|---|---|---|---|---|
| Residue conc. | 145 | 139 | 140 | 94 | 96 | 90 | 89 |
| Ra | 14.1 | 14 | 13.8 | 9.8 | 9.5 | 9.4 | 9.4 |
| t_{-10°C} | 6D NG | 8D NG | 8D NG | 10D OK | 10D OK | 10D OK | 10D OK |
| Alignment effect | Fair | Poor | Poor | Good | Good | Good | Good |
| PTA₍ᵢₙᵢₜᵢₐₗ₎ | 88.11 | 88.09 | 88.1 | 88.14 | 88.11 | 88.14 | 88.13 |
| PTA₍₁₆₅ₕ₎ | 87.79 | 87.78 | 87.78 | 87.93 | 87.89 | 87.93 | 87.92 |
| ΔPTA | 0.32 | 0.31 | 0.32 | 0.21 | 0.22 | 0.21 | 0.21 |

It can be seen from the comparison between Examples 79-82 and Comparative Examples 37-39 that, through structural optimization of the self-aligning agent, the liquid crystal composition of the present invention has a smaller polymer residue (89-96 ppm VS 139-145 ppm), a smaller roughness (9.4-9.8 nm VS 13.8-14.1 nm), a better low-temperature storage stability (10D OK VS 6-8D NG), a better alignment effect, and a better pre-tilt angle stability (0.21-0.22 VS 0.31-0.32) while maintaining an appropriate clearing point, an appropriate optical anisotropy, an appropriate absolute value of dielectric anisotropy, a larger K value (K₁₁ and K₃₃) and a smaller rotational viscosity.

### Comparative Examples 40-42 and Examples 83-86

The liquid crystal compositions of Comparative Examples 40-42 and Examples 83-86 are prepared according to the parts by weight of each compound listed in Table 38. The obtained liquid crystal compositions are tested for performance by filling each liquid crystal composition into a "non-aligned" test cell (cell gap d is 3.5 µm, with ITO coatings (structured ITO for multi-domain switching mode) on both substrates, no alignment layer, and no passivation layer). The test results for relevant performances of the liquid crystal compositions of Comparative Examples 40-42 and Examples 83-86 are shown in Table 39 below.

**Table 38 Formulations of the liquid crystal compositions of Comparative Examples 40-42 and Examples 83-86**

| | Comp. Ex. 40 | Comp. Ex. 41 | Comp. Ex. 42 | Ex. 83 | Ex. 84 | Ex. 85 | Ex. 86 |
|---|---|---|---|---|---|---|---|
| Host-21 | 100 | 100 | 100 | 100 | 100 | 100 | 100 |
| RM-2-1 | 0.3 | 0.3 | 0.3 | 0.3 | 0.3 | 0.3 | 0.3 |
| D-1 | 1 | | | | | | |
| D-4 | | 1 | | | | | |
| D-3' | | | 1 | | | | |
| AD-2 | | | | 1 | | | |
| AD-1 | | | | | 1 | | |
| AD-3 | | | | | | 1 | |
| AD-4 | | | | | | | 1 |

**Table 39 Test results for performances of the liquid crystal compositions of Comparative Examples 40-42 and Examples 83-86**

| | Comp. Ex. 40 | Comp. Ex. 41 | Comp. Ex. 42 | Ex. 83 | Ex. 84 | Ex. 85 | Ex. 86 |
|---|---|---|---|---|---|---|---|
| Residue conc. | 149 | 144 | 158 | 101 | 97 | 102 | 102 |
| Ra | 14.9 | 14.6 | 14.4 | 10.8 | 10.5 | 10.4 | 10.4 |
| t_{-10°C} | 8D NG | 7D NG | 7D NG | 10D OK | 10D OK | 10D OK | 10D OK |
| Alignment effect | Fair | Poor | Poor | Good | Good | Good | Good |
| PTA₍ᵢₙᵢₜᵢₐₗ₎ | 88.09 | 88.09 | 88.13 | 88.14 | 88.09 | 88.13 | 88.11 |
| PTA₍₁₆₅ₕ₎ | 87.77 | 87.76 | 87.8 | 87.9 | 87.85 | 87.88 | 87.89 |
| ΔPTA | 0.32 | 0.33 | 0.33 | 0.24 | 0.24 | 0.25 | 0.22 |

It can be seen from the comparison between Examples 83-86 and Comparative Examples 40-42 that, through structural optimization of the self-aligning agent, the liquid crystal composition of the present invention has a smaller polymer residue (97-102 ppm VS 144-158 ppm), a smaller roughness (10.4-10.8 nm VS 14.4-14.9 nm), a better low-temperature storage stability (10D OK VS 7-8D NG), a better alignment effect, and a better pre-tilt angle stability (0.22-0.25 VS 0.32-0.33) while maintaining an appropriate clearing point, an appropriate optical anisotropy, an appropriate absolute value of dielectric anisotropy, a larger K value (K₁₁ and K₃₃) and a smaller rotational viscosity.

The above embodiments are merely illustrative of the technical concepts and the features of the present invention, are included merely for purposes of illustration and implement of the present invention, and are not intended to limit the scope of the present invention. Equivalent variations or modifications are intended to be included within the scope of the present invention.

## Claims

1. A self-aligning agent of general formula O: 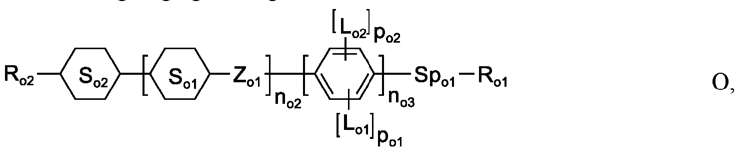 wherein,
Rₒ₂ represents -Spₒ₂-Pₒ₁, -H, C₁₋₁₂ linear alkyl, C₃₋₁₂ branched alkyl, 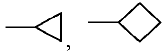 or 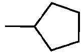 wherein one or more than two nonadjacent -CH₂- in the C₁₋₁₂ linear alkyl, 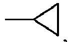 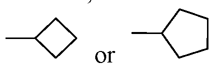 can each be independently replaced by -CH=CH-, -C≡C-, -O-, -CO-, -CO-O- or -O-CO-, and one or more -H in the C₁₋₁₂ linear alkyl can each be independently substituted by -F or -Cl;
ring 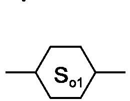 represents 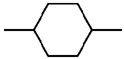 or 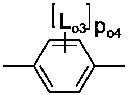 wherein one or more -CH₂-in 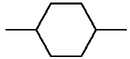 can be replaced by -O-, and one or at most two single bonds in the ring can be replaced by double bond;
ring 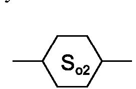 represents 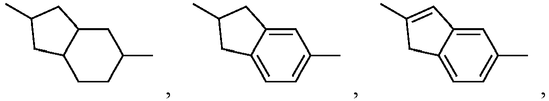 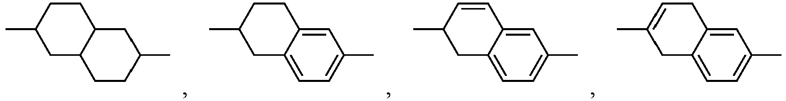 or 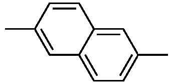 wherein, one or more than two nonadjacent -CH₂- in the above groups can each be independently replaced by -O- or -S-, and one or more -H on the above groups can each be independently substituted by -F or C₁₋₅ halogenated or unhalogenated linear alkyl;
Lₒ₁ and Lₒ₃ each independently represents -F, -Cl, -CN, -NO₂, -NCO, -NCS, -OCN, -SCN, -C(O)N(R^{o0})₂, -C(O)R^{o0}, C₁₋₁₂ linear alkyl, C₃₋₁₂ branched alkyl. 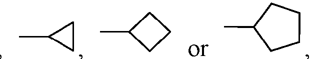 wherein one or more than two nonadjacent -CH₂- in the C₁₋₁₂ linear alkyl, 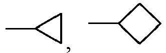 or 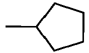 can each be independently replaced by -CH=CH-, -C≡C-, -O-, -CO-, -CO-O- or -O-CO-, and one or more -H in the C₁₋₁₂ linear alkyl can each be independently substituted by -F, wherein R^{o0} represents C₁₋₁₂ linear alkyl or C₃₋₁₂ branched alkyl;
Lₒ₂ represents - Spₒ₃- Pₒ₂ or 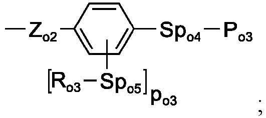
Rₒ₁ and Rₒ₃ each independently represents an anchoring group, the anchoring group is 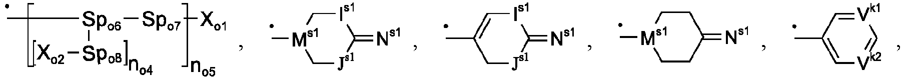 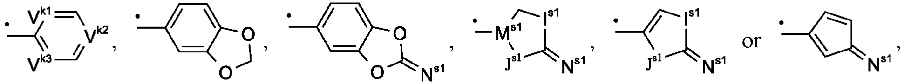 wherein 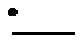 represents the binding site in the bound structure;
nₒ₄ represents 1 or 2, wherein when nₒ₄ represents 2, -Spₒ₈-Xₒ₂ can be the same or different;
nₒ₅ represents 0 or 1;
M^{S1} represents 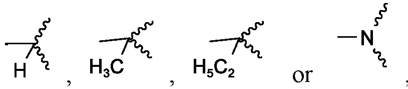 wherein, 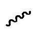 represents the binding site between M^{S1} and -CH₂- in the six-membered ring;
I^{S1} and J^{S1} each independently represents -CH₂-, -O- or -S-;
N^{S1} represents =O or =S;
V^{K1}, V^{K2} and V^{K3} each independently represents -CH= or -N=;
Xₒ₁ and Xₒ₂ each independently represents -H, -OH, -SH, -NH₂, -NHR¹¹, -N(R¹¹)₂, -NHC(O)R¹¹, -OR¹¹, -C(O)OH, -CHO, C₁₋₁₂ linear halogenated or unhalogenated alkyl or C₃₋₁₂ branched halogenated or unhalogenated alkyl, wherein at least one of Xₒ₁ and Xₒ₂ is selected from a group consisting of -OH, -SH, -NH₂, -NHR¹¹, -C(O)OH and -CHO, wherein R¹¹ represents C₁₋₁₂ linear alkyl or C₃₋₁₂ branched alkyl;
Pₒ₁, Pₒ₂ and Pₒ₃ each independently represents polymerizable group;
Spₒ₁, Spₒ₂, Spₒ₃, Spₒ₄, Spₒ₅, Spₒ₇ and Spₒ₈ each independently represents spacer group or single bond;
Spₒ₆ represents 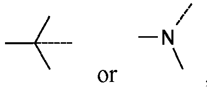 wherein, ----- represents the binding site to Spₒ₇ or Spₒ₈;
Zₒ₁ and Zₒ₂ each independently represents -O-, -S-, -CO-, -CO-O-, -O-CO-, -O-CO-O-, -CH₂O-, -OCH₂-, -CH₂S-, -SCH₂-, -CF₂O-, -OCF₂-, -CF₂S-, -SCF₂-, -(CH₂)_{d}-, -CF₂CH₂-, -CH₂CF₂-, -(CF₂)_{d}-, -CH=CH-, -CF=CF-, -CH=CF-, -CF=CH-, -C≡C-, -CH=CH-CO-O-, -O-CO-CH=CH-, -CH₂CH₂-CO-O-, -O-CO-CH₂CH₂-, -CHR¹-, -CR¹R²- or single bond, wherein R¹ and R² each independently represents C₁₋₁₂ linear alkyl or C₃₋₁₂ branched alkyl, and d represents an integer of 1-4;
pₒ₁, pₒ₂, pₒ₃ and pₒ₄ each independently represents 0, 1 or 2, wherein when pₒ₁ represents 2, Lₒ₁ can be the same or different, wherein when pₒ₂ represents 2, Lₒ₂ can be the same or different;
wherein when pₒ₃ represents 2, -Spₒ₅- Rₒ₃ can be the same or different; wherein when pₒ₄ represents 2, Lₒ₃ can be the same or different; and
nₒ₂ represents 0, 1, 2 or 3, nₒ₃ represents 1, 2 or 3, wherein when nₒ₂ represents 2 or 3, 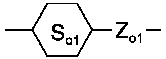
can be the same or different, wherein when nₒ₃ represents 2 or 3, 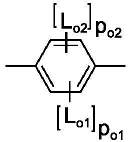
can be the same or different.

2. The self-aligning agent according to claim 1, **characterized in that**, the self-aligning agent of general formula O is selected from a group consisting of the following compounds: 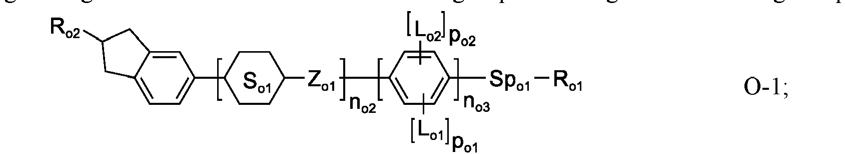 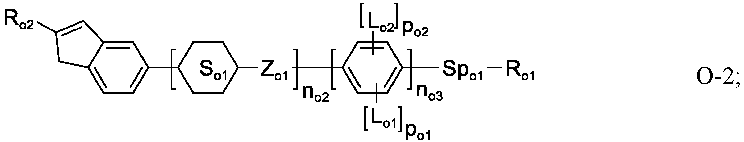 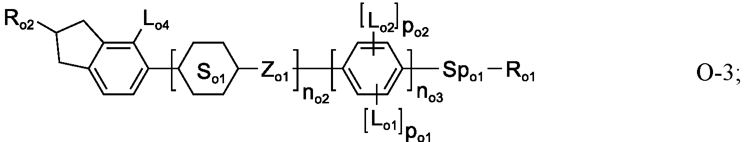 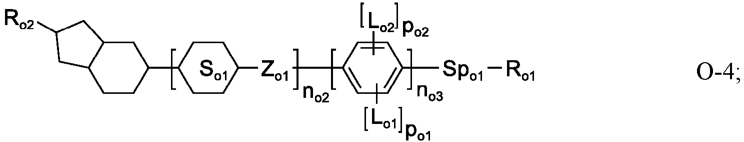 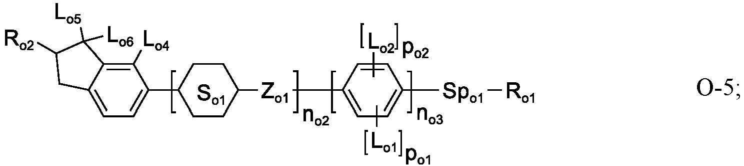 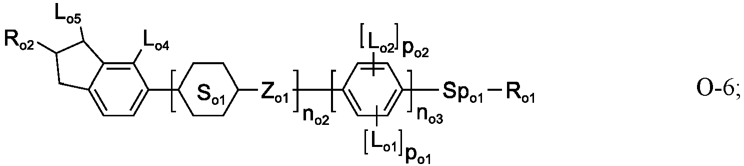 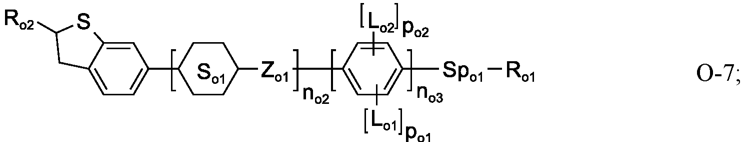 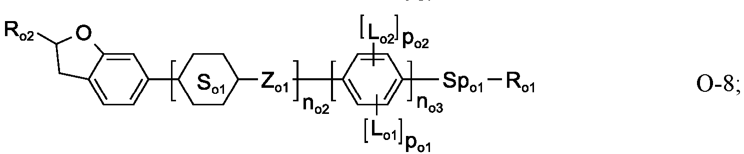 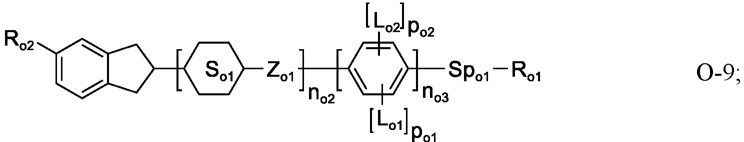 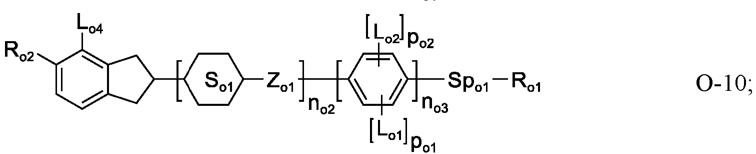 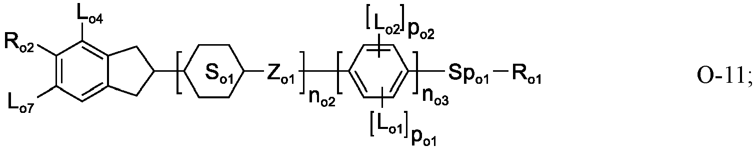 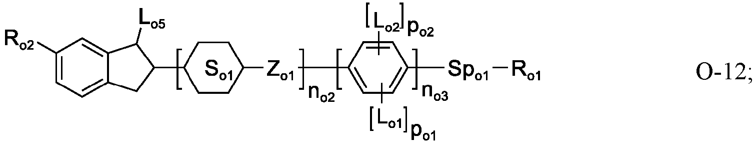 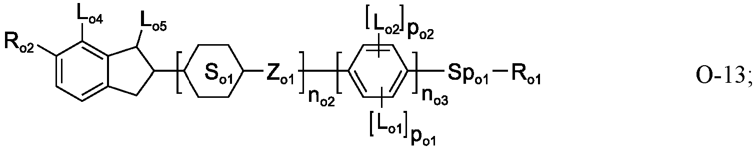 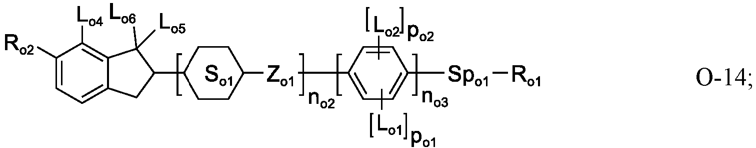 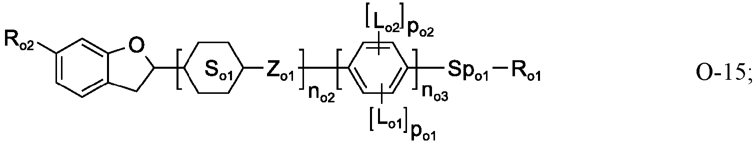 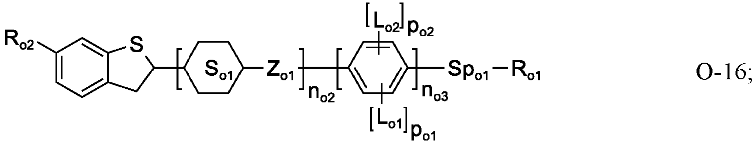 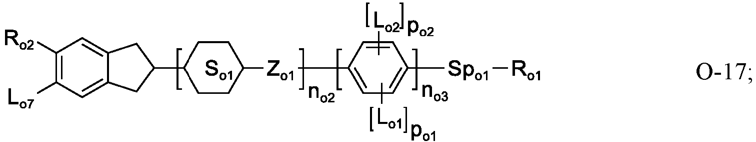 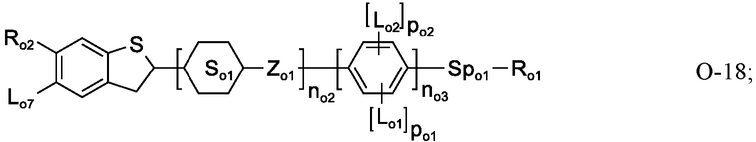 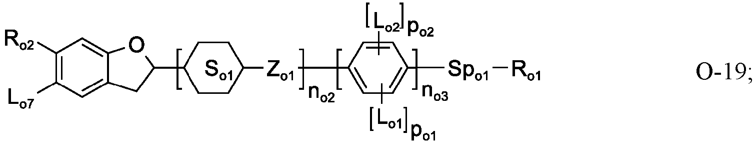 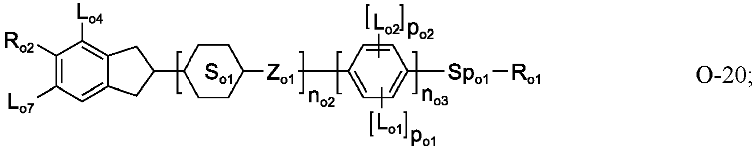 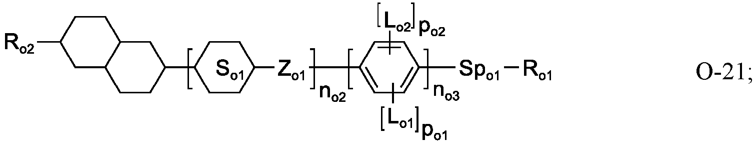 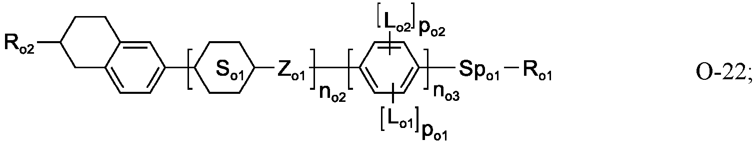 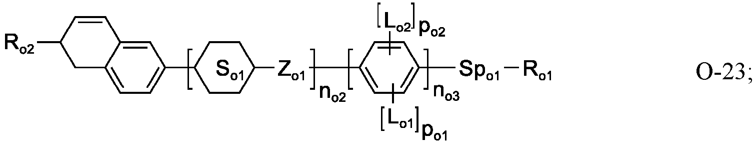 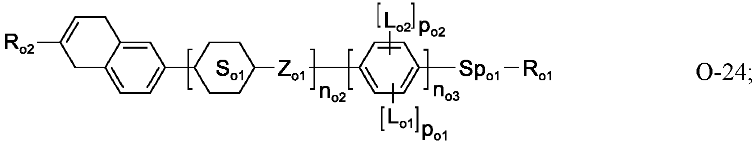 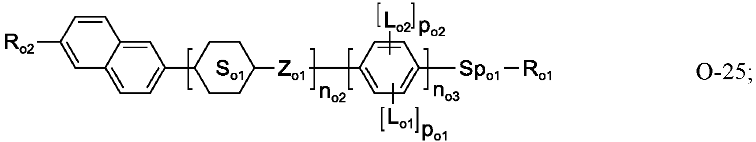 and 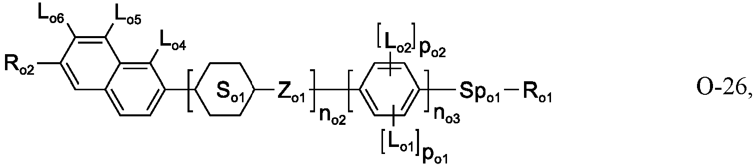 wherein,
Lₒ₄~Lₒ₇ each independently represents -F or C₁₋₅ halogenated or unhalogenated linear alkyl.

3. The self-aligning agent according to claim 2, **characterized in that**, the compound of general formula O-1 is selected from a group consisting of the following compounds: 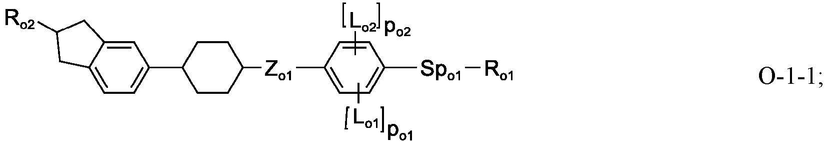 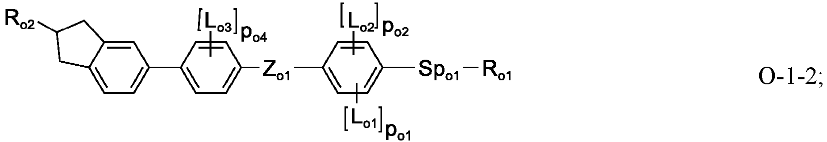 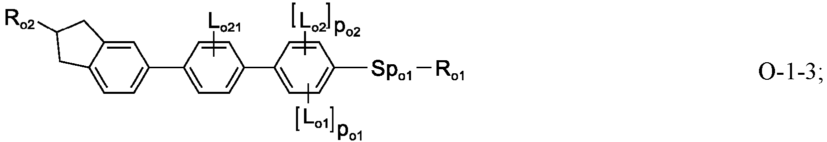 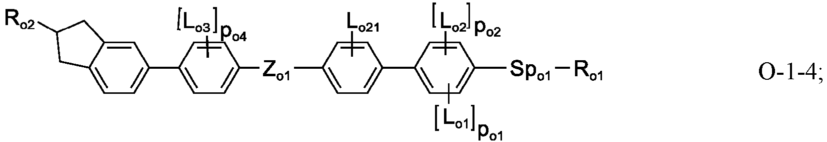 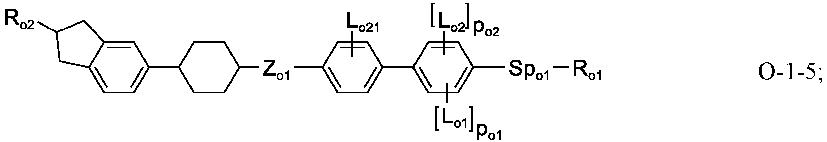 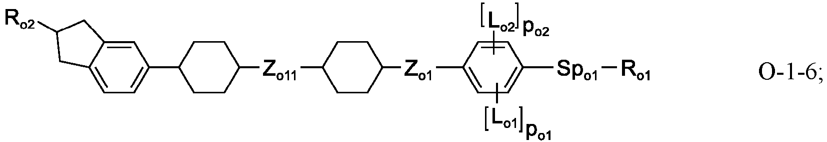 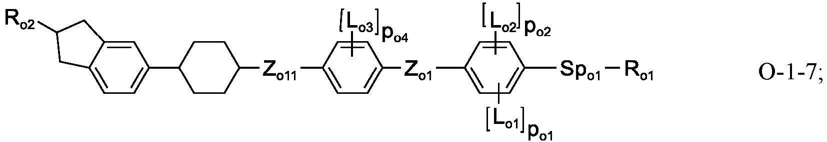 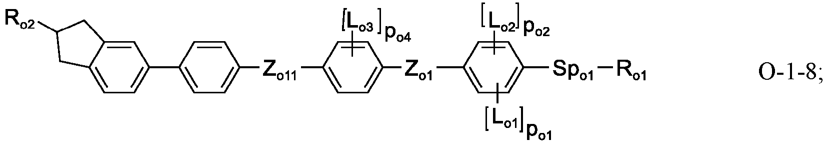 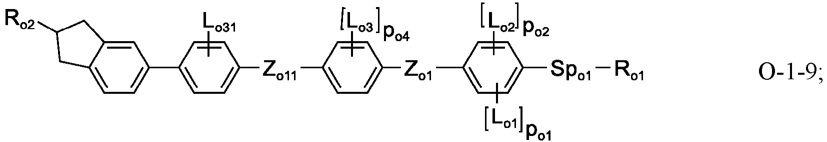 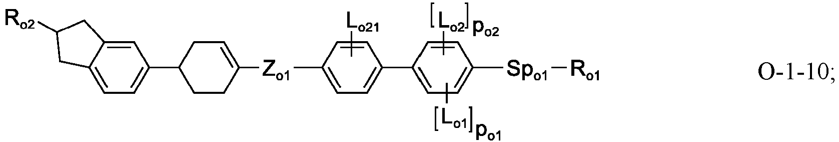 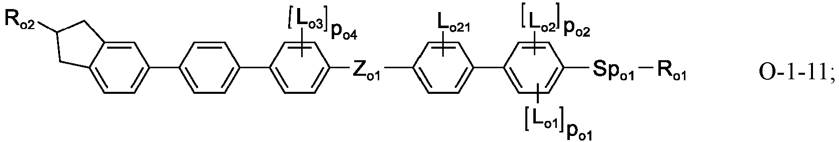 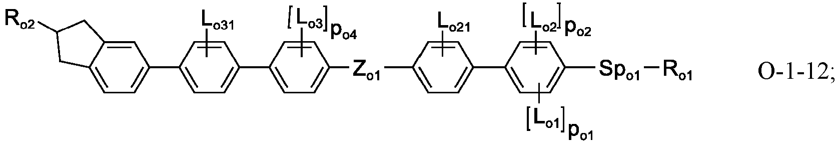 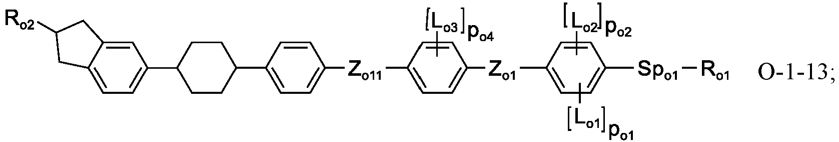 and 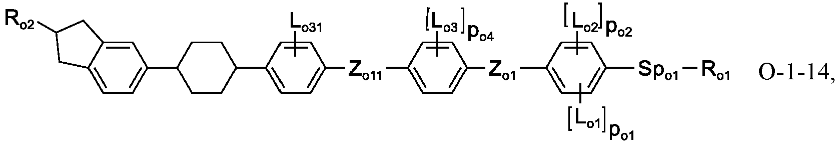 wherein,
Zₒ₁₁ represents -O-, -S-, -CO-, -CO-O-, -O-CO-, -O-CO-O-, -CH₂O-, -OCH₂-, -CH₂S-, -SCH₂-, -CF₂O-, -OCF₂-, -CF₂S-, -SCF₂-, -(CH₂)_{d}-, -CF₂CH₂-, -CH₂CF₂-, -(CF₂)_{d}-, -CH=CH-, -CF=CF-, -CH=CF-, -CF=CH-, -C≡C-, -CH=CH-CO-O-, -O-CO-CH=CH-, -CH₂CH₂-CO-O-, -O-CO-CH₂CH₂-, -CHR¹-, -CR¹R²- or single bond, wherein R¹ and R² each independently represents C₁₋₁₂ linear alkyl or C₃₋₁₂ branched alkyl, and d represents an integer of 1-4;
Lₒ₃₁ represents -F, -Cl, -CN, -NO₂, -NCO, -NCS, -OCN, -SCN, -C(O)N(R^{o0})₂, -C(O)R^{o0}, C₁₋₁₂ linear alkyl, C₃₋₁₂ branched alkyl, 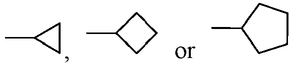 wherein one or more than two nonadjacent -CH₂- in the C₁₋₁₂ linear alkyl, 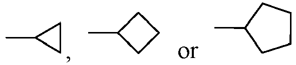 can each be independently replaced by -CH=CH-, -C≡C-, -O-, -CO-, -CO-O- or -O-CO-, and one or more -H in the C₁₋₁₂ linear alkyl can each be independently substituted by -F, wherein R^{o0} represents C₁₋₁₂ linear alkyl or C₃₋₁₂ branched alkyl; and
Lₒ₂₁ represents - Spₒ₃- Pₒ₂ or

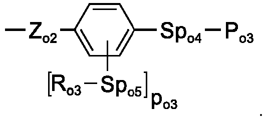

4. The self-aligning agent according to claim 3, **characterized in that**, the compound of general formula O-1 is selected from a group consisting of the following compounds: 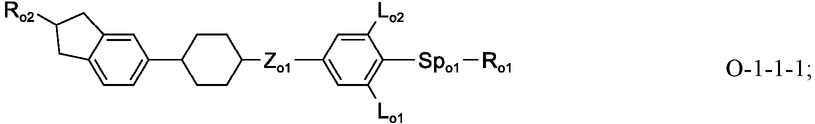 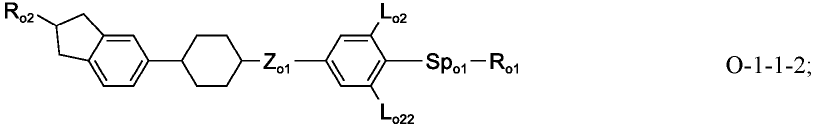 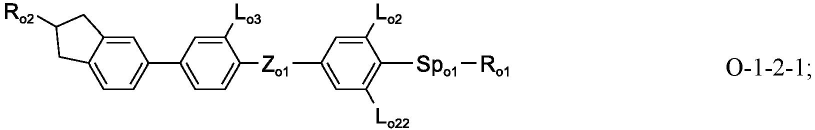 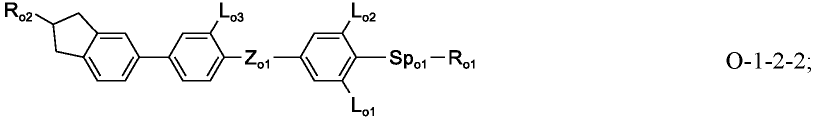 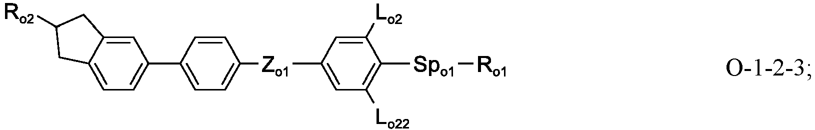 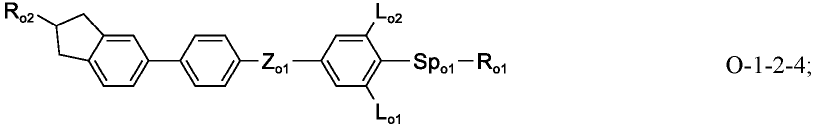 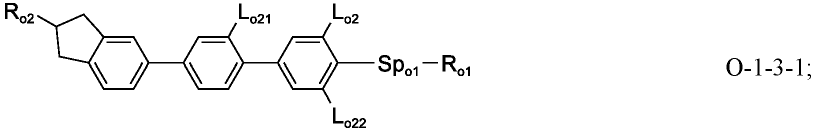 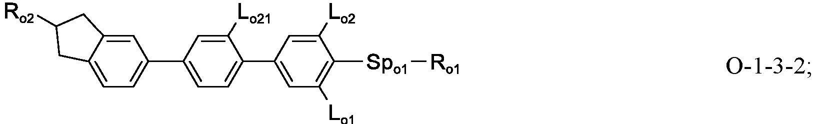 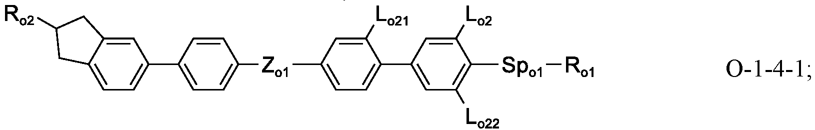 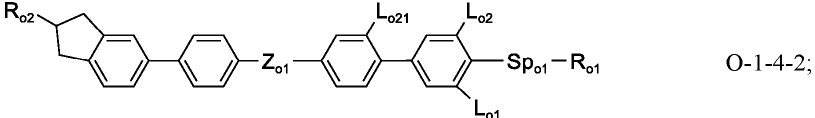 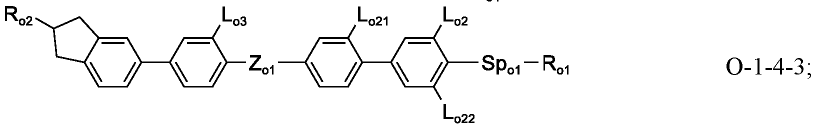 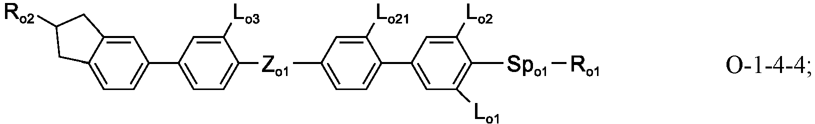 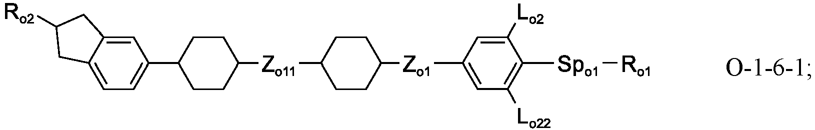 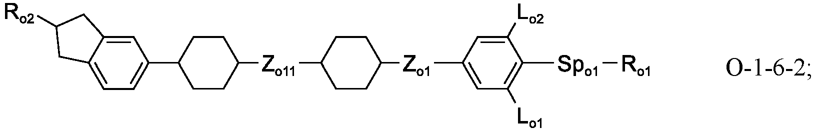 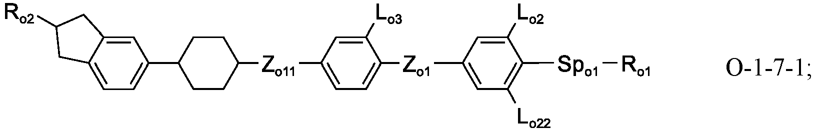 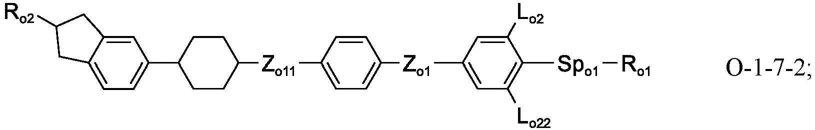 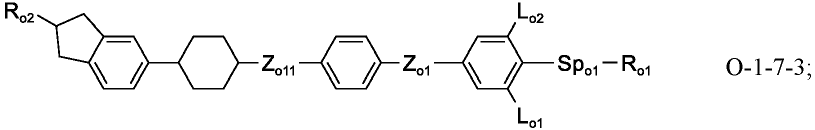 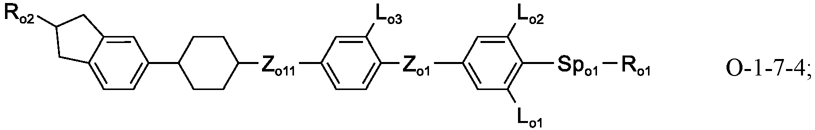 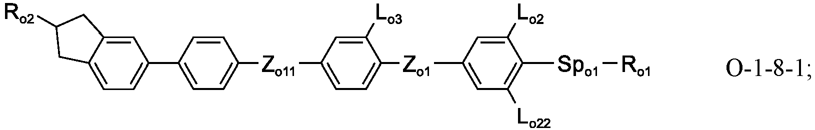 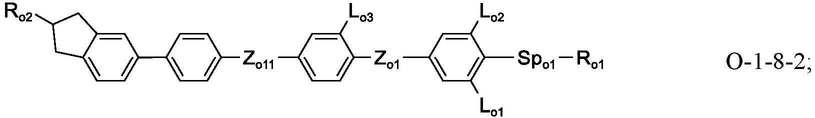 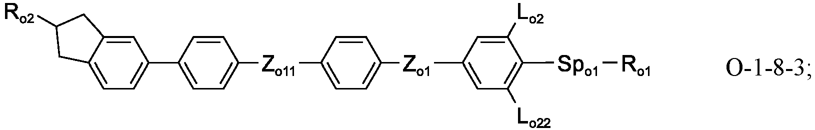 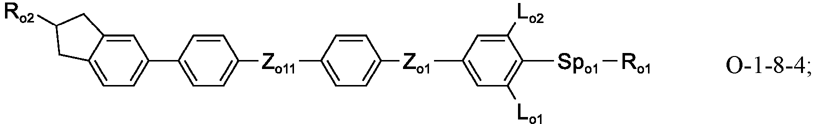 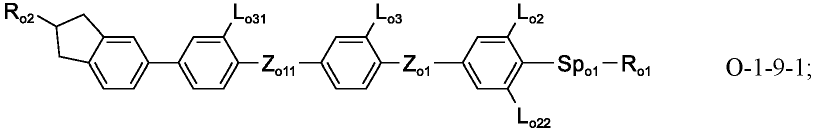 and 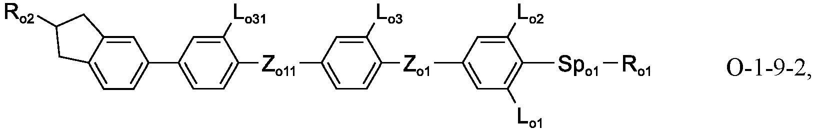 wherein,
Lₒ₂₂ represents - Spₒ₃- Pₒ₂ or

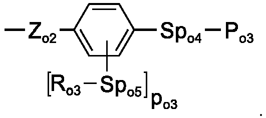

5. A liquid crystal composition comprising the self-aligning agent of any one of claims 1-4.

6. The liquid crystal composition according to claim 5, **characterized in that**, the liquid crystal composition comprises at least one compound of general formula M: 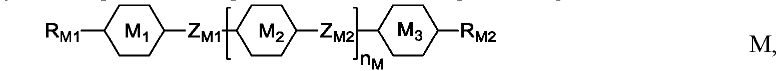 wherein,
R_{M1} and R_{M2} each independently represents C₁₋₁₂ linear alkyl, C₃₋₁₂ branched alkyl, 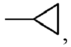 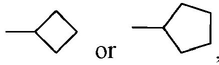 wherein one or more than two nonadjacent -CH₂- in the C₁₋₁₂ linear alkyl or C₃₋₁₂ branched alkyl can each be independently replaced by -CH=CH-, -C≡C-, -O-, -CO-, -CO-O- or -O-CO-;
ring 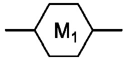 ring 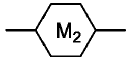 and ring 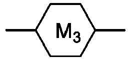 each independently represents 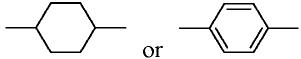 wherein one or more -CH₂- in 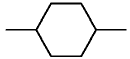 can be replaced by -O-, one or at most two single bonds in the ring can be replaced by double bond, at most one -H on 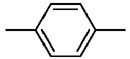 can be substituted by halo;
Z_{M1} and Z_{M2} each independently represents single bond,-CO-O-, -O-CO-, -CH₂O-, -OCH₂-, -C≡C-, -CH=CH-, -CH₂CH₂- or -(CH₂)₄-; and
n_{M} represents 0, 1 or 2, wherein when n_{M} = 2, ring 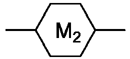 can be the same or different, Z_{M2} can be the same or different.

7. The liquid crystal composition according to claim 6, **characterized in that**, the compound of general formula M is selected from a group consisting of the following compounds: 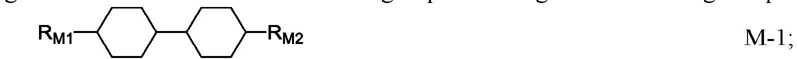 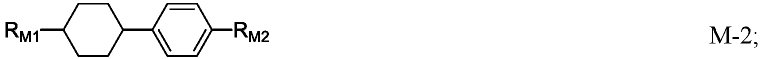 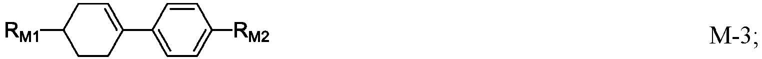 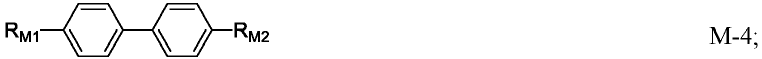 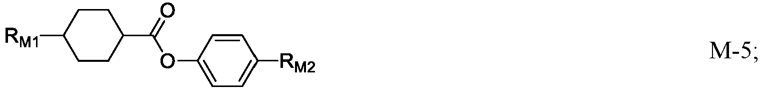 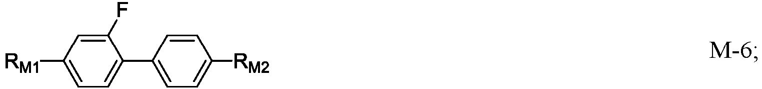 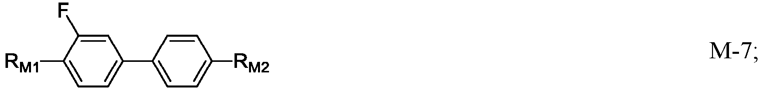 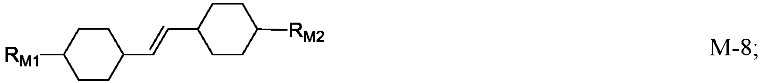 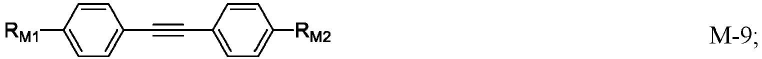 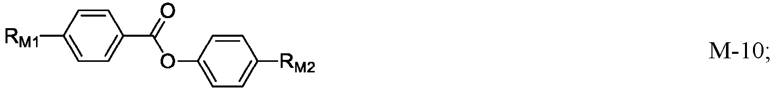 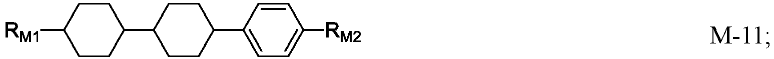 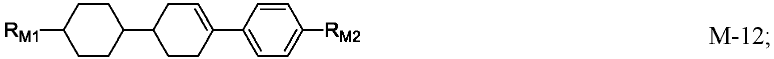 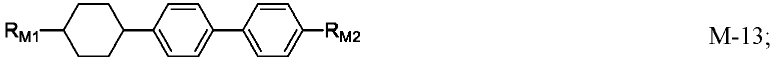 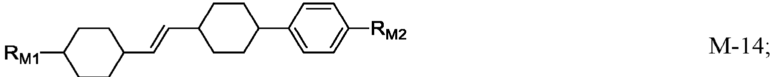 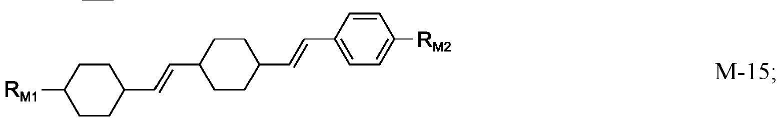 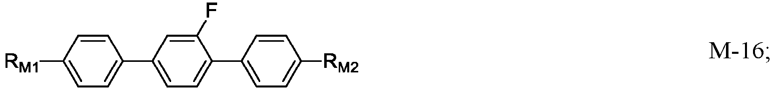 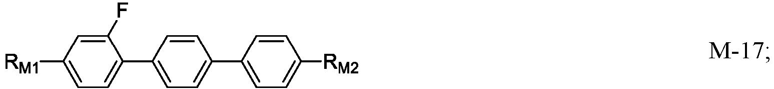 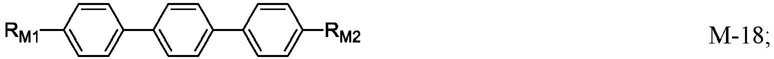 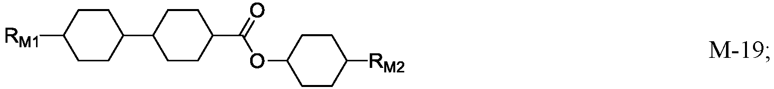 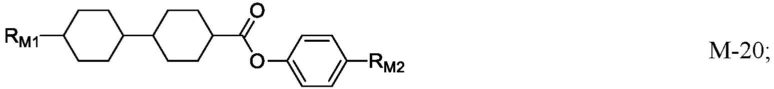 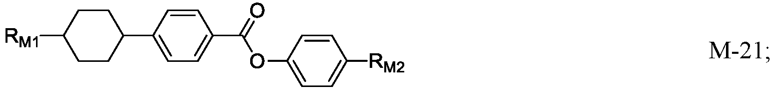 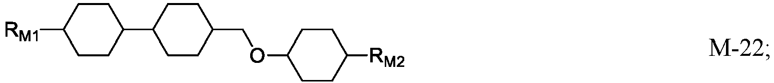 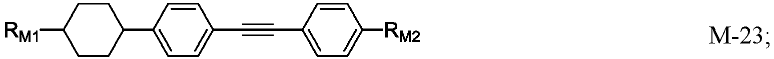 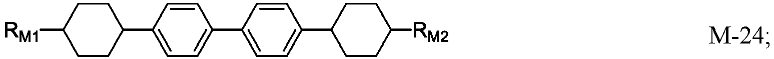 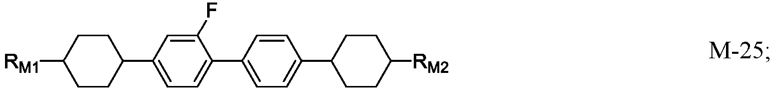 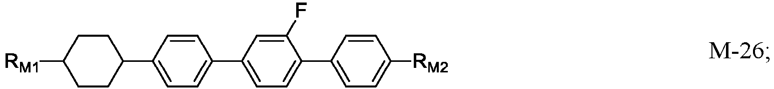 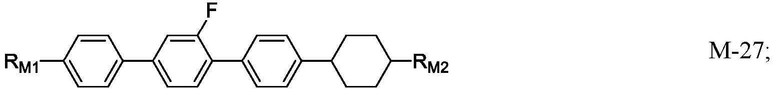 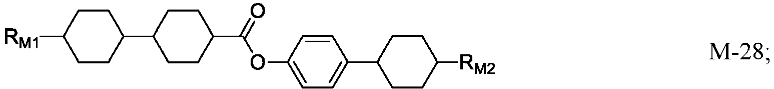 and

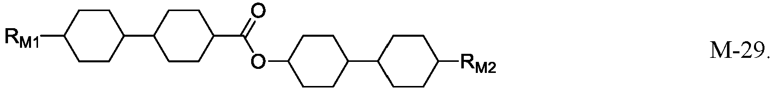

8. The liquid crystal composition according to claim 5, **characterized in that**, the liquid crystal composition comprises at least one compound of general formula N: 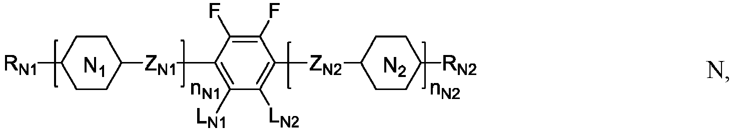 wherein,
R_{N1} and R_{N2} each independently represents -H, C₁₋₁₂ linear alkyl, C₃₋₁₂ branched alkyl, 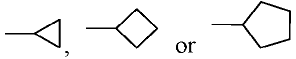 wherein one or more than two nonadjacent -CH₂- in the C₁₋₁₂ linear alkyl or C₃₋₁₂ branched alkyl can each be independently replaced by -CH=CH-, -C≡C-, -O-, -CO-, -CO-0- or -O-CO-;
ring 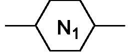 and ring 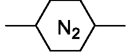 each independently represents 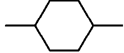 or 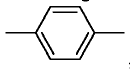 wherein one or more -CH₂- in 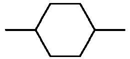 can be replaced by -O-, and one or at most two single bonds in the ring can be replaced by double bond, wherein one or more -H on 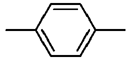 can each be independently substituted by -F, -Cl or -CN, and one or more -CH= in ring can be replaced by -N=;
Z_{N1} and Z_{N2} each independently represents single bond,-CO-O-, -O-CO-, -CH₂O-, -OCH₂-, -CH=CH-, -C≡C-, CH₂CH₂-, -CF₂CF₂-, -(CH₂)₄-, -CH=CH(CH₂)n_{N3}, -CF₂O- or -OCF₂-;
L_{N1} and L_{N2} each independently represents -H, halo or C₁₋₃ alkyl;
n_{N1} represents 0, 1, 2 or 3, n_{N2} represents 0 or 1, and 0 ≤ n_{N1} + n_{N2} ≤ 3, when n_{N1} = 2 or 3, ring 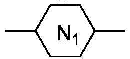 can be the same or different, Z_{N1} can be the same or different, and n_{N3} represents 0, 1, 2 or 3.

9. The liquid crystal composition according to claim 8, **characterized in that**, the compound of general formula N is selected from a group consisting of the following compounds: 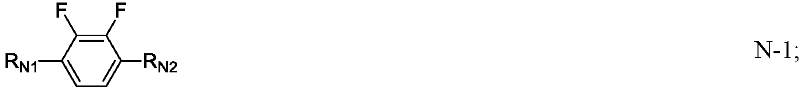 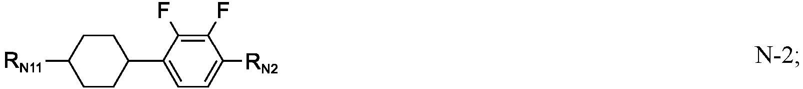 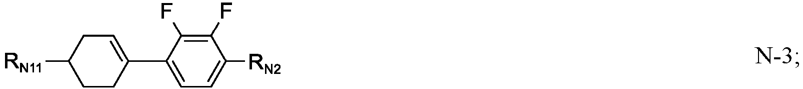 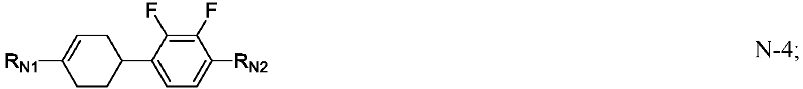 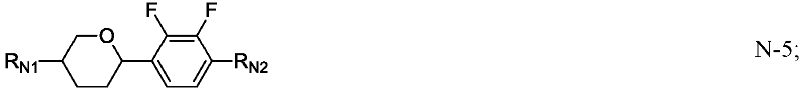 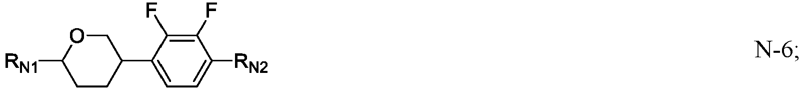 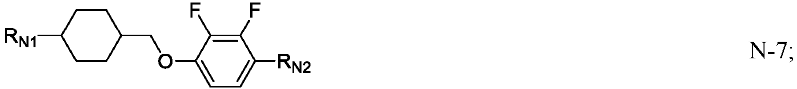 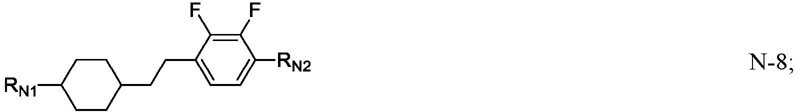 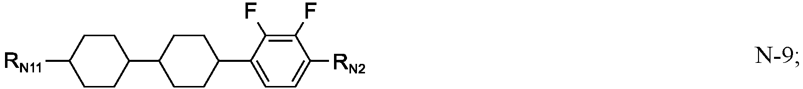 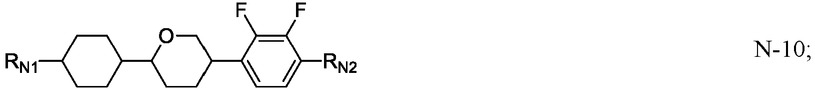 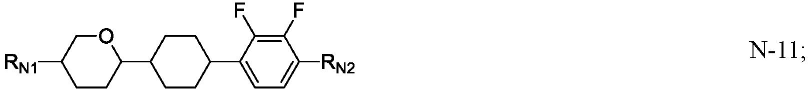 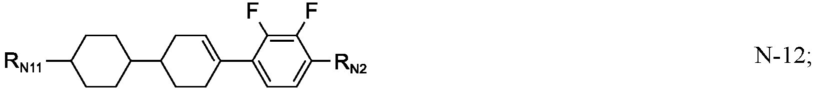 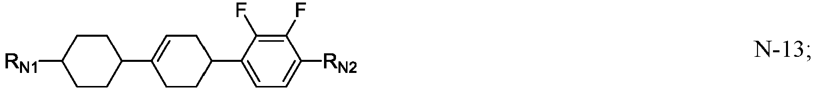 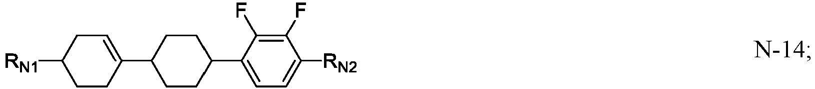 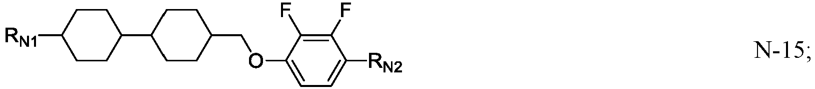 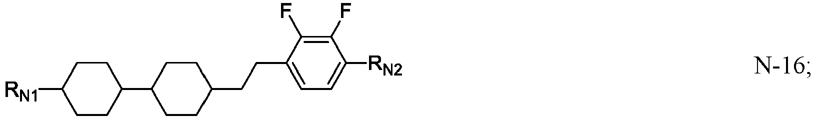 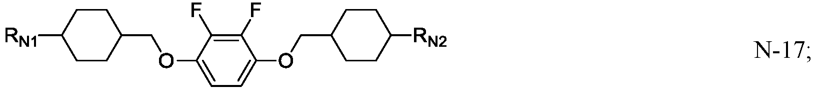 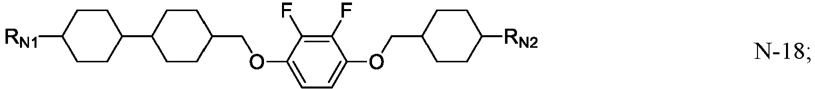 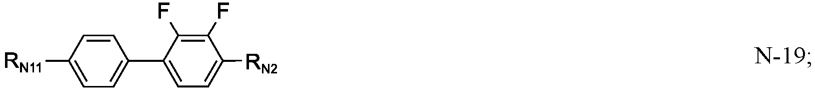 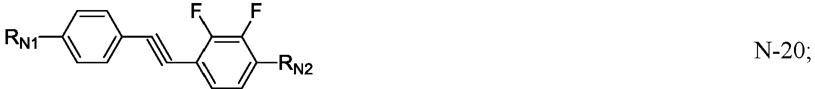 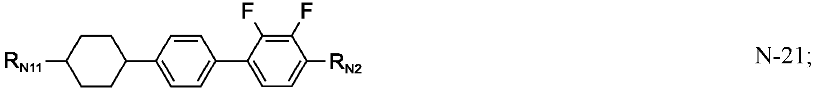 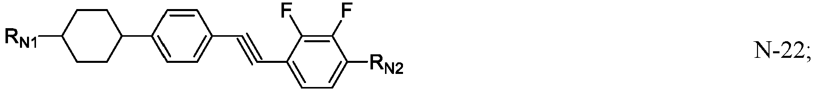 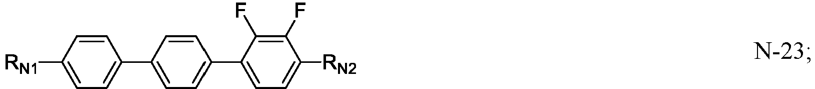 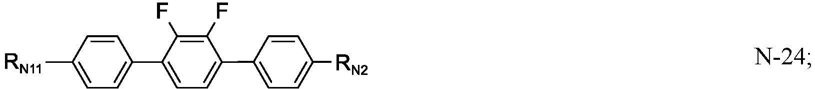 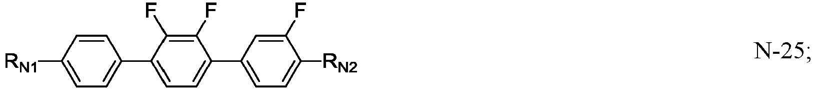 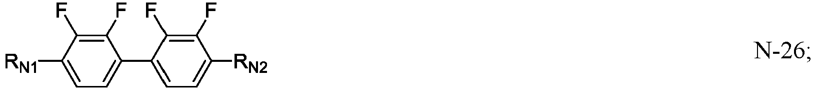 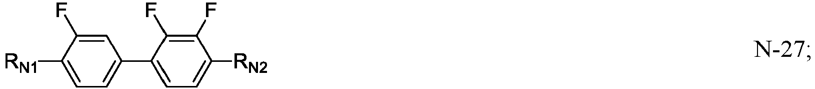 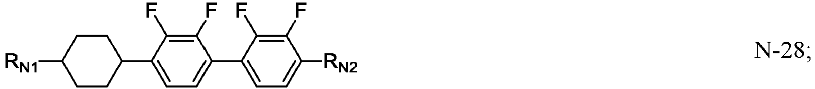 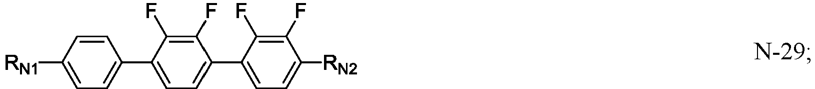 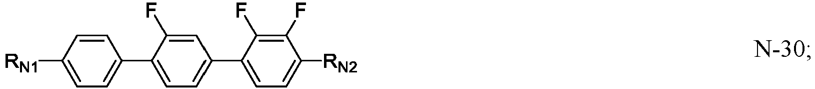 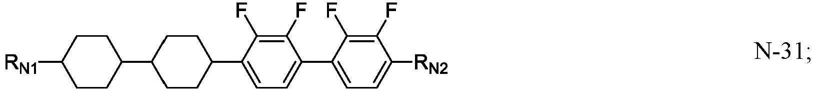 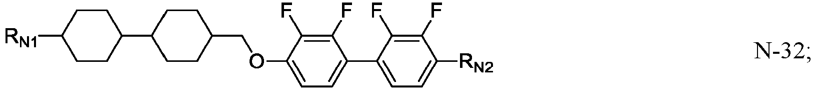 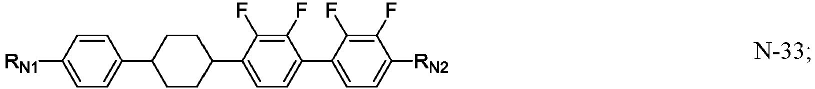 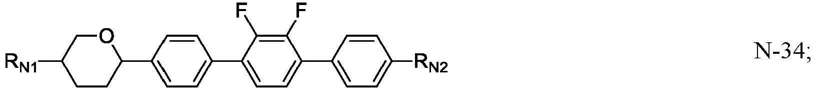 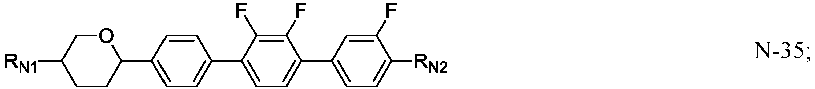 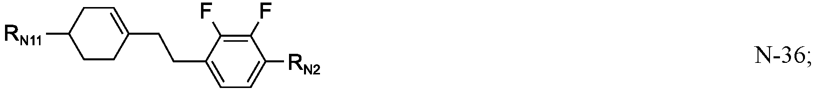 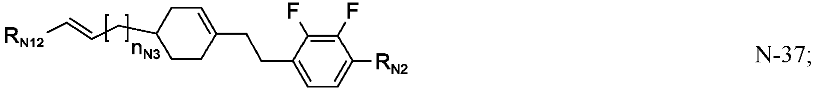 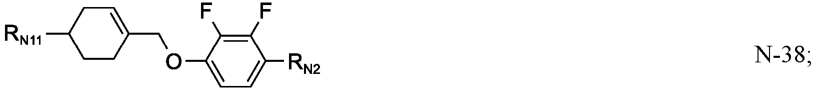 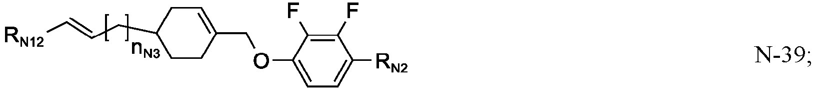 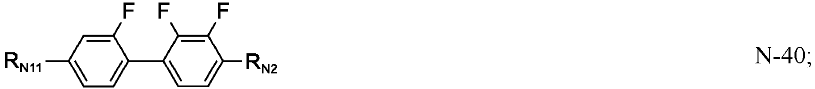 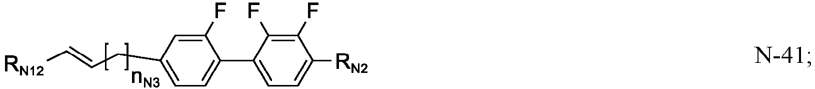 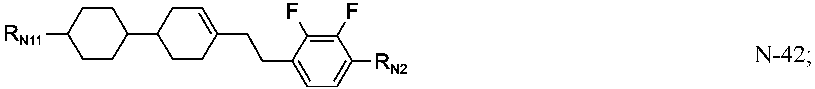 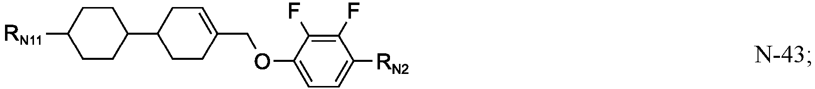 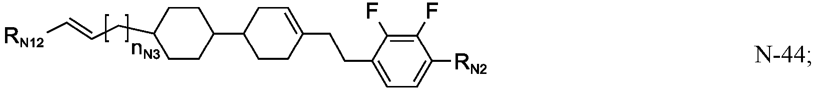 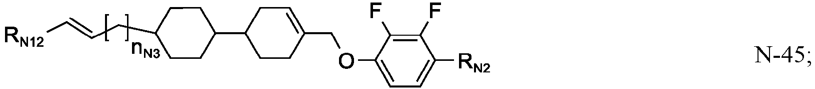 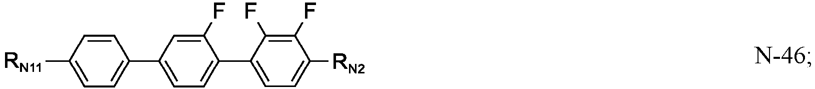 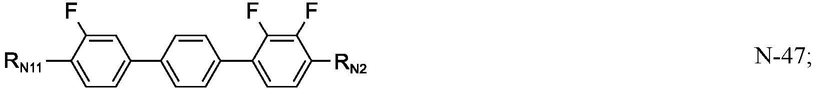 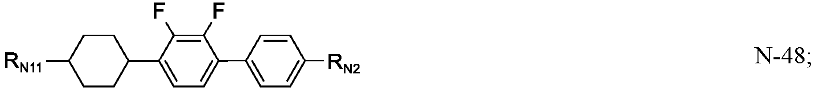 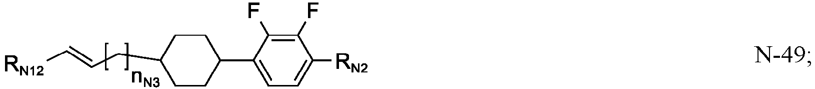 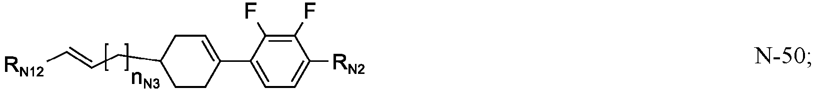 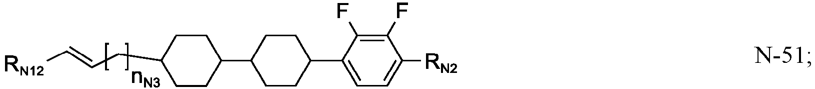 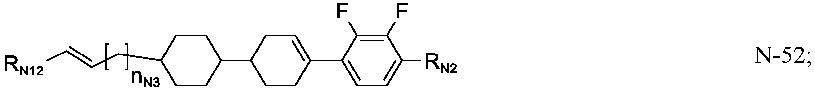 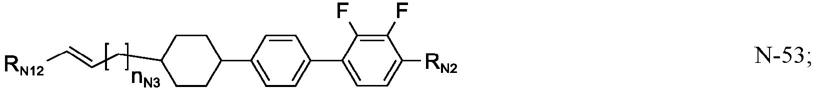 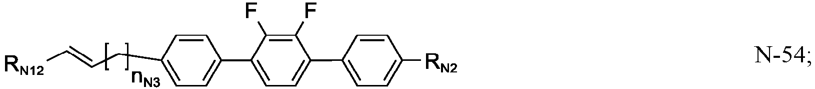 and 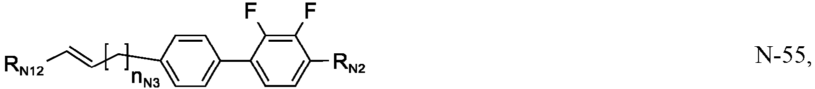 wherein,
R_{N11} represents C₁₋₅ linear alkyl, 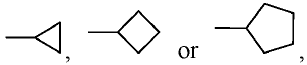 one or more than two nonadjacent -CH₂- in the C₁₋₅ linear alkyl can each be independently replaced by -O-, -CO-, -CO-O- or -O-CO-;
R_{N12} represents -H, C₁₋₅ linear alkyl, 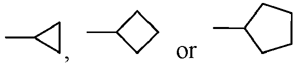 one or more than two nonadjacent -CH₂- in the C₁₋₅ linear alkyl can each be independently replaced by -CH=CH-, -C≡C-, -O-, -CO-, -CO-O- or -O-CO-; and
n_{N3} represents 0, 1, 2 or 3.

10. The liquid crystal composition according to claim 5, **characterized in that**, the liquid crystal composition comprises at least one polymerizable compound of general formula RM: 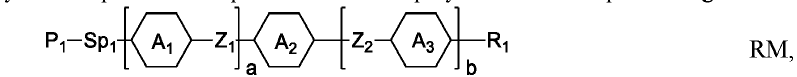 wherein,
R₁ represents -H, halo, -CN, -Sp₂-P₂, C₁₋₁₂ linear alkyl, C₃₋₁₂ branched alkyl, 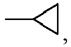 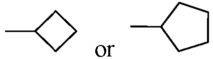 wherein one or more than two nonadjacent -CH₂- in the C₁₋₁₂ linear alkyl, C₃₋₁₂ branched alkyl, 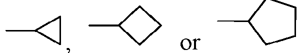 can each be independently replaced by -CH=CH-, -C≡C-, -O-, -CO-, -CO-O- or -O-CO-, and one or more -H can each be independently substituted by -F or -Cl;
ring 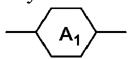 and ring 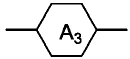 each independently represents 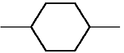 or 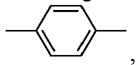 wherein one or more -CH₂- in 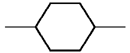 can be replaced by -O-, and one or at most two single bonds in the ring can be replaced by double bond, wherein one or more -H on 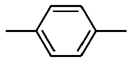 can each be independently substituted by -F, -Cl, -CN, -Sp₃-P₃, C₁₋₁₂ halogenated or unhalogenated linear alkyl, C₁₋₁₁ halogenated or unhalogenated linear alkoxy, 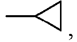 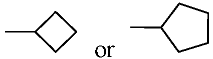 and one or more -CH= in the ring can be replaced by -N=;
ring 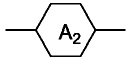 represents 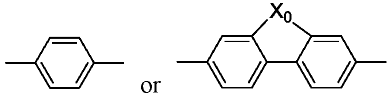 wherein one or more -H on 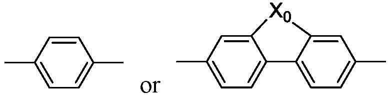 can each be independently substituted by -F, -Cl, -CN, -Sp₃-P₃, C₁₋₁₂ halogenated or unhalogenated linear alkyl, C₁₋₁₁ halogenated or unhalogenated linear alkoxy, 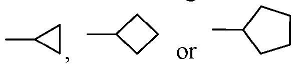 and one or more -CH= in the rings can be replaced by -N=;
P₁, P₂ and P₃ each independently represents polymerizable group;
X₀ represents -O-, -S- or -CO-;
Sp₁, Sp₂ and Sp₃ each independently represents spacer group or single bond;
Z₁ and Z₂ each independently represents -O-, -S-, -CO-, -CO-O-, -O-CO-, -O-CO-O-, -CH₂O-, -OCH₂-, -CH₂S-, -SCH₂-, -CF₂O-, -OCF₂-, -CF₂S-, -SCF₂-, -(CH₂)_{d}-, -CF₂CH₂-, -CH₂CF₂-, -(CF₂)_{d}-, -CH=CH-, -CF=CF-, -CH=CF-, -CF=CH-, -C≡C-, -CH=CH-CO-O-, -O-CO-CH=CH-, -CH₂CH₂-CO-O-, -O-CO-CH₂CH₂-, -CHR¹-, -CR¹R²- or single bond, wherein R¹ and R² each independently represents C₁₋₁₂ linear alkyl or C₃₋₁₂ branched alkyl, and d represents an integer of 1-4;
a represents 0, 1 or 2, b represents 0 or 1, wherein when a represents 2, ring 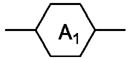 can be the same or different, Z₁ can be the same or different.

11. The liquid crystal composition according to claim 10, **characterized in that**, the polymerizable compound of general formula RM is selected from a group consisting of the following compounds: 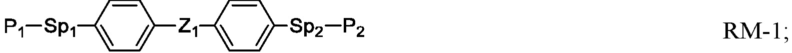 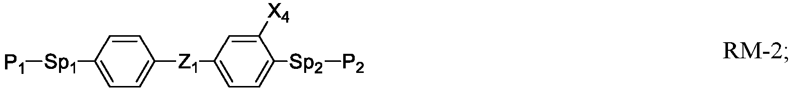 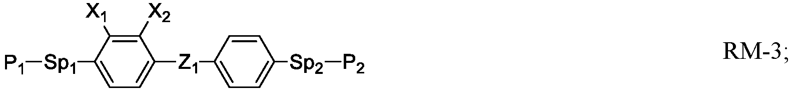 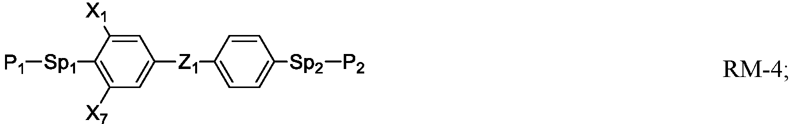 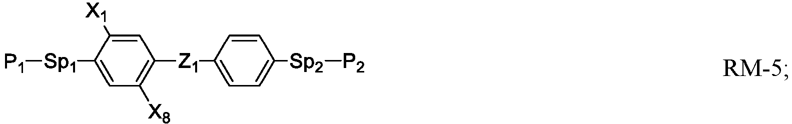 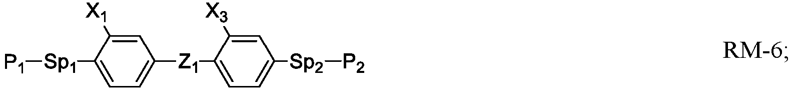 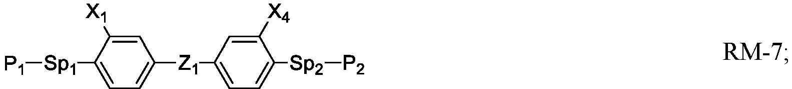 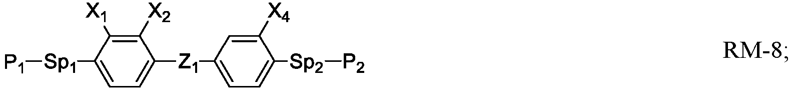 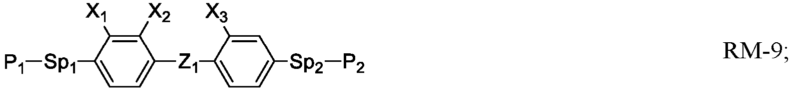 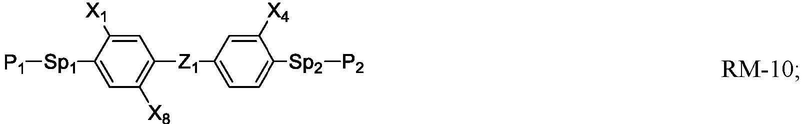 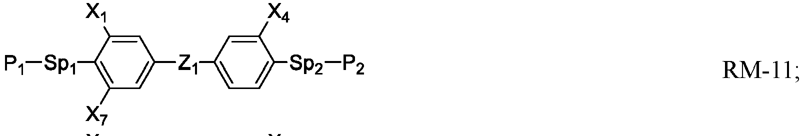 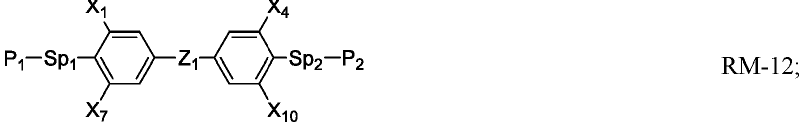 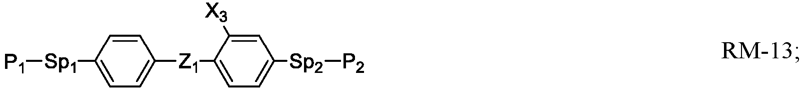 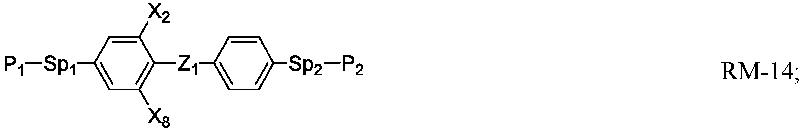 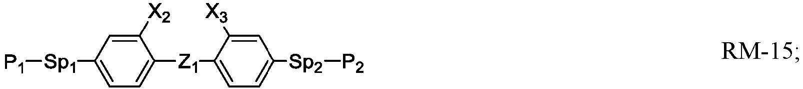 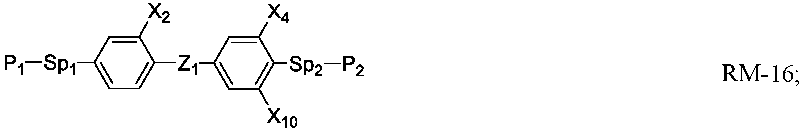 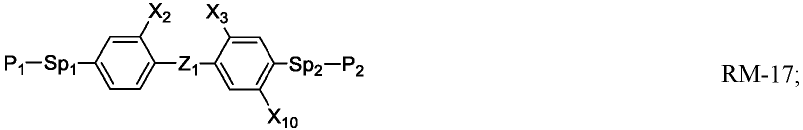 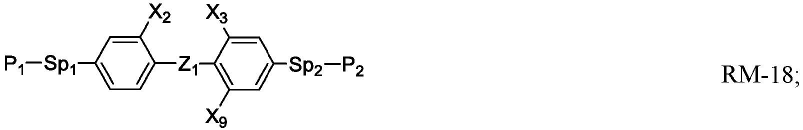 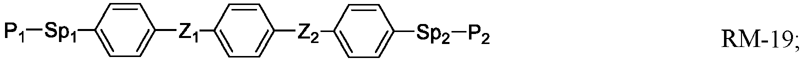 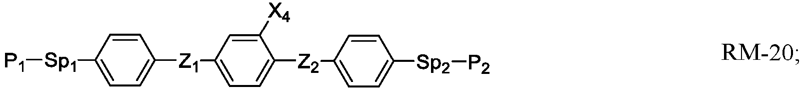 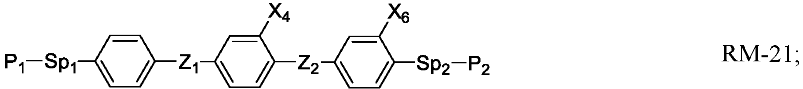 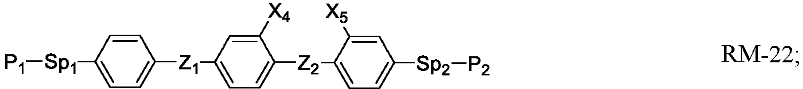 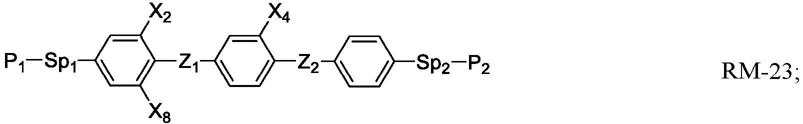 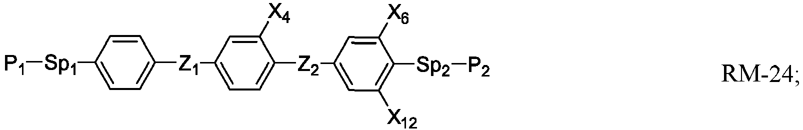 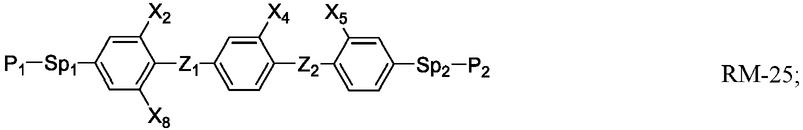 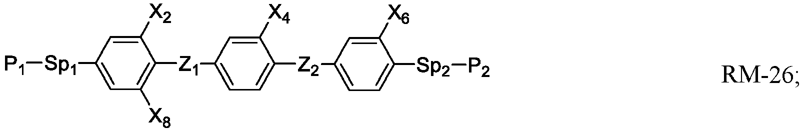 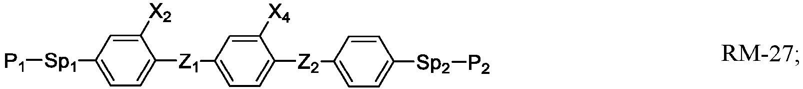 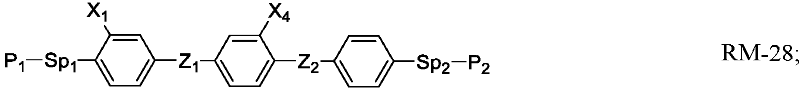 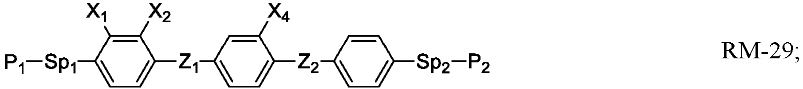 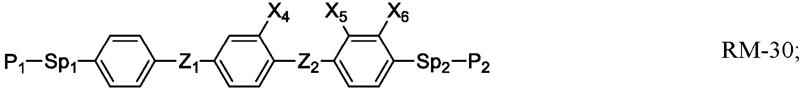 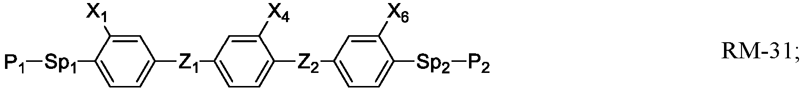 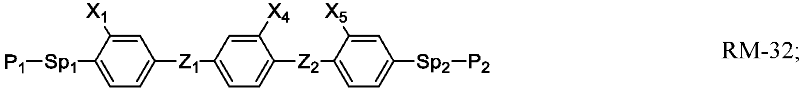 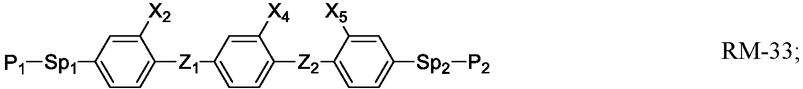 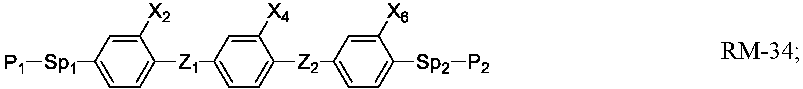 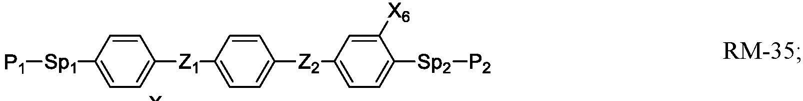 and 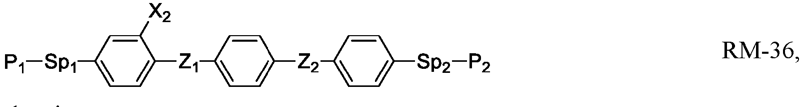 wherein,
X₁-X₁₀ and X₁₂ each independently represents -F, -Cl, -Sp₃-P₃, C₁₋₅ linear alkyl or alkoxy,

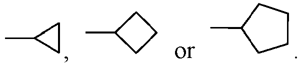

12. The liquid crystal composition according to claim 5, **characterized in that**, the liquid crystal composition comprises at least one compound of general formula B: 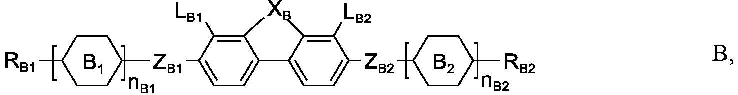 wherein,
R_{B1} and R_{B2} each independently represents halo, C₁₋₁₂ halogenated or unhalogenated linear alkyl, C₃₋₁₂ halogenated or unhalogenated branched alkyl, 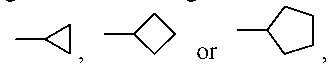 wherein one or more than two nonadjacent -CH₂- in the C₁₋₁₂ halogenated or unhalogenated linear alkyl, C₃₋₁₂ halogenated or unhalogenated branched alkyl, 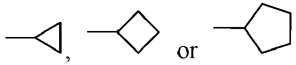 can each be independently replaced by -CH=CH-, -CH=CF-, -C≡C-, -O-, -CO-, -CO-O- or -O-CO-, wherein at most one single bond in 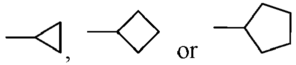 can be replaced by double bond;
ring 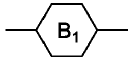 and ring 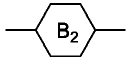 each independently represents 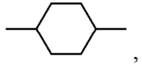 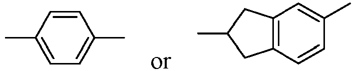 wherein one or more -CH₂- in 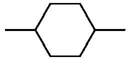 or 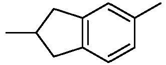 can be replaced by -O-, and one or at most two single bonds in the rings can be replaced by double bond, wherein one or more -H on 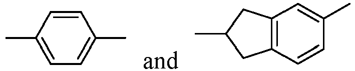 can each be independently substituted by -CN, -F or -Cl, and one or more -CH= in the rings can be replaced by -N=;
X_{B} represents -O-, -S- or -CO-;
L_{B1} and L_{B2} each independently represents -H, -F, -Cl, -CF₃ or -OCF₃;
Z_{B1} and Z_{B2} each independently represents -CO-O-, -O-CO-, -OCH₂-, -CH=CH-, -C≡C-, -CH₂CH₂-, -CF₂CF₂-, -(CH₂)n_{B3}-, -(CH₂)n_{B3}O-, -(CH₂)n_{B3}S-, -CF₂O- or -OCF₂-, wherein n_{B3} represents an integer of 0-5; and
n_{B1} and n_{B2} each independently represents 0, 1 or 2, wherein when n_{B1} represents 2, ring 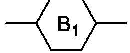 can be the same or different, wherein when n_{B2} represents 2, ring 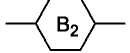 can be the same or different.

13. The liquid crystal composition according to claim 12, **characterized in that**, the compound of general formula B is selected from a group consisting of the following compounds: 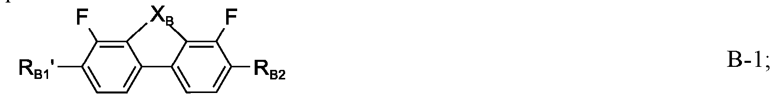 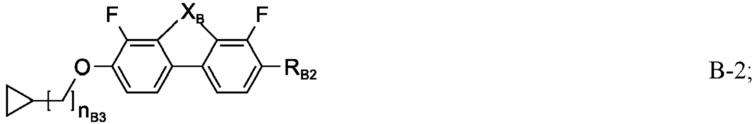 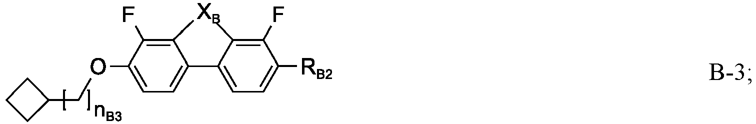 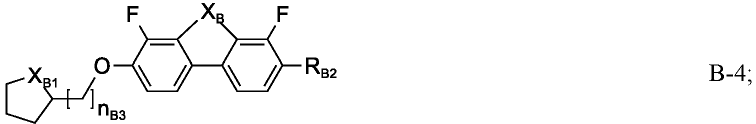 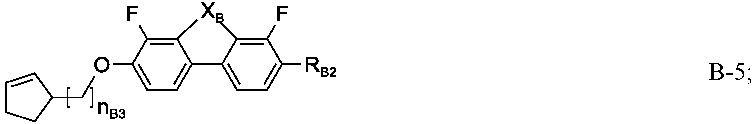 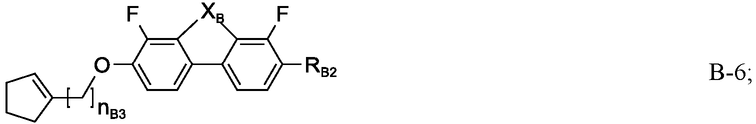 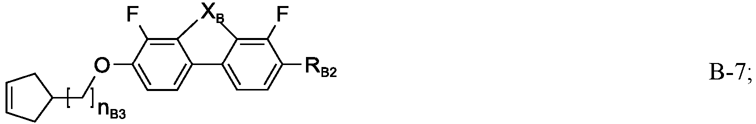 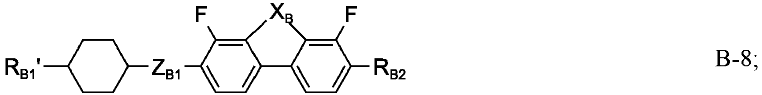 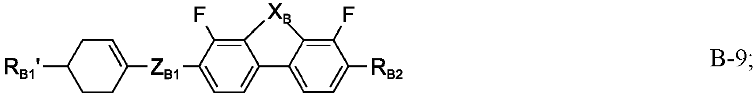 and 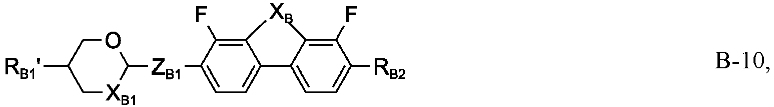
wherein, R_{B1}' represents C₁₋₈ linear alkyl or alkoxy, or C₂₋₈ linear alkenyl or alkenoxy; and
X_{B1} represents -O- or -CH₂-.

14. A liquid crystal display device comprising the liquid crystal composition of any one of claims 5-13.
